# EUROPEAN PATENT SPECIFICATION

(11) **EP 3 830 120 B1**
(45) Date of publication and mention of the grant of the patent: **24.05.2023**
(21) Application number: 19746089.2
(22) Date of filing: 31.07.2019
(51) Int. Cl.: C07K 16/28, C07K 14/47, A61K 38/00, A61P 35/00

(54) **NOVEL FUSION PROTEIN SPECIFIC FOR CD137 AND PD-L1**
NEUARTIGES, FÜR CD137 UND PD-L1 SPEZIFISCHES FUSIONSPROTEIN
NOUVELLE PROTÉINE DE FUSION SPÉCIFIQUE À CD137 ET PD-L1

(30) Priority: 31.07.2018 EP 18186445; 06.11.2018 EP 18204548
(43) Date of publication of application: 09.06.2021
(60) Divisional application: 23167877.2
(73) Proprietor: Pieris Pharmaceuticals GmbH, 85399 Hallbergmoos (DE); Les Laboratoires Servier, 92284 Suresnes (FR)
(72) Inventor: PAVLIDOU, Marina, 85356 Freising (DE); PATTARINI, Lucia, 75013 Paris (FR); SCHOLER-DAHIREL, Alix, 92000 Nanterre (FR); ROTHE, Christine, 85221 Dachau (DE); OLWILL, Shane, 84354 Freising (DE); BEL AIBA, Rachida, 80469 Munich (DE); HINNER, Marlon, 81247 Munich (DE); PEPER, Janet, 85354 Freising (DE)
(74) Representative: Schiweck Weinzierl Koch Patentanwälte Partnerschaft mbB
(86) International application number: PCT/EP2019/070596
(87) International publication number: WO 2020/025659

(56) References cited:
- EP-A1- 3 309 177
- WO-A1-2016/177762
- WO-A1-2016/184882
- WO-A1-2017/215590
- WO-A1-2018/087108
- WO-A1-2019/042153
- WO-A2-2015/104406
- WO-A2-2017/123650
- MARLON J. HINNER ET AL: "Abstract B023: Costimulatory T-cell engagement via a novel bispecific anti-CD137 /anti-HER2 protein based on Anticalin technology", CANCER IMMUNOLOGY RESEARCH, vol. 4, no. 1, 1 January 2016 (2016-01-01) , page B023, XP055634176, -& Marlon J Hinner ET AL: "Costimulatory T cell engagement via a novel bispecific anti-CD137/anti-HER2 protein based on Anticalin ? technology", , 18 September 2015 (2015-09-18), XP055250958, DOI: 10.1158/2326-6074.CRICIMTEATIAACR15-B023 Retrieved from the Internet: URL:http://c.eqcdn.com/pierisag/db/164/197 5/pdf/150914 CRI Poster postFinal.pdf [retrieved on 2016-02-17]
- JOHN-PAUL SILVA ET AL: "The S228P Mutation Prevents in Vivo and in Vitro IgG4 Fab-arm Exchange as Demonstrated using a Combination of Novel Quantitative Immunoassays and Physiological Matrix Preparation", JOURNAL OF BIOLOGICAL CHEMISTRY, vol. 290, no. 9, 27 February 2015 (2015-02-27), pages 5462-5469, XP055299482, ISSN: 0021-9258, DOI: 10.1074/jbc.M114.600973 cited in the application
- WOLFGANG GLAESNER ET AL: "Engineering and characterization of the long-acting glucagon-like peptide-1 analogue LY2189265, an Fc fusion protein", DIABETES/METABOLISM RESEARCH AND REVIEWS, vol. 26, no. 4, 30 April 2010 (2010-04-30) , pages 287-296, XP055181624, ISSN: 1520-7552, DOI: 10.1002/dmrr.1080 cited in the application

## Description

### I. BACKGROUND

Programmed death-ligand 1, or PD-L1 (also known as cluster of differentiation 274 or CD274 and B7 homolog 1 or B7-H1) is a single pass type I membrane protein belonging to the B7 family of co-stimulatory/co-inhibitory molecules of antigen presentation. The extracellular portion of PD-L1 contains two domains, an N-terminal IgV-type domain and an IgC-type domain. PD-L1 has a short cytoplasmatic domain without any obvious signal transduction motif, which led to the initial belief that there is no intrinsic signaling by PD-L1 as receptor. Recent data, however, show that the cytoplasmatic domain of PD-L1 contains non-classical conserved signal transduction motifs capable of inhibiting interferon (IFN) transduction and protecting cancer cells from IFN cytotoxicity (Gato-Canas et al., Cell Rep, 2017).

PD-L1 plays a crucial role in the suppression of the immune system during pregnancy, chronic infections, tissue allografts, autoimmune diseases, and cancer. PD-L1 is expressed on a variety of cell types including B cells, T cells, macrophages, myeloid dendritic cells, mast cells, epithelial, and vascular endothelial cells. It is also expressed in several cancer types including but not limited to melanoma, lung, bladder, colon, and breast cancer. High PD-L1 expression levels are associated with increased tumor aggressiveness by mediating the exhaustion and anergy of tumor infiltrating T cells, the secretion of immunosuppressive cytokines, and protection from lysis by cytotoxic T cells.

PD-L1 is a ligand of the programmed cell death protein 1 (PD-1), a key immune checkpoint inhibitory receptor that is primarily expressed on activated T cells but also on other cells of the immune system including B cells and monocytes. PD-1 is a member of the immunoglobulin family containing an IgV-like extracellular domain, a transmembrane domain and a cytoplasmatic tail with an ITIM (immunoreceptor tyrosine-based inhibitory motif) and an ITSM (immunoreceptor tyrosine-based switch motifj. Engagement of PD-1 by PD-L1 leads to the recruitment of src homology 2 domain-containing tyrosine phosphatases 1 and 2 (SHP 1 and 2) to the intracellular switch motifs of PD-1 and to the expression of E3 ubiquitin ligases of the CBL family. These ubiquitin ligases subsequently ubiquitinate and inactivate key TCR signal transduction mediators leading to the removal of TCR's from the cell surface (Karwacz et al., EMBO Mol Med, 2011). The SHP 1 and SHP 2 phosphatases inhibit TCR signaling directly by terminating ZAP70 and PI3K phosphorylation. In addition, PD-L1 engaged PD-1 can cause inhibition of TCR signaling pathways by affecting the expression and activity of CK2 and cyclin-dependent kinases (CDKs) (Arasanz et al., Oncotarget, 2017). It was also shown that PD-1 engagement leads to re-programming of the T cell metabolism from increased glycolysis, which is required to produce energy for effector functions, to fatty acid β-oxidation, which is associated with long-lived cells. This may also explain the survival and persistence of high PD-1 expressing cells in patients with chronic infections and cancer (Patsoukis et al., Nat Commun, 2015).

Blocking the PD-1/PD-L1 interaction by anti-PD-1 or anti-PD-L1 targeting agents can reverse the immune checkpoint function and release the brake on T cell responses. Currently three PD-L1 antibodies, atezolizumab (TECENTRIQ, MPDL3280A, RG7466), avelumab (BAVENCIO, MSB0010718C), and durvalumab (IMFINZI, MEDI4736), are approved for the treatment of cancer. Several successful clinical trials with these antibodies have shown high objective response rates, durability of response, or improved survival rates in bladder cancer, skin cancer and lung cancer (Xu-Monette et al., Front Immunol, 2017).

Cluster of differentiation 137 or CD137 (also known as 4-1 BB or TNFRS9) is a co-stimulatory immune receptor and a member of the tumor necrosis factor receptor (TNFR) super-family. It is primarily expressed on activated CD4+ and CD8+ T cells, activated B cells, and natural killer (NK) cells but can also be found on resting monocytes and dendritic cells (Li and Liu, Clin Pharmacol, 2013), or endothelial cells (Snell et al., Immunol Rev, 2011). CD137 plays an important role in regulation of the immune response and thus is a target for cancer immunotherapy. CD137 ligand (CD137L) is the only known natural ligand of CD137, and is constitutively expressed on several types of antigen presenting cells, such as activated B cells, monocytes, and splenic dendritic cells, and can be induced on T lymphocytes.

CD137L is a trimeric protein that exists as a membrane-bound form and as a soluble variant. The ability of soluble CD137L to activate CD137, e.g., on CD137-expressing lymphocytes is limited, however, and large concentrations are required to elicit an effect (Wyzgol et al., J Immunol, 2009). The natural way of activation of CD137 is via the engagement of a CD137-positive cell with a CD137L-positive cell. CD137 activation is then thought to be induced by clustering through CD137L on the opposing cell, leading to signaling via TRAF1, 2 and 3 (Yao et al., Nat Rev Drug Discov, 2013, Snell et al., Immunol Rev, 2011) and further concomitant downstream effects in the CD137-positive T-cell. In the case of T-cells activated by recognition of their respective cognate targets, the effects elicited by costimulation of CD137 are a further enhanced activation, enhanced survival and proliferation, the production of pro-inflammatory cytokines and an improved capacity to kill.

The benefit of CD137 costimulation for the elimination of cancer cells has been demonstrated in a number of *in vivo* models. The forced expression of CD137L on a tumor, for example, leads to tumor rejection (Melero et al., Eur J Immunol, 1998). Likewise, the forced expression of an anti-CD137 scFv on a tumor leads to a CD4⁺ T-cell and NK-cell dependent elimination of the tumor (Yang et al., CancerRes, 2007, Zhang et al., Mol Cancer Ther, 2006, Ye et al., Nat Med, 2002). A systemically administered anti-CD137 antibody has also been demonstrated to lead to retardation of tumor growth (Martinet et al., Gene Ther, 2002).

It has been shown that CD137 is an excellent marker for naturally occurring tumor-reactive T cells in human tumors (Ye et al., Clin Cancer Res, 2014), and that anti-CD137 antibodies can be employed to improve the expansion and activity of CD8⁺ melanoma tumor-infiltrating lymphocytes for the application in adoptive T-cell therapy (Chacon et al., PLoS One, 2013).

The preclinical demonstration of the potential therapeutic benefit of CD137 costimulation has spurred the development of therapeutic antibodies targeting CD137, including BMS-663513 (described in U.S. Patent No. 7,288,638) and PF-05082566 (Fisher et al., Cancer Immunol Immunother, 2012). WO 2017/123650 A2 relates to molecules that specifically engage 4-1 BB, a member of the TNF receptor superfamily (TNFRSF). More specifically, this invention relates to multivalent and multispecilic molecules that bind at least 4-1BB. WO 2017/215590 A1 relates to anti-PD-L1 antibodies or fragments thereof. The antibodies or fragments thereof specifically bind to the immunoglobulin C domain of the PD-L1 protein. Marlon J. Hinner et al: "Abstract B023: Costimulatory T-cell engagement via a novel bispecific anti-CD137 /anti-HER2 protein based on Anticalin technology", Cancer Immunology Research, vol. 4, no. 1, 1 January 2016 & Marlon J Hinner et al: "Costimulatory T cell engagement via a novel bispecific antiCD137/anti-HER2 protein based on Anticalin technology" relate to costimulatory T-cell engagement via a bispecific anti-CD137/anti-HER2 protein based on lipocalin mutein technology. WO 2018/087108 A1 relates to human lipocalin muteins that bind CD137 and can be used in pharmaceutical applications, for example, as anti-cancer agents and/or immune modulators for the treatment or prevention of human diseases such as cancer, infectious diseases, and autoimmune diseases.

The present disclosure provides, among other things, novel approaches for simultaneously engaging CD137 and PD-L1 via one or more fusion proteins having the properties of binding specificity for CD137 and binding specificity for PD-L1.

### II. DEFINITIONS

The following list defines terms, phrases, and abbreviations used throughout the instant specification. All terms listed and defined herein are intended to encompass all grammatical forms.

As used herein, unless otherwise specified, "CD137" means human CD137 (huCD137). Human CD137 means a full-length protein defined by UniProt Q07011, a fragment thereof, or a variant thereof. CD137 is also known as 4-1BB, tumor necrosis factor receptor superfamily member 9 (TNFRSF9), and induced by lymphocyte activation (ILA).

As used herein, unless otherwise specified, "programmed cell death 1 ligand 1" or "PD-L1" means human PD-L1 (huPD-L1). Human PD-L1 means a full-length protein defined by UniProt Q9NZQ7, a fragment thereof, or a variant thereof. Human PD-L1 is encoded by the *CD274* gene. PD-L1 is also known as cluster of differentiation 274 (CD274) or B7 homolog 1 (B7-H1).

As used herein, "binding affinity" describes the ability of a biomolecule (e.g., a polypeptide or a protein) of the disclosure (e.g., a lipocalin mutein, an antibody, a fusion protein, or any other peptide or protein) to bind a selected target and form a complex. Binding affinity is measured by a number of methods known to those skilled in the art including, but are not limited to, fluorescence titration, enzyme-linked immunosorbent assay (ELISA)-based assays, including direct and competitive ELISA, calorimetric methods, such as isothermal titration calorimetry (ITC), and surface plasmon resonance (SPR). These methods are well-established in the art and some examples of such methods are further described herein. Binding affinity is thereby reported as a value of dissociation constant (K_{D}), half maximal effective concentration (EC₅₀), or half maximal inhibitory concentration (IC₅₀) measured using such these methods. A lower K_{D}, EC₅₀, or IC₅₀ value reflects better (higher) binding ability (affinity). Accordingly, the binding affinities of two biomolecules toward a selected target can be measured and compared. When comparing the binding affinities of two biomolecules toward the selected target, the term "about the same," "substantially the same" or "substantially similar" means one biomolecule has a binding affinity reported as a K_{D}, an EC₅₀, or an IC₅₀ value that is identical or similar to another molecule within the experimental variability of the binding affinity measurement. The experimental variability of the binding affinity measurement is dependent upon the specific method used and is known to those skilled in the art.

As used herein, the term "substantially" may also refer to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

As used herein, the term "detect," "detection," "detectable," or "detecting" is understood both on a quantitative and a qualitative level, as well as a combination thereof. It thus includes quantitative, semi-quantitative, and qualitative measurements performed on a biomolecule of the disclosure.

As used herein, "detectable affinity" generally means the binding ability between a biomolecule and its target, reported by a K_{D}, EC₅₀, or IC₅₀ value, is at most about 10⁻⁵ M or lower. A binding affinity, reported by a K_{D}, EC₅₀, or IC₅₀ value, higher than 10⁻⁵ M is generally no longer measurable with common methods such as ELISA and SPR and is therefore of secondary importance.

It is noted that the complex formation between the biomolecule of the disclosure and its target is influenced by many different factors such as the concentrations of the respective target, the presence of competitors, pH and the ionic strength of the buffer system used, the experimental method used for determination of the binding affinity (e.g., fluorescence titration, competitive ELISA (also called competition ELISA), and surface plasmon resonance), and even the mathematical algorithm used for evaluation of the experimental data. Therefore, it is clear to the skilled person that binding affinity reported by a K_{D}, EC₅₀, or IC₅₀ value may vary within a certain experimental range, depending on the method and experimental setup. This means that there may be a slight deviation in the measured K_{D}, EC₅₀, or IC₅₀ values or a tolerance range depending, for example, on whether such values were determined by ELISA (including direct or competition ELISA), by SPR, or by another method.

As used herein, "specific for," "specific binding," "specifically bind," or "binding specificity" relates to the ability of a biomolecule to discriminate between the desired target (for example, CD137 and PD-L1) and one or more reference targets (for example, cellular receptor for neutrophil gelatinase-associated lipocalin). It is understood that such specificity is not an absolute but a relative property and can be determined, for example, in accordance with SPR, western blots, ELISA, fluorescence activated cell sorting (FACS), radioimmunoassay (RIA), electrochemiluminescence (ECL), immunoradiometric assay (IRMA), ImmunoHistoChemistry (IHC), and peptide scans.

When used herein in the context of the fusion protein of the present disclosure that bind to CD137 and PD-L1, the term "specific for," "specific binding," "specifically bind," or "binding specificity" means that the fusion protein binds to, reacts with, or is directed against CD137 and PD-L1, as described herein, but does not essentially bind another protein. The term "another protein" includes any proteins that are not CD137 or PD-L1 or proteins closely related to or being homologous to CD137 or PD-L1. However, CD137 or PD-L1 from species other than human and fragments and/or variants of CD137 or PD-L1 are not excluded by the term "another protein." The term "does not essentially bind" means that the fusion proteins of the present disclosure bind another protein with lower binding affinity than CD137 and/or PD-L1, i.e., shows a cross-reactivity of less than 30%, preferably 20%, more preferably 10%, particularly preferably less than 9, 8, 7, 6, or 5%. Whether the fusion protein specifically reacts as defined herein above can easily be tested, inter alia, by comparing the reaction of a fusion protein of the present disclosure with CD137 and/or PD-L1 and the reaction of said fusion protein with (an)other protein(s).

As used herein, the term "lipocalin" refers to a monomeric protein of approximately 18-20 kDa in weight, having a cylindrical β-pleated sheet supersecondary structural region comprising a plurality of β-strands (preferably eight β-strands designated A to H) connected pair-wise by a plurality of (preferably four) loops at one end to thereby comprise a ligand-binding pocket and define the entrance to the ligand-binding pocket. It is well-established that the diversity of the said loops in the otherwise rigid lipocalin scaffold gives rise to a variety of different binding modes among the lipocalin family members, each capable of accommodating targets of different sizes, shape, and chemical character (reviewed, e.g. in Skerra, Biochim Biophys Acta, 2000, Flower et al., Biochim Biophys Acta, 2000, Flower, Biochem J, 1996). It is understood that the lipocalin family of proteins has naturally evolved to bind a wide spectrum of ligands, sharing unusually low levels of overall sequence conservation (often with sequence identities of less than 20%) yet retaining a highly conserved overall folding pattern. The correspondence between positions in various lipocalins is also well-known to one of skill in the art (see, e.g., U.S. Patent No. 7,250,297). Proteins fall in the definition of "lipocalin" as used herein include, but not limited to, human lipocalins including tear lipocalin (Tlc, Lcn1), Lipocalin-2 (Lcn2) or neutrophil gelatinase-associated lipocalin (NGAL), apolipoprotein D (ApoD), apolipoprotein M, α₁-acid glycoprotein 1, α₁-acid glycoprotein 2, α₁-microglobulin, complement component 8γ, retinol-binding protein (RBP), the epididymal retinoic acid-binding protein, glycodelin, odorant-binding protein Ila, odorant-binding protein lib, lipocalin-15 (Lcn15), and prostaglandin D synthase.

As used herein, unless otherwise specified, "tear lipocalin" refers to human tear lipocalin (hTIc) and further refers to mature human tear lipocalin. The term "mature" when used to characterize a protein means a protein essentially free from the signal peptide. A "mature hTlc" of the instant disclosure refers to the mature form of human tear lipocalin, which is free from the signal peptide. Mature hTlc is described by residues 19-176 of the sequence deposited with the SWISS-PROT Data Bank under Accession Number P31025, and the amino acid of which is indicated in SEQ ID NO: 1.

As used herein, "Lipocalin-2" or "neutrophil gelatinase-associated lipocalin" refers to human Lipocalin-2 (hLcn2) or human neutrophil gelatinase-associated lipocalin (hNGAL) and further refers to the mature human Lipocalin-2 or mature human neutrophil gelatinase-associated lipocalin. The term "mature" when used to characterize a protein means a protein essentially free from the signal peptide. A "mature hNGAL" of the instant disclosure refers to the mature form of human neutrophil gelatinase-associated lipocalin, which is free from the signal peptide. Mature hNGAL is described by residues 21-198 of the sequence deposited with the SWISS-PROT Data Bank under Accession Number P80188, and the amino acid of which is indicated in SEQ ID NO: 2.

As used herein, a "native sequence" refers to a protein or a polypeptide having a sequence that occurs in nature or having a wild-type sequence, regardless of its mode of preparation. Such native sequence protein or polypeptide can be isolated from nature or can be produced by other means, such as by recombinant or synthetic methods.

The "native sequence lipocalin" refers to a lipocalin having the same amino acid sequence as the corresponding polypeptide derived from nature. Thus, a native sequence lipocalin can have the amino acid sequence of the respective naturally-occurring (wild-type) lipocalin from any organism, in particular, a mammal. The term "native sequence", when used in the context of a lipocalin specifically encompasses naturally-occurring truncated or secreted forms of the lipocalin, naturally-occurring variant forms such as alternatively spliced forms and naturally-occurring allelic variants of the lipocalin. The terms "native sequence lipocalin" and "wild-type lipocalin" are used interchangeably herein.

As used herein, a "mutein," a "mutated" entity (whether protein or nucleic acid), or "mutant" refers to the exchange, deletion, or insertion of one or more amino acids or nucleotides, compared to the naturally-occurring (wild-type) protein or nucleic acid. Said term also includes fragments of a mutein as described herein. The present disclosure explicitly encompasses lipocalin muteins, as described herein, having a cylindrical β-pleated sheet supersecondary structural region comprising eight β-strands connected pair-wise by four loops at one end to thereby comprise a ligand-binding pocket and define the entrance of the ligand-binding pocket, wherein at least one amino acid of each of at least three of said four loops has been mutated as compared to the native sequence lipocalin.

As used herein, the term "fragment," in connection with the lipocalin muteins of the disclosure, refers to proteins or polypeptides derived from full-length mature hTIc or hNGAL or lipocalin muteins that are N-terminally and/or C-terminally truncated, i.e., lacking at least one of the N-terminal and/or C-terminal amino acids. Such fragments may include at least 10 or more, such as 20 or 30 or more consecutive amino acids of the primary sequence of mature hTIc or hNGAL or the lipocalin mutein it is derived and are usually detectable in an immunoassay of mature hTIc or hNGAL. Such a fragment may lack up to 2, up to 3, up to 4, up to 5, up to 10, up to 15, up to 20, up to 25, or up to 30 (including all numbers in between) of the N-terminal and/or C-terminal amino acids. As an illustrative example, such a fragment may lack the one, two, three, or four N-terminal (His-His-Leu-Leu) and/or one or two C-terminal amino acids (Ser-Asp) of mature hTlc. It is understood that the fragment is preferably a functional fragment of mature hTIc or hNGAL or the lipocalin mutein from which it is derived, which means that it preferably retains the binding specificity, preferably to CD137, of mature hTlc/hNGAL or lipocalin mutein it is derived from. As an illustrative example, such a functional fragment may comprise at least amino acids at positions 5-153, 5-150, 9-148, 12-140, 20-135, or 26-133 corresponding to the linear polypeptide sequence of mature hTlc. As another illustrative example, such a functional fragment may comprise at least amino acids at positions 13-157, 15-150, 18-141, 20-134, 25-134, or 28-134 corresponding to the linear polypeptide sequence of mature hNGAL.

A "fragment" with respect to the corresponding target CD137 or PD-L1 of a fusion protein of the disclosure, refers to N-terminally and/or C-terminally truncated CD137 or PD-L1 or protein domains of CD137 or PD-L1. Fragments of CD137 or fragments of PD-L1 as described herein retain the capability of the full-length CD137 or PD-L1 to be recognized and/or bound by a fusion protein of the disclosure. As an illustrative example, the fragment may be an extracellular domain of CD137 or PD-L1. As an illustrative example, such an extracellular domain may comprise amino acids of the extracellular subdomains of CD137, such as the individual or combined amino acid sequences of domain 1 (residues 24-45 of UniProt Q07011), domain 2 (residues 46-86), domain 3 (87-118) and domain 4 (residues 119-159). As another illustrative example, such an extracellular domain may comprise amino acids residues 19-238 of UniProt Q9NZQ7.

As used herein, the term "variant" relates to derivatives of a protein or polypeptide that include mutations, for example by substitutions, deletions, insertions, and/or chemical modifications of an amino acid sequence or nucleotide sequence. Such substitutions may be conservative, i.e., an amino acid residue is replaced with a chemically similar amino acid residue. Examples of conservative substitutions are the replacements among the members of the following groups: 1) alanine, serine, threonine, and valine; 2) aspartic acid, glutamic acid, glutamine, and asparagine, and histidine; 3) arginine, lysine, glutamine, asparagine, and histidine; 4) isoleucine, leucine, methionine, valine, alanine, phenylalanine, threonine, and proline; and 5) isoleucine, leucine, methionine, phenylalanine, tyrosine, and tryptophan. Such variants include proteins or polypeptides, wherein one or more amino acids have been substituted by their respective D-stereoisomers or by amino acids other than the naturally occurring 20 amino acids, such as, for example, ornithine, hydroxyproline, citrulline, homoserine, hydroxylysine, norvaline. Such variants also include, for instance, proteins or polypeptides in which one or more amino acid residues are added or deleted at the N- and/or C-terminus. Generally, a variant has at least about 50%, 60%, 70%, 75%, 80%, 85%, 90%, 92%, 95% or at least about 98% amino acid sequence identity with the native sequence protein or polypeptide. A variant preferably retains the biological activity, e.g. binding the same target, of the protein or polypeptide it is derived.

The term "variant", as used herein with respect to the corresponding protein ligand CD137 or PD-L1 of a fusion protein of the disclosure, relates to CD137 or PD-L1 or fragment thereof, respectively, that has one or more such as 1, 2, 3, 4 ,5 ,6, 7, 8, 9, 10, 12, 14, 16, 18, 20, 22, 24, 26, 28, 30, 40, 50, 60, 70, 80 or more amino acid substitutions, deletions and/or insertions in comparison to the native sequence of CD137 or PD-L1 (wild-type CD137 or PD-L1),such as CD137 as deposited with UniProt Q07011 or PD-L1 as deposited with UniProt Q9NZQ7 as described herein. A CD137 variant or a PD-L1 variant, respectively, has preferably an amino acid identity of at least 50%, 60%, 70%, 80%, 85%, 90% or 95% with a wild-type CD137 or PD-L1. A CD137 variant or a PD-L1 variant as described herein retains the ability to bind fusion proteins specific to CD137 and PD-L1 as set out in the appended set of claims.

The term "variant", as used herein with respect to a lipocalin mutein, relates to a lipocalin mutein or fragment thereof of the disclosure, wherein the sequence has mutations, including substitutions, deletions, and insertions, and/or chemical modifications. A variant of lipocalin mutein as described herein retains the biological activity, e.g., binding to CD137, of the lipocalin mutein from which it is derived. Generally, a lipocalin mutein variant has at least about 50%, 60%, 70%, 75%, 80%, 85%, 90%, 92%, 95%, 98% amino acid sequence identity with the lipocalin mutein from which it is derived.

As used herein, the term "mutagenesis" refers to the introduction of mutations into a polynucleotide or amino acid sequence. Mutations are preferably introduced under experimental conditions such that the amino acid naturally occurring at a given position of the protein or polypeptide sequence of can be altered, for example substituted by at least one amino acid. The term "mutagenesis" also includes the (additional) modification of the length of sequence segments by deletion or insertion of one or more amino acids. Thus, it is within the scope of the disclosure that, for example, one amino acid at a chosen sequence position is replaced by a stretch of three amino acids, leading to an addition of two amino acid residues compared to the length of the respective segment of the native protein or polypeptide amino acid sequence. Such an insertion or deletion may be introduced independently from each other in any of the sequence segments that can be subjected to mutagenesis in the disclosure.

As used herein, the term "random mutagenesis" means that no predetermined mutation (alteration of amino acid) is present at a certain sequence position but that at least two amino acids can be incorporated with a certain probability at a predefined sequence position during mutagenesis.

As used herein, the term "sequence identity" or "identity" denotes a property of sequences that measures their similarity or relationship. The term "sequence identity" or "identity" as used in the present disclosure means the percentage of pair-wise identical residues - following (homologous) alignment of a sequence of a protein or polypeptide of the disclosure with a sequence in question - with respect to the number of residues in the longer of these two sequences. Sequence identity is measured by dividing the number of identical amino acid residues by the total number of residues and multiplying the product by 100.

As used herein, the term "sequence homology" or "homology" has its usual meaning and homologous amino acid includes identical amino acids as well as amino acids which are regarded to be conservative substitutions at equivalent positions in the linear amino acid sequence of a protein or polypeptide of the disclosure (e.g., any fusion proteins or lipocalin muteins of the disclosure).

A skilled artisan will recognize available computer programs, for example BLAST (Altschul et al., Nucleic Acids Res, 1997), BLAST2 (Altschul et al., J Mol Biol, 1990), and Smith-Waterman (Smith and Waterman, J Mol Biol, 1981), for determining sequence homology or sequence identity using standard parameters. The percentage of sequence homology or sequence identity can, for example, be determined herein using the program BLASTP, version 2.2.5 (November 16, 2002; (Altschul et al., Nucleic Acids Res, 1997). In this example not being part of the claimed invention, the percentage of homology is based on the alignment of the entire protein or polypeptide sequences (matrix: BLOSUM 62; gap costs: 11.1; cutoff value set to 10⁻³) including the propeptide sequences, preferably using the wild-type protein scaffold as reference in a pairwise comparison. It is calculated as the percentage of numbers of "positives" (homologous amino acids) indicated as result in the BLASTP program output divided by the total number of amino acids selected by the program for the alignment.

Specifically, in order to determine whether an amino acid residue of the amino acid sequence of a lipocalin mutein is different from a wild-type lipocalin corresponding to a certain position in the amino acid sequence of a wild-type lipocalin, a skilled artisan can use means and methods well-known in the art, e.g., alignments, either manually or by using computer programs such as BLAST 2.0, which stands for Basic Local Alignment Search Tool, or ClustalW, or any other suitable program which is suitable to generate sequence alignments. Accordingly, a wild-type sequence of lipocalin can serve as "subject sequence" or "reference sequence," while the amino acid sequence of a lipocalin mutein different from the wild-type lipocalin described herein serves as "query sequence." The terms "wild-type sequence," "reference sequence," and "subject sequence" are used interchangeably herein. A preferred wild-type sequence of lipocalin is the sequence of hTLc as shown in SEQ ID NO: 1 or hNGAL as shown in SEQ ID NO: 2.

"Gaps" are spaces in an alignment that are the result of additions or deletions of amino acids. Thus, two copies of exactly the same sequence have 100% identity, but sequences that are less highly conserved, and have deletions, additions, or replacements, may have a lower degree of sequence identity.

As used herein, the term "position" means the position of either an amino acid within an amino acid sequence depicted herein or the position of a nucleotide within a nucleic acid sequence depicted herein. It is to be understood that when the term "correspond" or "corresponding" as used herein in the context of the amino acid sequence positions of one or more lipocalin muteins, a corresponding position is not only determined by the number of the preceding nucleotides or amino acids. Accordingly, the absolute position of a given amino acid in accordance with the disclosure may vary from the corresponding position due to deletion or addition of amino acids elsewhere in a (mutant or wild-type) lipocalin. Similarly, the absolute position of a given nucleotide in accordance with the present disclosure may vary from the corresponding position due to deletions or additional nucleotides elsewhere in a mutein or wild-type lipocalin 5'-untranslated region (UTR) including the promoter and/or any other regulatory sequences or gene (including exons and introns).

A "corresponding position" in accordance with the disclosure may be the sequence position that aligns to the sequence position it corresponds to in a pairwise or multiple sequence alignment according to the present disclosure. It is preferably to be understood that for a "corresponding position" in accordance with the disclosure, the absolute positions of nucleotides or amino acids may differ from adjacent nucleotides or amino acids but said adjacent nucleotides or amino acids which may have been exchanged, deleted, or added may be comprised by the same one or more "corresponding positions".

In addition, for a corresponding position in a lipocalin mutein based on a reference sequence in accordance with the disclosure, it is preferably to be understood that the positions of nucleotides or amino acids of a lipocalin mutein can structurally correspond to the positions elsewhere in a reference lipocalin (wild-type lipocalin) or another lipocalin mutein, even if they may differ in the absolute position numbers, as appreciated by the skilled in light of the highly-conserved overall folding pattern among lipocalins.

As used interchangeably herein, the terms "conjugate," "conjugation," "fuse," "fusion," or "linked" refer to the joining together of two or more subunits, through all forms of covalent or non-covalent linkage, by means including, but not limited to, genetic fusion, chemical conjugation, coupling through a linker or a cross-linking agent, and non-covalent association.

The term "fusion polypeptide" or "fusion protein" as used herein refers to a polypeptide or protein comprising two or more subunits. A fusion protein as set out in the appended set of claims comprises two or more subunits, at least one of these subunits being capable of specifically binding to CD137, and a further subunit capable of specifically binding to PD-L1. Within the fusion protein, these subunits may be linked by covalent or non-covalent linkage. Preferably, the fusion protein is a translational fusion between the two or more subunits. The translational fusion may be generated by genetically engineering the coding sequence for one subunit in a reading frame with the coding sequence of a further subunit. Both subunits may be interspersed by a nucleotide sequence encoding a linker. However, the subunits of a fusion protein of the present disclosure may also be linked through chemical conjugation. The subunits forming the fusion protein are typically linked to each other C-terminus of one subunit to the N-terminus of another subunit, or C-terminus of one subunit to C-terminus of another subunit, or N-terminus of one subunit to N-terminus of another subunit, or N-terminus of one subunit to C-terminus of another subunit. The subunits of the fusion protein can be linked in any order and may include more than one of any of the constituent subunits. If one or more of the subunits is part of a protein (complex) that consists of more than one polypeptide chain, the term "fusion protein" may also refer to the protein comprising the fused sequences and all other polypeptide chain(s) of the protein (complex). As an illustrative example, where a full-length immunoglobulin is fused to a lipocalin mutein via a heavy or light chain of the immunoglobulin, the term "fusion protein" may refer to the single polypeptide chain comprising the lipocalin mutein and the heavy or light chain of the immunoglobulin. The term "fusion protein" may also refer to the entire immunoglobulin (both light and heavy chains) and the lipocalin mutein fused to one or both of its heavy and/or light chains.

As used herein, the term "subunit" of a fusion protein disclosed herein refers to a single protein or a separate polypeptide chain, which may form a stable folded structure by itself and define a unique function of providing binding motif towards a target.

A "linker" that may be comprised by a fusion protein of the present disclosure joins together two or more subunits of a fusion protein as described herein. The linkage can be covalent or non-covalent. A preferred covalent linkage is via a peptide bond, such as a peptide bond between amino acids. A preferred linker is a peptide linker. Accordingly, in a preferred embodiment, said linker comprises one or more amino acids, such as 1, 2, 3, 4, 5, 6, 7, 8, 9, 10, 11, 12, 13, 14, 15, 16, 17, 18, 19, 20 or more amino acids. Preferred peptide linkers are described herein, including glycine-serine (GS) linkers, glycosylated GS linkers, and proline-alanine-serine polymer (PAS) linkers. In some preferred embodiments, a GS linker is a (G₄S)₃ as described in SEQ ID NO: 13 is used to join together the subunits of a fusion protein. Other preferred linkers include chemical linkers.

As used herein, the term "albumin" includes all mammal albumins such as human serum albumin or bovine serum albumin or rat serum albumin.

As used herein, the term "organic molecule" or "small organic molecule" denotes an organic molecule comprising at least two carbon atoms, but preferably not more than 7 or 12 rotatable carbon bonds, having a molecular weight in the range between 100 and 2,000 daltons, preferably between 100 and 1,000 daltons, and optionally including one or two metal atoms.

A "sample" is defined as a biological sample taken from any subject. Biological samples include, but are not limited to, blood, serum, urine, feces, semen, or tissue, including tumor tissue.

A "subject" is a vertebrate, preferably a mammal, more preferably a human. The term "mammal" is used herein to refer to any animal classified as a mammal, including, without limitation, humans, domestic and farm animals, and zoo, sports, or pet animals, such as sheep, dogs, horses, cats, cows, rats, pigs, apes such as cynomolgus monkeys, to name only a few illustrative examples. Preferably, the "mammal" used herein is human.

An "effective amount" is an amount sufficient to yield beneficial or desired results. An effective amount can be administered in one or more individual administrations or doses.

As used herein, "antibody" includes whole antibodies or any antigen binding fragment (i.e., "antigen-binding portion") or single chain thereof. An whole antibody refers to a glycoprotein comprising at least two heavy chains (HCs) and two light chains (LCs) interconnected by disulfide bonds. Each heavy chain is comprised of a heavy chain variable domain (V_{H} or HCVR) and a heavy chain constant region (C_{H}). The heavy chain constant region is comprised of three domains, C_{H1}, C_{H2} and C_{H3}. Each light chain is comprised of a light chain variable domain (V_{L} or LCVR) and a light chain constant region (C_{L}). The light chain constant region is comprised of one domain, C_{L}. The V_{H} and V_{L} regions can be further subdivided into regions of hypervariability, termed complementarity determining regions (CDRs), interspersed with regions that are more conserved, termed framework regions (FRs). Each V_{H} and V_{L} is composed of three CDRs and four FRs, arranged in the following order from the amino-terminus to the carboxy-terminus: FR1, CDR1, FR2, CDR2, FR3, CDR3, FR4. The variable regions of the heavy and light chains contain a binding domain that interacts with an antigen (for example, PD-L1). The constant regions of the antibodies may optionally mediate the binding of the immunoglobulin to host tissues or factors, including various cells of the immune system (e.g., effector cells) and the first component (C1q) of the classical complement system.

As used herein, "antigen binding fragment" of an antibody refers to one or more fragments of an antibody that retain the ability to specifically bind to an antigen (e.g., PD-L1). It has been shown that the antigen-binding function of an antibody can be performed by fragments of a full-length antibody. Examples of binding fragments encompassed within the term "antigen-binding fragment" of an antibody include (i) a Fab fragment consisting of the V_{H}, V_{L}, C_{L} and C_{H1} domains; (ii) a F(ab')₂ fragment comprising two Fab fragments linked by a disulfide bridge at the hinge region; (iii) a Fab' fragment consisting of the V_{H}, V_{L}, C_{L} and C_{H1} domains and the region between C_{H1} and C_{H2} domains; (iv) a Fd fragment consisting of the V_{H} and C_{H1} domains; (v) a single-chain Fv fragment consisting of the V_{H} and V_{L} domains of a single arm of an antibody, (vi) a dAb fragment (Ward et al., Nature, 1989) consisting of a V_{H} domain; and (vii) an isolated complementarity determining region (CDR) or a combination of two or more isolated CDRs which may optionally be joined by a synthetic linker; (viii) a "diabody" comprising the V_{H} and V_{L} connected in the same polypeptide chain using a short linker (see, e.g., patent documents EP 404,097; WO 93/11161; and Holliger et al., Proc Natl Acad Sci U S A, 1993); (ix) a "domain antibody fragment" containing only the V_{H} or V_{L}, where in some instances two or more V_{H} regions are covalently joined.

Antibodies may be polyclonal or monoclonal; xenogeneic, allogeneic, or syngeneic; or modified forms thereof (e.g., humanized, chimeric, or multispecific). Antibodies may also be fully human.

As used herein, "framework" or "FR" refers to the variable domain residues other than the hypervariable region (CDR) residues.

"Fragment crystallizable region" or "Fc region" refers to the C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof numbering according to EU index of Kabat (Johnson and Wu, Nucleic Acids Res, 2000). The C-terminal lysine (residue 447 according to EU index of Kabat) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue.

"Fc receptor" or "FcR" refers to a receptor that binds to the Fc region of an antibody.

As used herein, "isolated antibody" refers to an antibody that is substantially free of its natural environment. For instance, an isolated antibody is substantially free of cellular material and other proteins from the cell or tissue source from which it is derived. An "isolated antibody" further refers to an antibody that is substantially free of other antibodies having different antigenic specificities. In the present case, an isolated antibody that binds specifically PD-L1 is substantially free of antibodies that specifically bind antigens other than PD-L1. However, an isolated antibody that specifically binds PD-L1 may have cross-reactivity to other antigens, such as PD-L1 molecules from other species.

As used herein, "monoclonal antibody" refers to a preparation of antibody molecules of single molecular composition. A monoclonal antibody composition displays a single binding specificity and affinity for a particular epitope.

As used herein, "humanized antibody" refers to an antibody that consists of the CDR of antibodies derived from mammals other than human, and the FR region and the constant region of a human antibody or derived from a human antibody. The term "therapeutic agent" or "therapeutically active agent", as used herein, refers to an agent which is therapeutically useful. A therapeutic agent may be any agent for the prevention, amelioration, or treatment of a diseases, a physiological condition, a symptom, or for the evaluation or diagnosis thereof.

As used herein, "human antibody" includes antibodies having variable regions in which both the framework and CDR regions are derived from human germline immunoglobulin sequences. Furthermore, if the antibody contains a constant region, the constant region is also derived from human germline immunoglobulin sequences. However, the term "human antibody", as used herein, is not intended to include antibodies in which CDR sequences derived from the germline of another mammalian species, such as a mouse, have been grafted onto human framework sequences.

### III. DESCRIPTIONS OF FIGURES

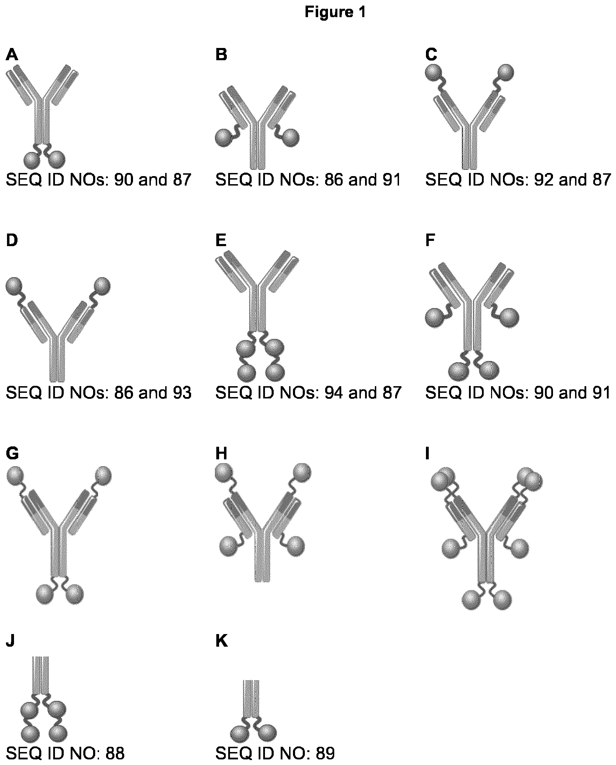

**Figure 1****:** provides an overview over the design of the representative fusion proteins described in this application that are bispecific for the targets CD137 and PD-L1. Representative fusion proteins were made based on an antibody specific for PD-L1 (e.g. an antibody whereby heavy chains are provided by SEQ ID NO: 86, or comprise a heavy chain variable domain of SEQ ID NO: 77, or comprise the CDR sequences of GFSLSNYD (HCDR1, SEQ ID NO: 60), IWTGGAT (HCDR2, SEQ ID NO: 61), and VRDSNYRYDEPFTY (HCDR3; SEQ ID NO: 62), and light chains are provided by SEQ ID NO: 87, or comprise a heavy chain variable domain of SEQ ID NO: 82, or comprise the CDR sequences of QSIGTN (LCDR1, SEQ ID NO: 63), YAS (LCDR2), and QQSNSWPYT (LCDR3; SEQ ID NO: 64)) and one or more lipocalin mutein specific for CD137 (e.g., the lipocalin mutein of SEQ ID NO: 42). One or more lipocalin muteins were genetically fused to the C- and/or the N-terminus of either the heavy chain or the light chain of a PD-L1 specific antibody as depicted in **Figure 1A-1I****,** resulting in the fusion proteins, e.g., SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91. The generated fusion proteins can be bivalent to CD137 (e.g., as depicted in **Figure 1A-1D**), or tetravalent to CD137 (e.g., as depicted in **Figure 1E-1H**), or have even higher valency to CD137 (e.g., as depicted in **Figure 1I**). Additional monospecific fusion proteins were generated by fusing one or more CD137 specific lipocalin muteins (e.g., as depicted in **Figure 1J-1K**) to the C-terminus of the Fc region of an antibody provided as described herein via a peptide linker. The resulting monospecific fusion proteins are provided in, e.g., SEQ ID NO: 88 and SEQ ID NO: 89.

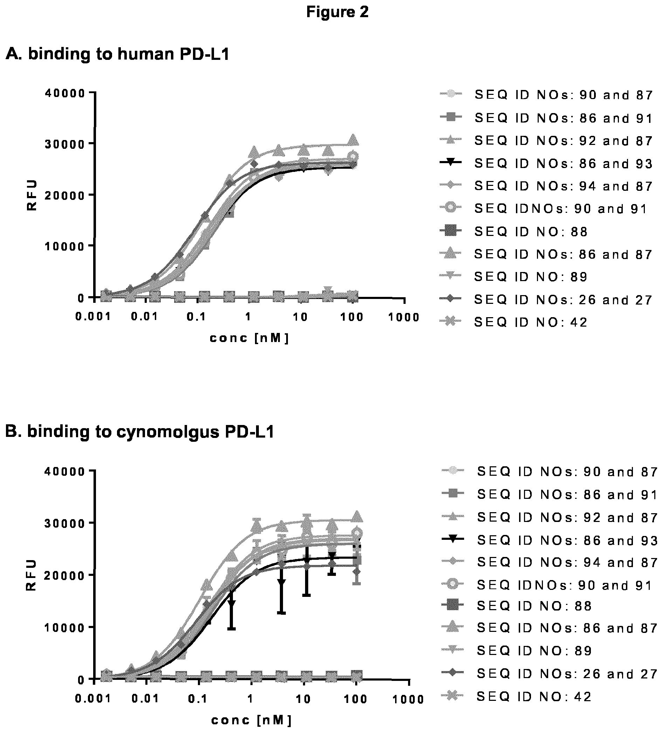

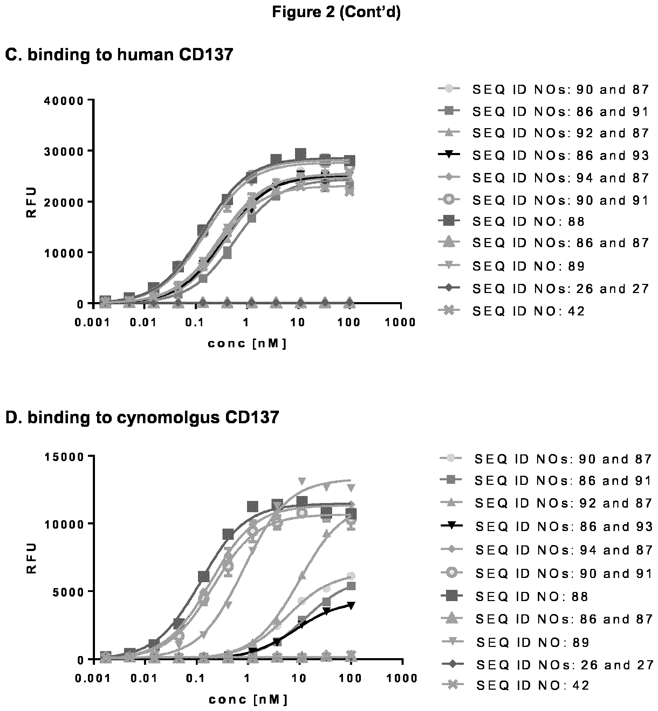

**Figure 2****:** shows the results of ELISA experiments in which the binding to PD-L1 or CD137 of representative fusion proteins was determined as described in **Example 4**. PD-L1 or CD137 (with C-terminal His or Fc tag) was coated on a microtiter plate, and the tested agents were titrated starting with the highest concentration of 100 nM. Bound agents under study were detected via anti-human IgG Fc-HRP or anti-NGAL-HRP respectively. The data was fit with a 1:1 binding model with EC₅₀ value and the maximum signal as free parameters, and a slope that was fixed to one. The resulting EC₅₀ values are provided in **Table 4.**

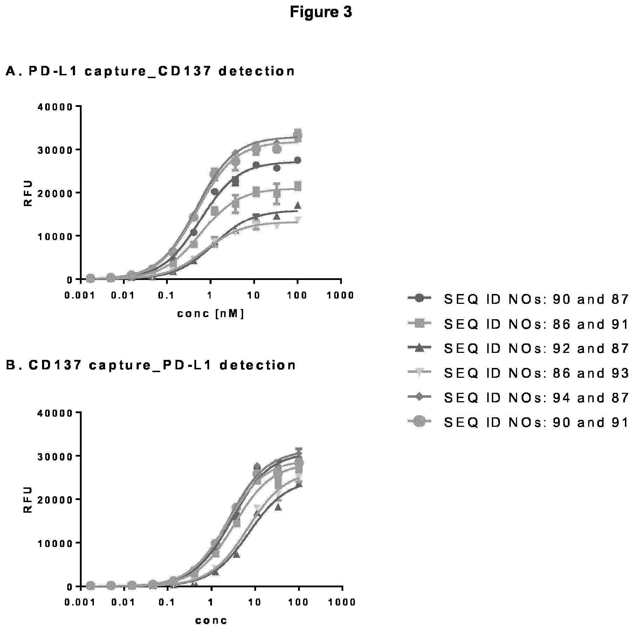

**Figure 3****:** illustrates the results of an ELISA experiment in which the ability of representative fusion proteins to simultaneously bind both targets, PD-L1 and CD137, was determined as described in **Example 5.** Recombinant huPD-L1-His or huCD137-His was coated on a microtiter plate, followed by a titration of the fusion proteins starting with the highest concentration of 100 nM. Subsequently, a constant concentration of biotinylated huCD137-His or biotinylated huPD-L1-His, respectively, was added, which was detected via ExtrAvidin-Peroxidase.

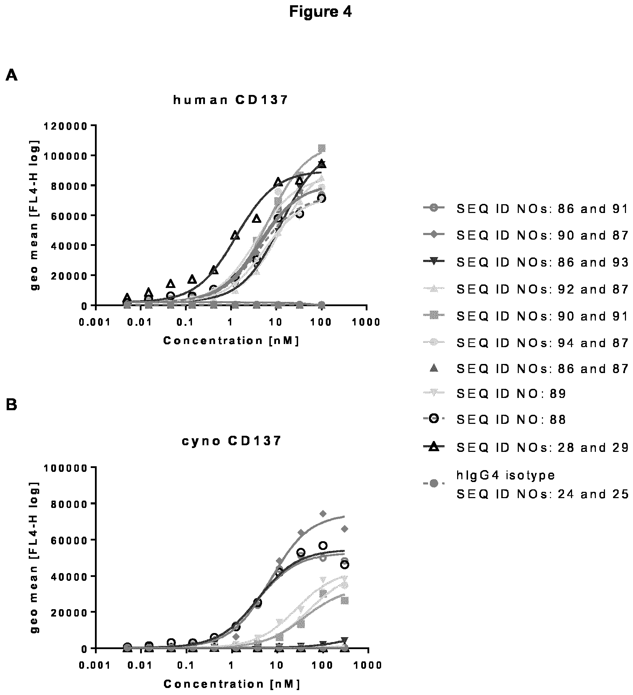

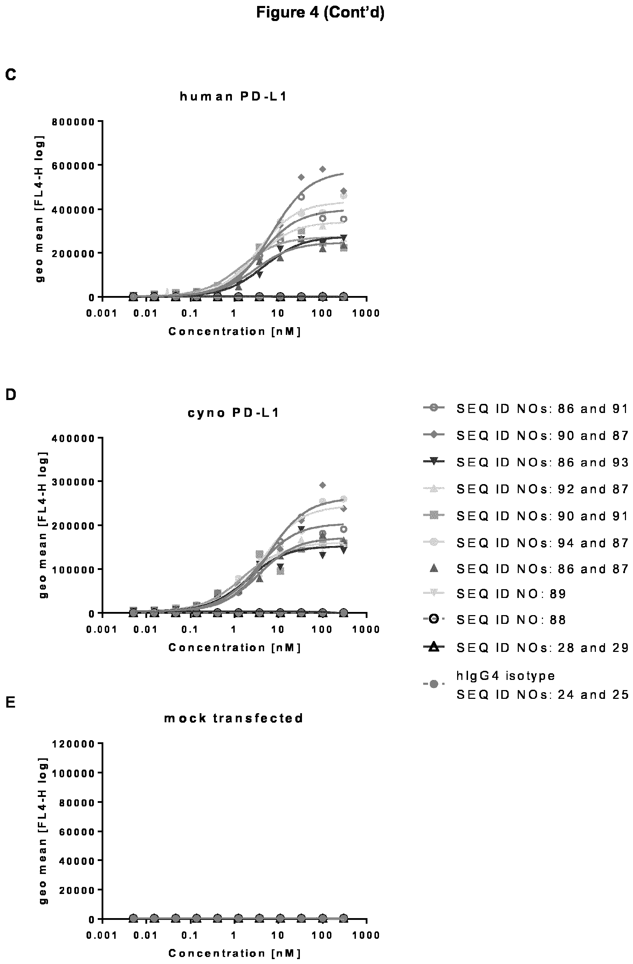

**Figure 4****:** shows the results of an assessment of the target binding of fusion proteins by flow cytometry using human or cynomolgus CD137 (**Figure 4A-4B**) as well as human or cynomolgus PD-L1 (**Figure 4C-4D**) expressing Flp-In-CHO cells as described in **Example 6**. No binding was observed when using mock transfected Flp-In-CHO cells (**Figure 4E**). The geometric means of the fluorescence intensity were used to calculate EC₅₀ values using nonlinear regression (shared bottom, SLOPE =1). EC₅₀ values are provided in **Table 6**.

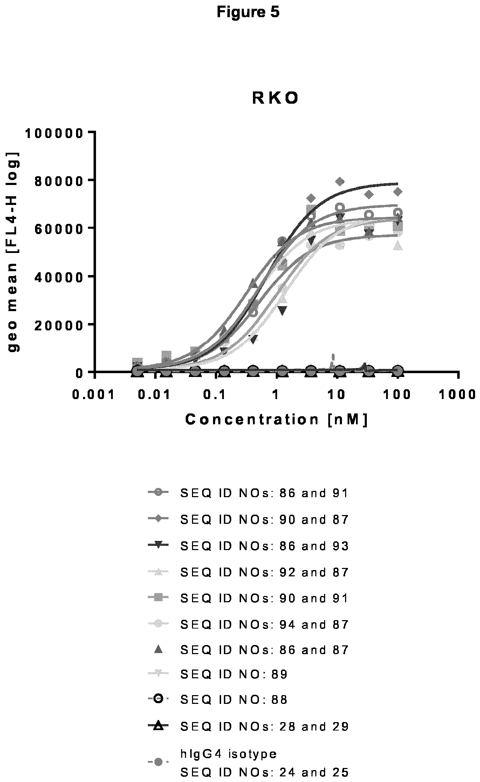

**Figure 5****:** shows the binding of fusion proteins to PD-L1-positive tumor cells evaluated using flow cytometry by incubating RKO cells and fusion proteins as described in **Example 7**.

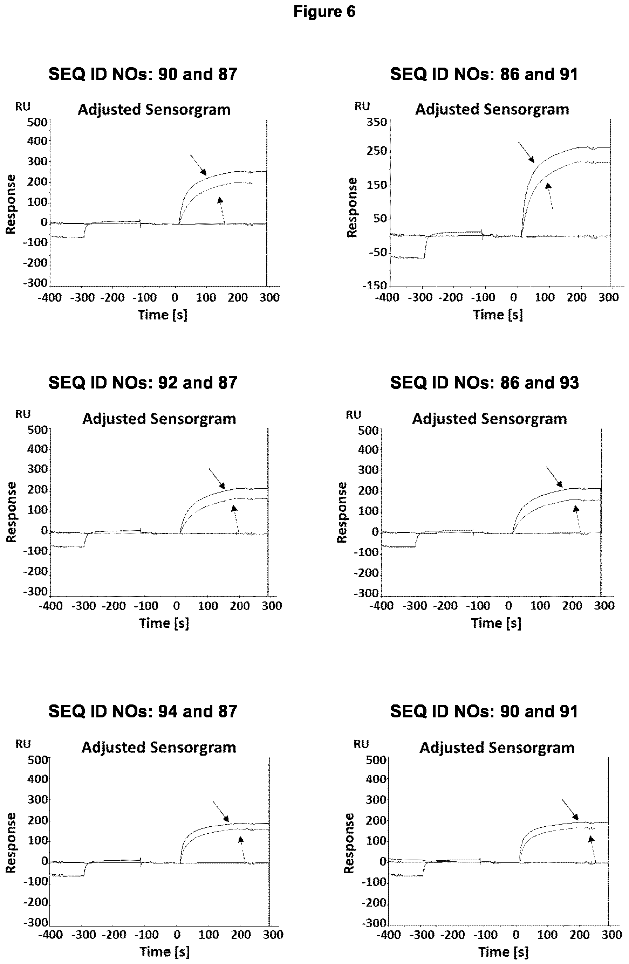

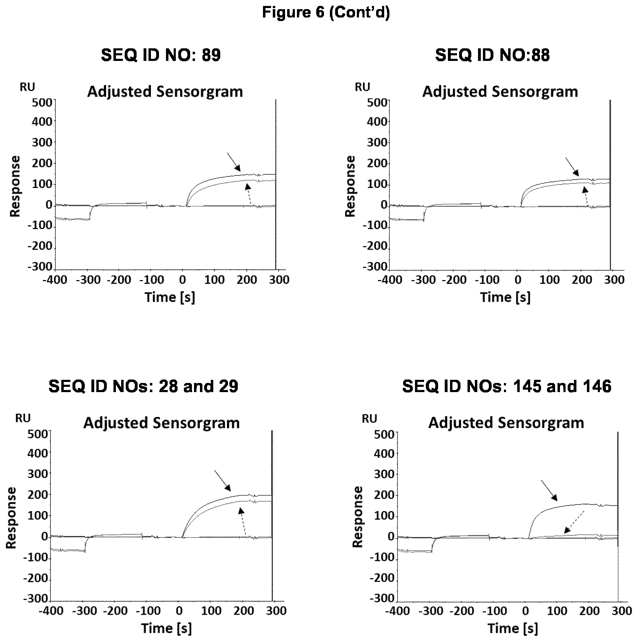

**Figure 6**: provides examples of a multi-binding SPR-based experiment designed to investigate whether the fusion protein interactions with CD137 are hampered by the binding of CD137L to CD137, as described in **Example 8**. This is assessed by generating a complex of huCD137 (C-terminal Fc fusion) and huCD137L (with a C-terminal His tag) on the SPR sensor chip, and checking whether the fusion proteins can still bind the complex of huCD137 and CD137L. As a reference, huCD137 in the absence of huCD137L is also incubated with the tested fusion proteins. The SPR trace for the binding of the respective fusion protein to huCD137 alone is marked with an arrow with a solid stem. The SPR trace for the binding of the respective fusion protein to huCD137 that has been saturated with huCD137L is marked with an arrow with a broken stem. As controls, blank injections without fusion proteins were used. The experiment shows that all tested fusion proteins were able to bind CD137 in the presence of CD137L.

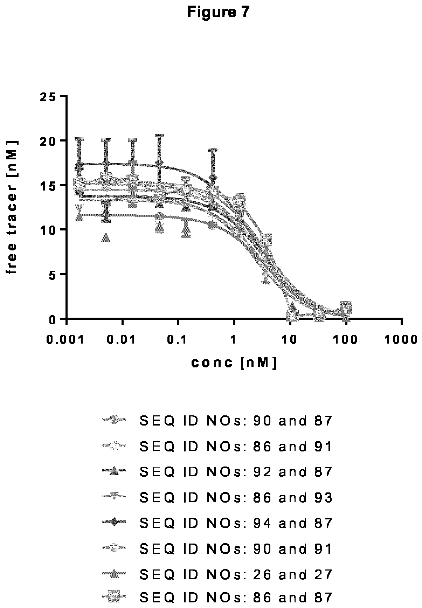

**Figure 7****:** shows that the fusion proteins compete with PD-L1 for binding to PD-1, depicted in competitive ELISA studies as described in **Example 9**. A constant concentration of huPD-1-His was coated on a microtiter plate, followed by adding a mixture of testing molecules at different concentrations and tracer huPD-L1-Fc at a fixed concentration. Bound tracer was detected using a HRP-labelled anti-IgG Fc antibody. The dose dependent inhibition of huPD-L1-Fc binding to PD-1 by the CD137 and PD-L1 bispecific fusion proteins or PD-L1 specific antibodies were observed.

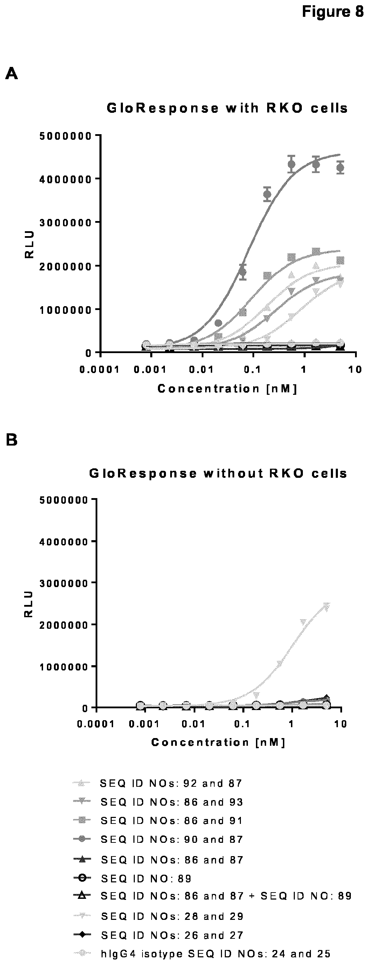

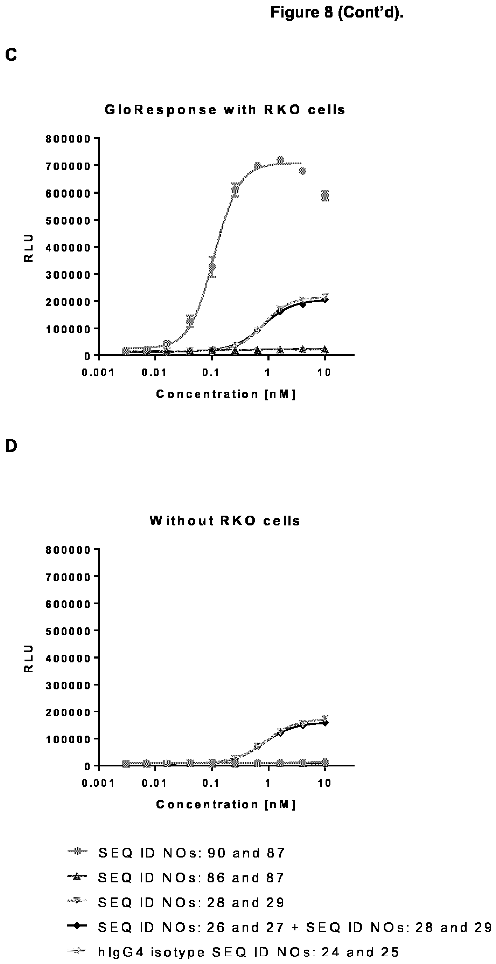

**Figure 8****:** The potential of representative fusion proteins to co-stimulate T-cell activation in a PD-L1-target-dependent manner was assessed using a CD137 Bioassay. NFκB-luc2/CD137 Jurkat cells were co-cultured with the PD-L1 expressing tumor cell line RKO in the presence of various concentrations of the fusion proteins or controls. After 4h, Luciferase assay reagent was added and luminescent signals were measured. Four-parameter logistic curve analysis was performed with GraphPad Prism^{®} to calculate EC₅₀ values (see **Table 9**). The fusion proteins only co-stimulate T-cell activation in the presence of PD-L1 (**Figure 8A** **and** **8C**) but not in the absence of PD-L1 (**Figure 8B** **and** **8D**). In contrast, the reference anti-CD137 mAb (SEQ ID NOs: 28 and 29) displays similar activation in the presence and absence of PD-L1 positive RKO cells.

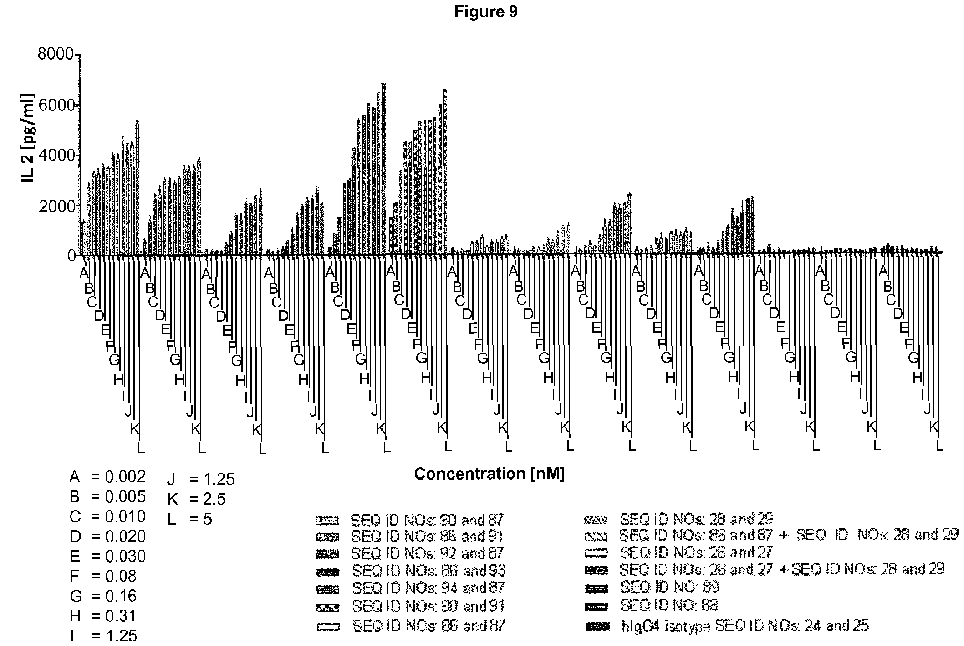

**Figure 9****:** shows the results of a representative experiment in which the ability of selected fusion proteins to induce T-cell activation was investigated. PD-L1 antibodies, including the respective PD-L1 antibody building block, CD137 binding lipocalin muteins as Fc fusions, and an anti-CD137 benchmark antibody were tested alone and in combination as an anti-PD-L1/anti-CD137 cocktail. In the experiment, human peripheral blood mononuclear cells (PBMCs) were incubated with the fusion proteins, antibodies, lipocalin mutein Fc fusions, cocktails, or control in the presence of 1 ng/mL staphylococcal enterotoxin B (SEB). Levels of secreted interleukin 2 (IL-2), reflective of T-cell activation, were determined by an electrochemoluminescence-based assay as readout for T-cell activation and normalized to the levels of corresponding IgG4 control, as described in **Example 11**. All fusion proteins are capable of inducing T-cell activation, and more strongly or at least comparable to single building blocks or a cocktail of benchmark anti-PD-L1/anti-CD137 antibodies.

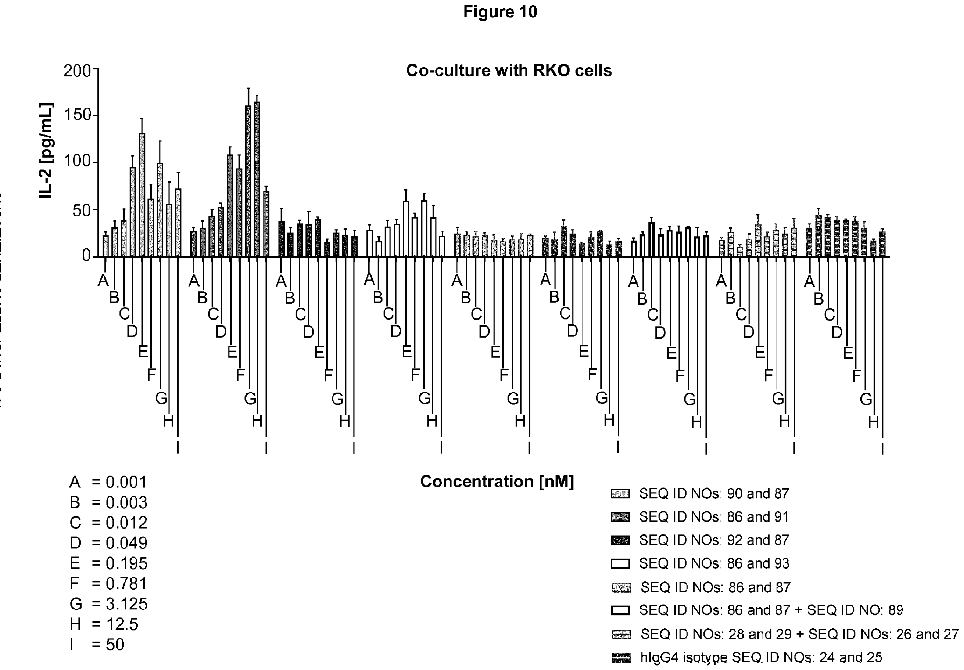

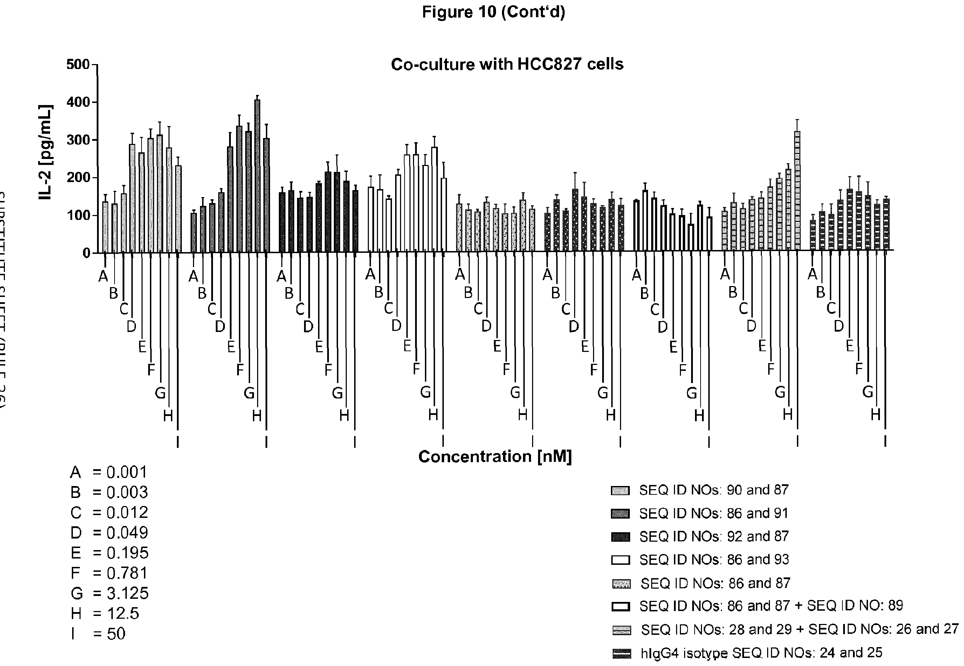

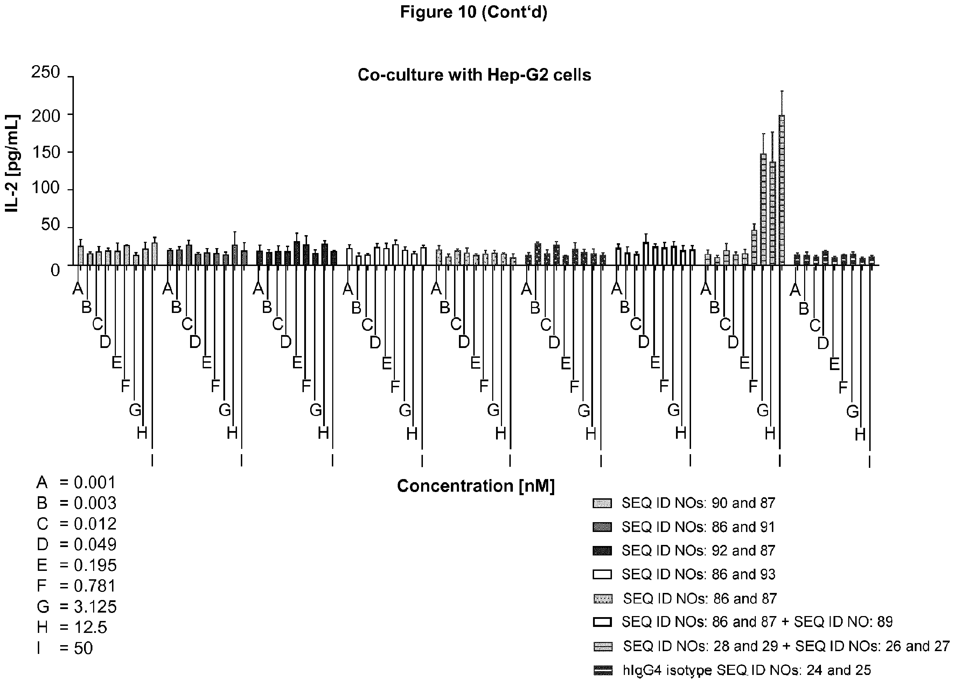

**Figure 10****:** shows the ability of representative fusion proteins to co-stimulate T-cell activation in a PD-L1-target-dependent manner. PD-L1 antibodies, including the respective PD-L1 antibody building block, CD137 binding lipocalin muteins as Fc fusions, and an anti-CD137 benchmark antibody were tested alone and in combination as an anti-PD-L1/anti-CD137 cocktail. Various tumor cell lines expressing different PD-L1 levels (High: RKO; Moderate: HCC827; Negative: HepG) were seeded into anti-human CD3 coated plates. Pan T cells and various concentrations of fusion proteins and single building blocks were added and incubated for 3 days. Levels of secreted IL-2 were determined by an electrochemoluminescence-based assay, as described in **Example 12**. All fusion proteins are capable of increasing IL-2 secretion in a PD-L1 dependent manner.

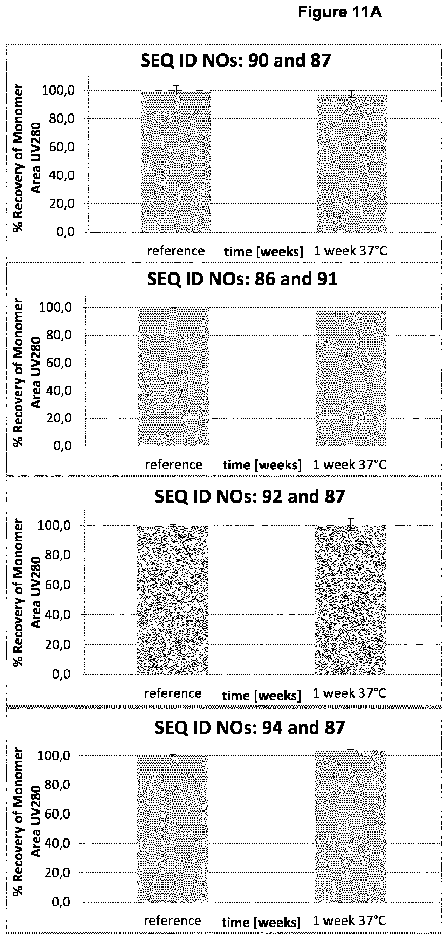

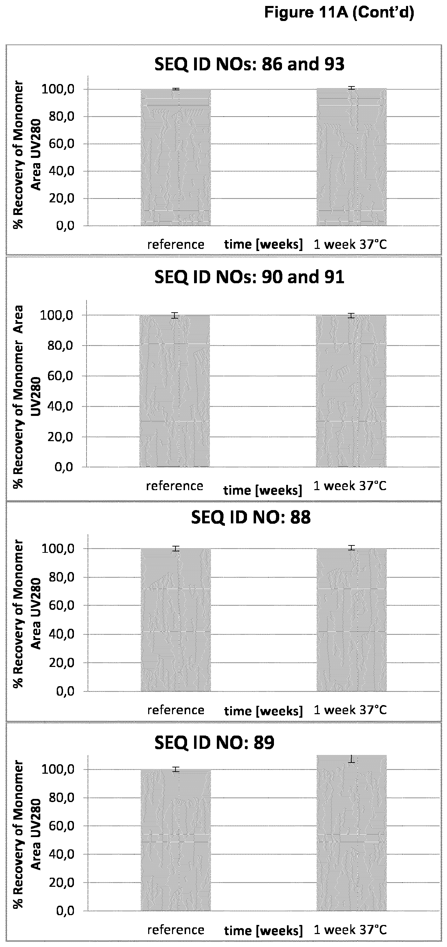

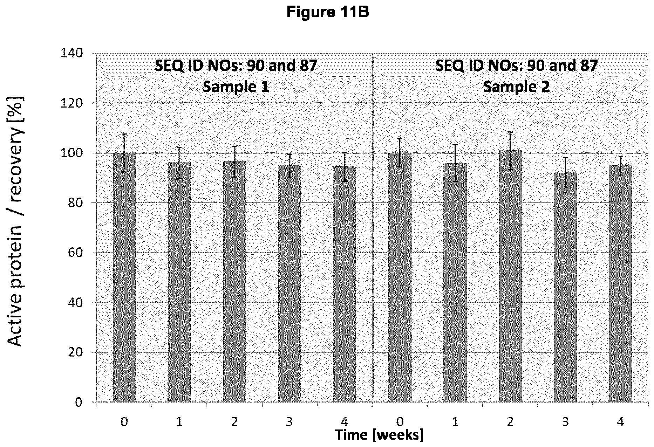

**Figure 11****:** illustrates the storage stability of fusion proteins in PBS or 25 mM histidine, 60 mM NaCl, 200 mM arginine pH 6 after 1-, 2-, 3-, or 4-week incubation at 37°C or 40 °C at the concentration of 1mg/ml or 20 mg/mL. The stability is assessed by the recovery of monomers from analytical size exclusion or by recovery of functional proteins from quantitative ELISA, as described in **Example 13.**

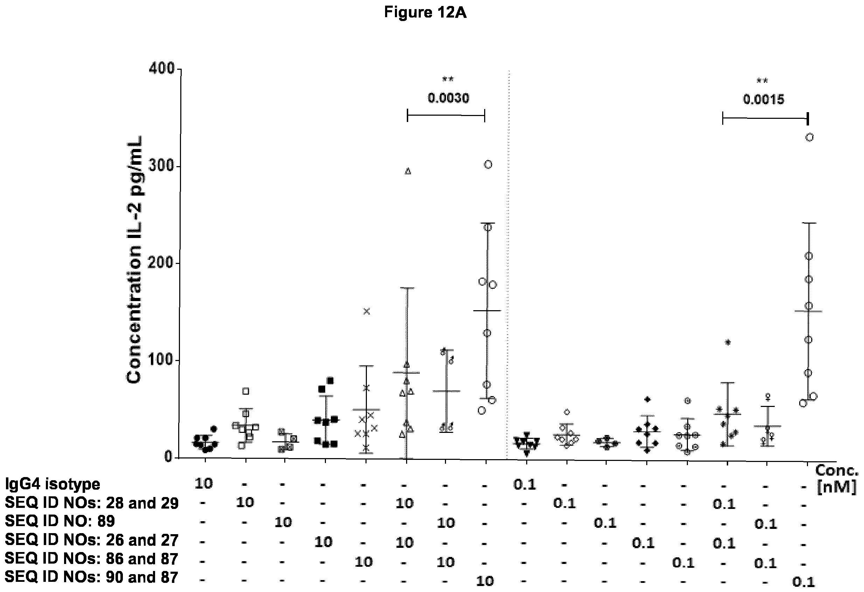

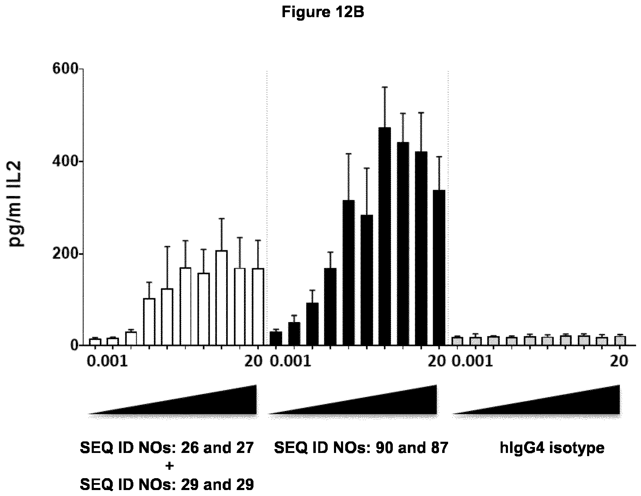

**Figure 12****:** shows the ability of a representative fusion protein (SEQ ID NOs: 90 and 87) to stimulate IL-2 secretion in a mixed lymphocyte reaction (MLR) with CD4⁺ T cells. The fusion proteins, the PD-L1 antibody building block (SEQ ID NOs: 86 and 87), the CD137 binding lipocalin mutein as an Fc fusion (SEQ ID NO: 89), and an anti-CD137 benchmark antibody or anti-PD-L1 benchmark antibody alone or in combination as an anti-PD-L1/anti-CD137 cocktail, were tested at equimolar concentrations, as described in **Example 14.** IL-2 secretion was measured in the supernatants after 6 days of incubation with total human CD4⁺ T cells and monocyte derived dendritic cells (moDCs) from different healthy donors. **Figure 12A** illustrates that the fusion protein SEQ ID NOs: 90 and 87 showed significant increase in IL-2 secretion as compared to the building block alone (the PD-L1 antibody SEQ ID NOs: 86 and 87 or CD137 specific lipocalin mutein SEQ ID NO: 89) and a reference anti-PD-L1 or anti-CD137 antibody (SEQ ID NOs: 26 and 27 or SEQ ID NOs: 28 and 29, respectively), at equimolar concentrations (equivalent to 10 or 0.1 µg/ml of the tested fusion protein). Data from 8 independent experiments are shown. **Figure 12B** shows the fusion protein SEQ ID NOs: 90 and 87 was able to induce a dose-dependent secretion of IL-2, over concentrations ranging from 0.001 to 20 µg/mL. The IL-2 levels induced by the fusion protein were higher as compared to equimolar concentrations of the cocktail of a reference anti-PD-L1 antibody (SEQ ID NOs: 26 and 27) and a reference anti-CD137 antibody (SEQ ID NOs: 28 and 29). Data from a representative donor are shown.

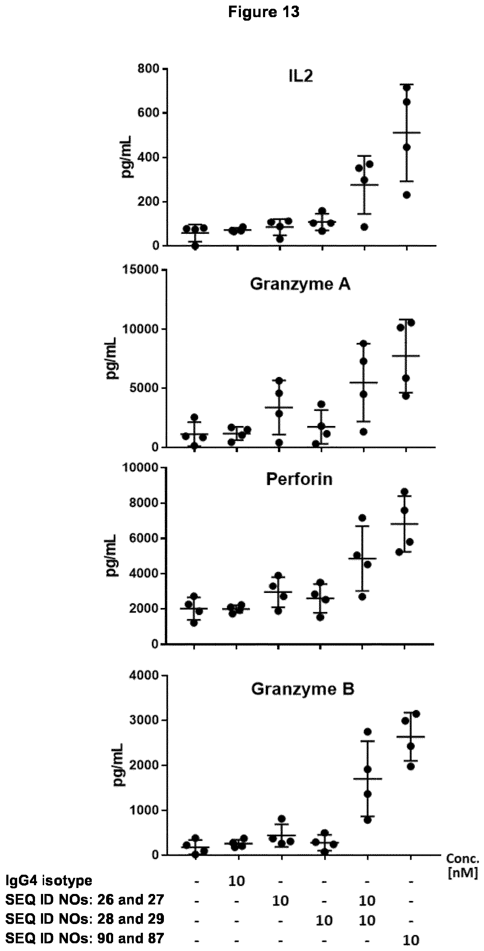

**Figure 13****:** shows the ability of an exemplary fusion protein (SEQ ID NOs: 90 and 87) to induce secretion of CD8⁺ T-cell effector molecules. The fusion protein was cultured with moDCs and CD8⁺ T cells from mismatching healthy donors for 6 days, after which the secretion of IL-2 and CD8⁺ T-cell effector molecules in the supernatants were quantified using a Luminex assay as described in **Example 15.** The fusion protein SEQ ID NOs: 90 and 87 showed increase in the secretion of IL-2 and cytotoxic factors (perforin, granzyme B, and granzyme A) at 10 µg/mL, as compared to the reference anti-PD-L1 antibody (SEQ ID NOs: 26 and 27) and reference anti-CD137 antibody (SEQ ID NOs: 28 and 29) when used alone or as a cocktail.

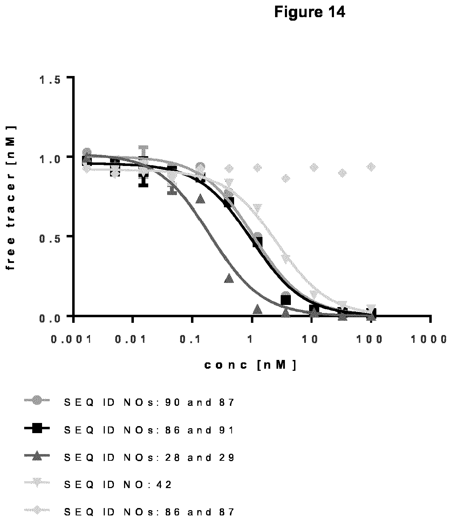

**Figure 14****:** shows that the fusion proteins bind overlapping epitopes with a clinically active CD137 antibody (SEQ ID NOs: 28 and 29), depicted in the competitive ELISA studies as described in **Example 17**. A constant concentration of SEQ ID NOs: 28 and 29 was coated on a microtiter plate, followed by adding a mixture of testing molecules at different concentrations and the tracer of biotinylated huCD137-Fc at a fixed concentration. Bound tracer was detected via ExtrAvidin-Peroxidase. The fusion proteins compete with the CD137 antibody for CD137 binding.

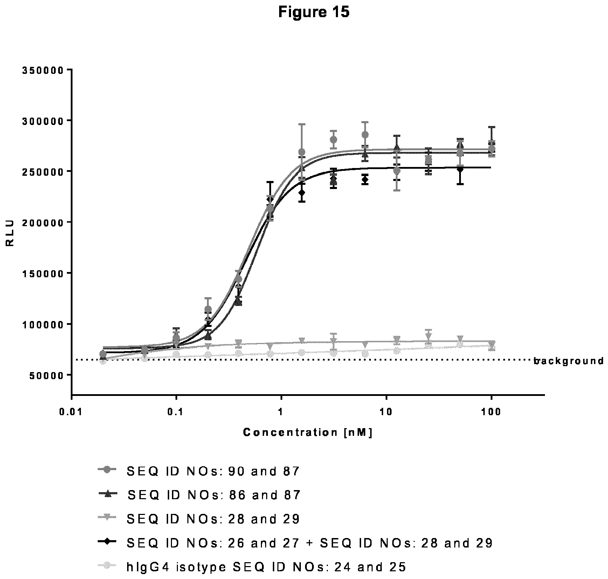

**Figure 15****:** shows the potential of representative fusion proteins to block the inhibitory signal mediated by PD-1/PD-L1 interaction, evaluated using a PD-1/PD-L1 blockade bioassay as described in **Example 18**. PD-1-NFAT-luc Jurkat T cells (a Jurkat cell line expressing PD-1 and a NFAT-mediated luciferase gene under the NFAT promoter control) were co-cultured with PD-L1 aAPC/CHO-K1 cells in presence of various concentrations of testing molecules. After 6 hours, luciferase assay reagent was added and luminescent signals measured. Background signal is PD-1-NFAT-luc Jurkat T cells co-cultured with only PD-L1 aAPC/CHO-K1 cells. The fusion protein of SEQ ID NOs: 90 and 87 blocks the PD-1/PD-L1 pathway, comparable to tested PD-L1 antibodies, including the building block PD-L1 antibody shown in SEQ ID NOs: 86 and 87 and the reference PD-L1 antibody shown in SEQ ID NOs: 26 and 27.

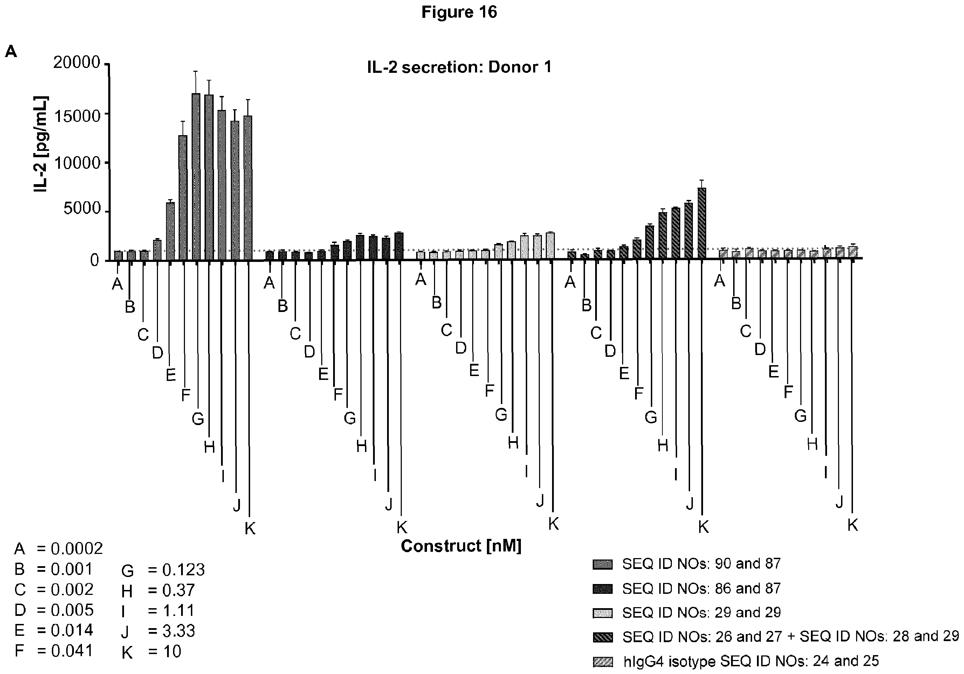

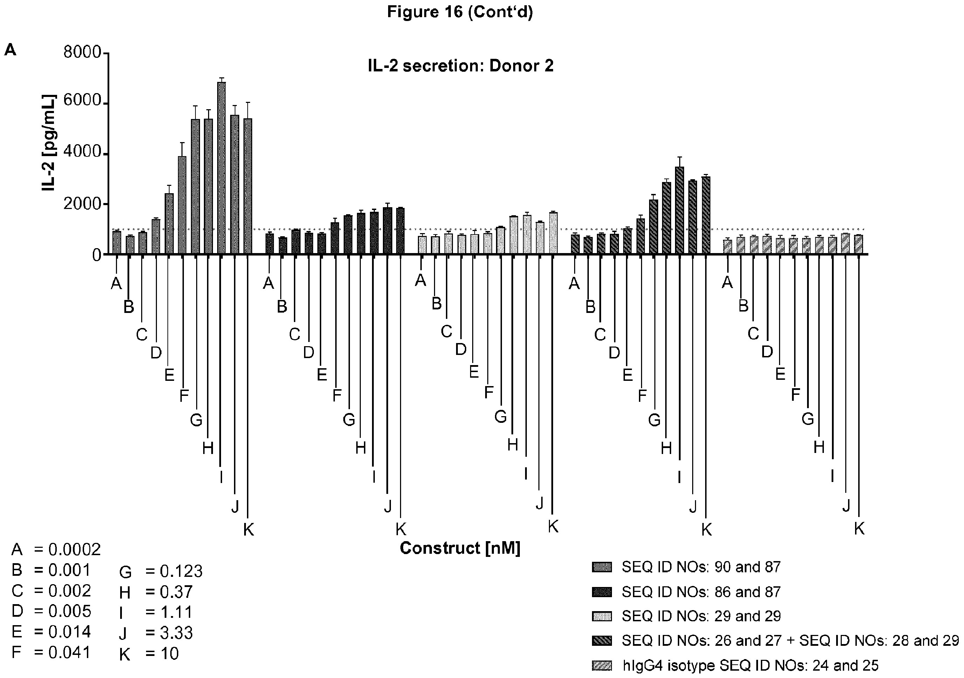

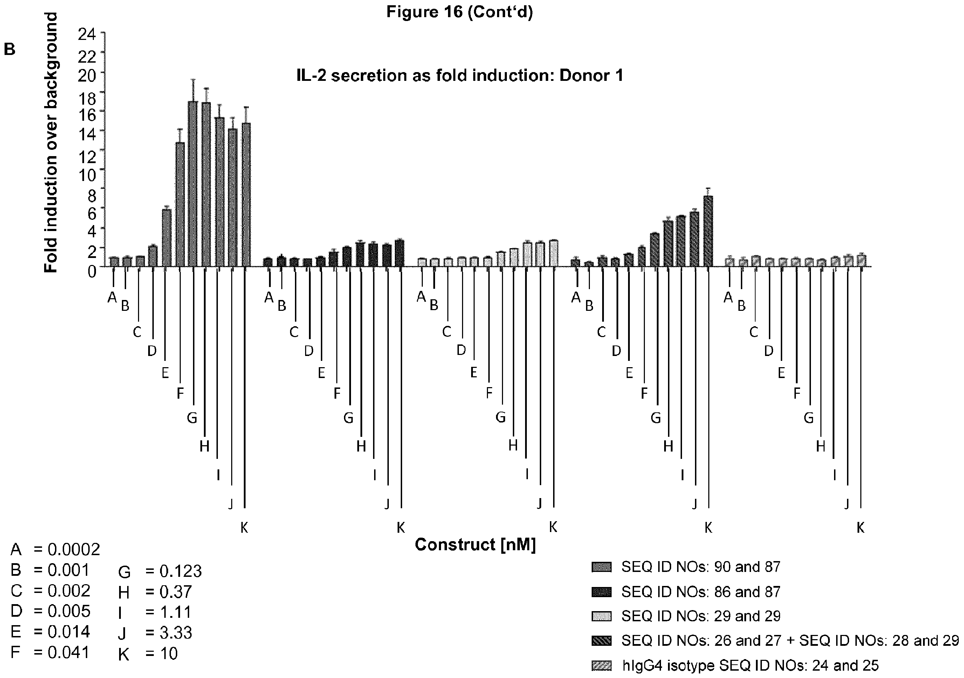

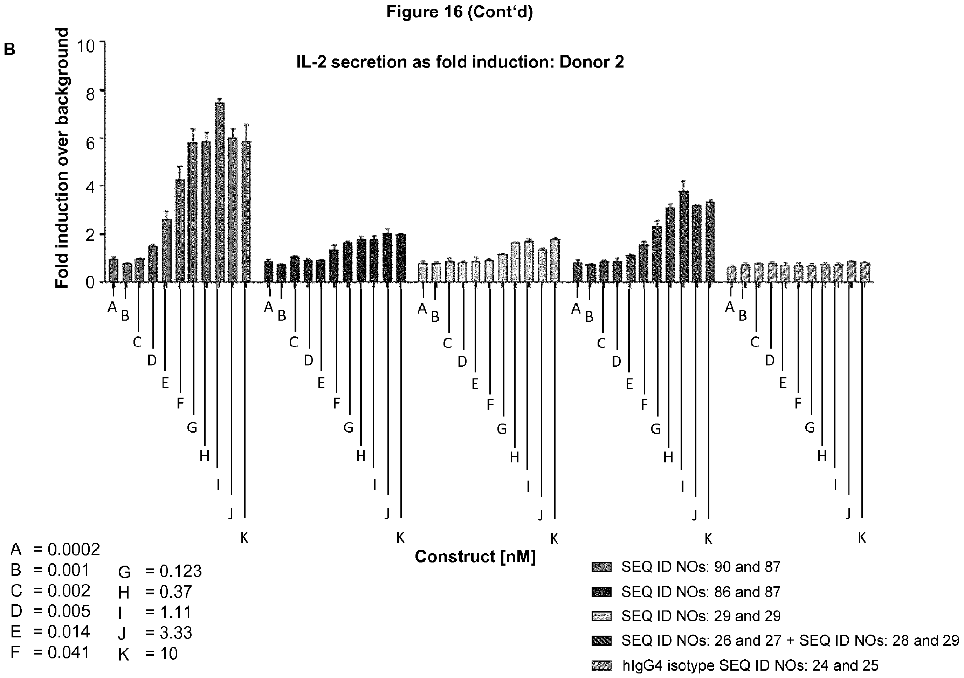

**Figure 16****:** shows the ability of a representative fusion protein to induce T-cell activation. The PD-L1 antibody building block and a reference CD137 antibody, when used alone and in combination with a PD-L1 antibody, were tested as well. In the experiment, human PBMCs were incubated with the fusion protein, antibodies, cocktails, or control in the presence of 0.1 ng/mL SEB. Levels of secreted IL-2 were determined by an electrochemoluminescence-based assay as readout for T-cell activation, as described in **Example 19 and** depicted in **Figure 16A****.** **Figure 16B** displays the fold increase in IL-2 secretion levels induced by the testing molecules when compared to the level of background IL-2 secretion (PBMCs stimulated with 0.1 ng/mL SEB and without any testing molecules). The fusion protein leads to a dose-dependent increase in IL-2 secretion, which is stronger than a PD-L1 antibody or a CD137 antibody alone or in combination.

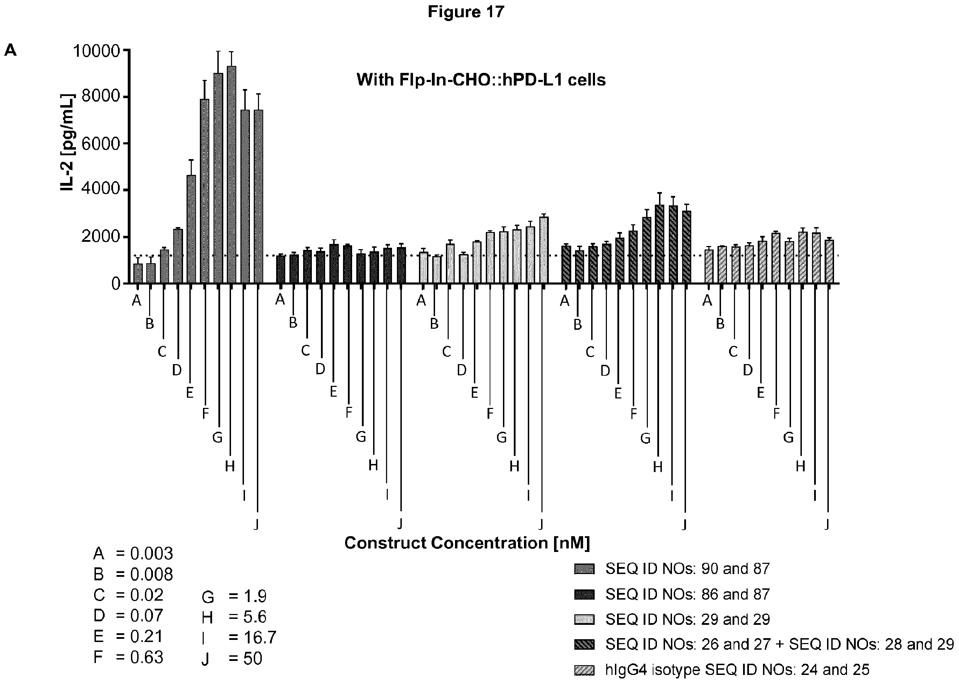

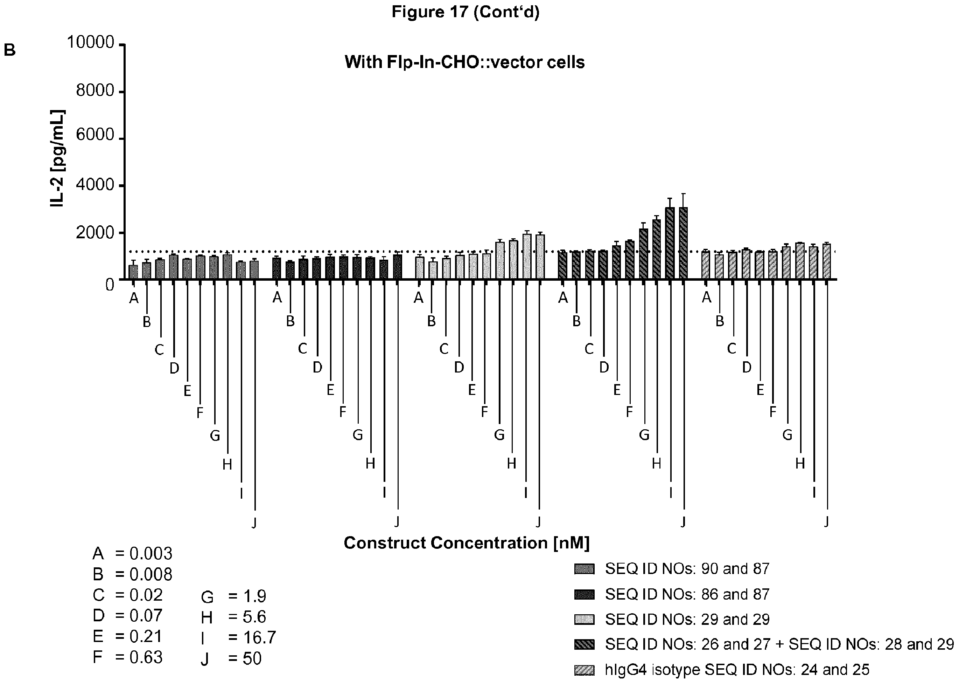

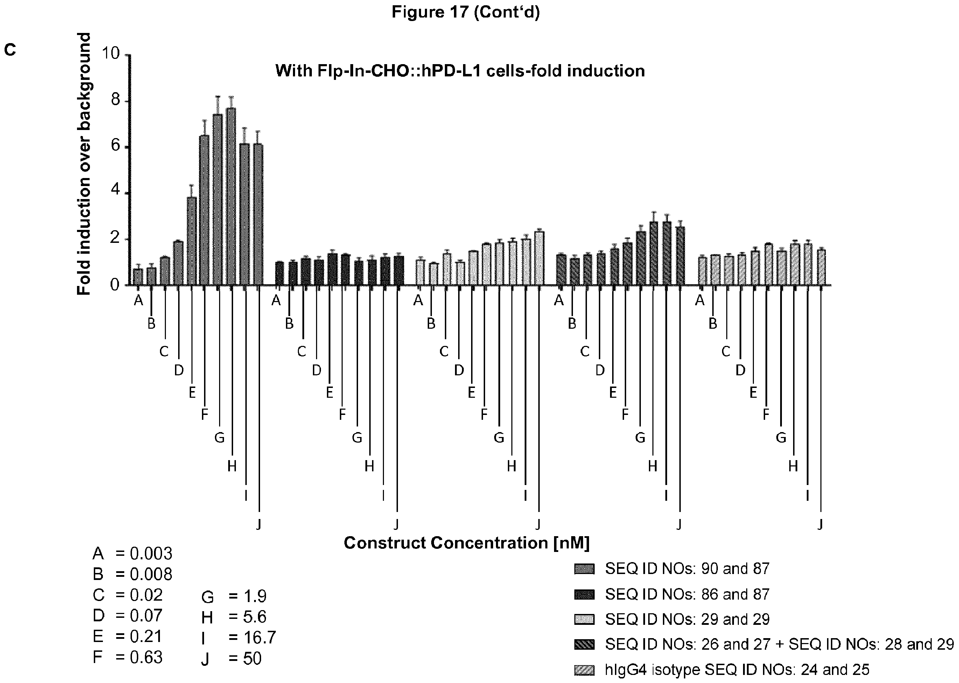

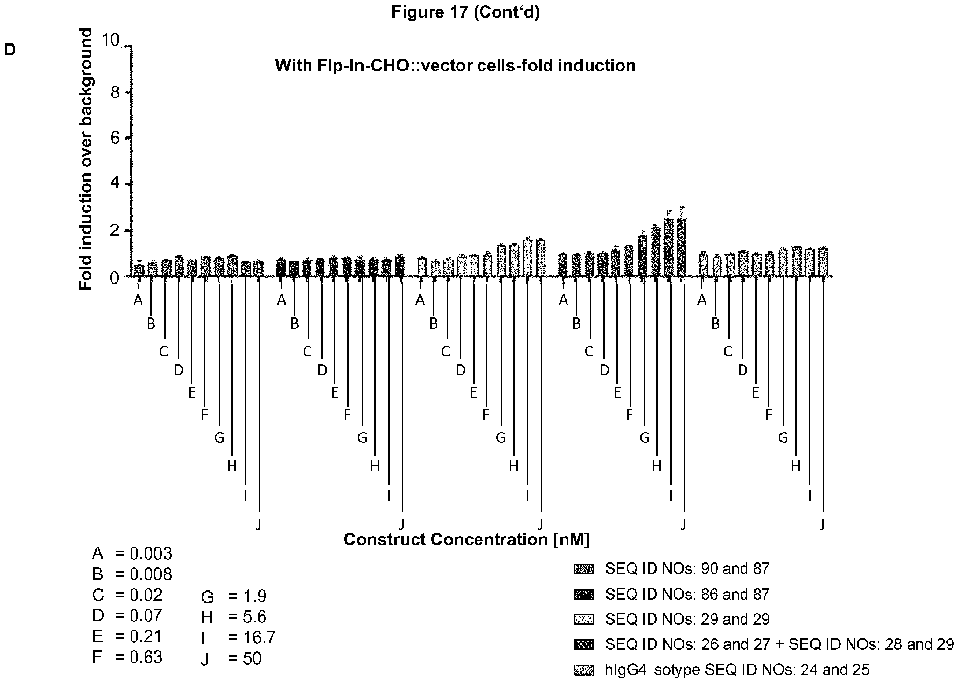

**Figure 17****:** demonstrates the ability of a representative fusion protein to co-stimulate T-cell activation in the presence of PD-L1. The PD-L1 antibody building block, a reference CD137 antibody, and a cocktail of the CD137 antibody and a reference PD-L1 antibody were tested in parallel. CHO cells either transfected with human PD-L1 (**Figure 17A**) or mock transfected (human PD-L1 negative, **Figure 17B**) were seeded into anti-human anti-CD3 coated plates. Pan T cells as well as various concentrations of testing molecules were added and incubated for 2 days. Levels of secreted IL-2 in the supernatant were determined by an electrochemoluminescence-based assay, as described in **Example 20.** The IL-2 secretion levels were normalized to background levels (Pan T cells + anti-CD3 + CHO cells) to depict the fold increase in IL-2 secretion in the presence of human PD-L1 expressing CHO cells (**Figure 17C**) or mock transfected CHO cells (**Figure 17D**). The fusion protein induces a strong dose-dependent increase in IL-2 secretion only in the presence of PD-L1, more strongly than the reference CD137 antibody alone or in combination with the reference PD-L1 antibody.

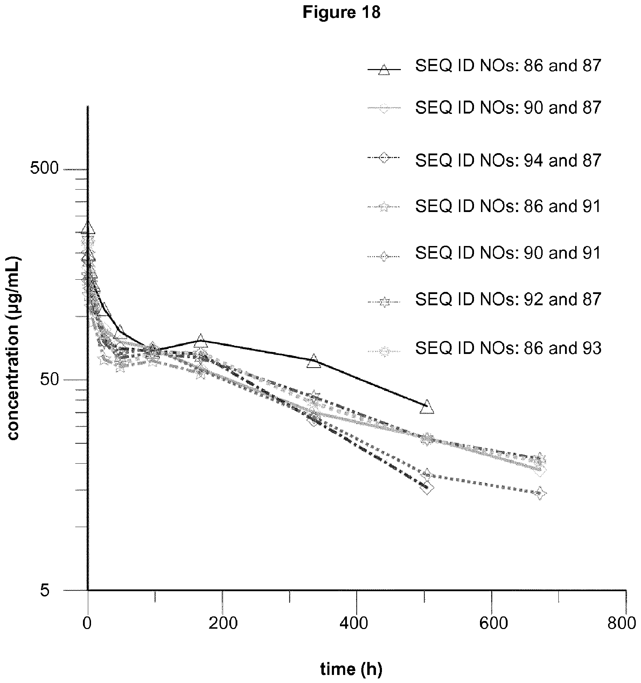

**Figure 18****:** provides the result of pharmacokinetic analyses of the bispecific fusion proteins and the building block PD-L1 antibody (SEQ ID NOs: 86 and 87) in mice, as described in **Example 21.** Male CD-1 mice (3 mice per timepoint) were injected intravenously with fusion proteins at a dose of 10mg/kg. Drug levels were detected using a Sandwich ELISA detecting the full molecule via the targets PD-L1 and CD137. The anti-PD-L1 antibody plasma levels were determined using a Sandwich ELISA with targets PD-L1 and human Fc.

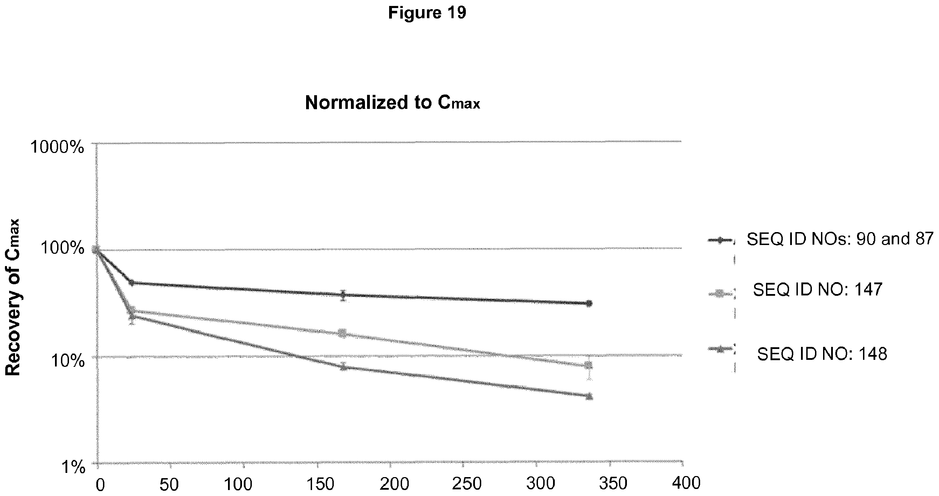

**Figure 19****:** provides the results of a pharmacokinetic analysis of a representative fusion protein (SEQ ID NOs: 90 and 87) in comparison with two previously described CD137- and PD-L1-binding fusion proteins (SEQ ID NO: 147 and SEQ ID NO: 148) in mice as described in **Example 22.** Male CD-1 mice (2 mice per timepoint) were injected intravenously with testing molecules at a dose of 2 mg/kg. Drug levels were detected using an ELISA at the indicated time points. The data were plotted in a time vs. concentration graph. SEQ ID NOs: 90 and 87 described herein, but not SEQ ID NO: 147 or SEQ ID NO: 148, displays a favorable pharmacokinetic profile or antibody-like pharmacokinetics.

### IV. DETAILED DESCRIPTION OF THE DISCLOSURE

As is described herein, the present disclosure encompasses the recognition that a bivalent CD137-binder, such as an antibody, may not be sufficient by itself to cluster CD137 on T cells or NK cells and lead to efficient activation, similar to the lack of activity of the trivalent soluble CD137L. In recent publications utilizing preclinical mouse models, *in vivo* evidence has been presented that the mode of action of other anti-TNFR antibodies requires the interaction of the antibodies via their Fc-part with Fc-gamma receptors on Fc-gamma-receptor expressing cells (Bulliard et al., Immunol Cell Biol, 2014, Bulliard et al., J Exp Med, 2013). The mode of action of these anti-TNFR antibodies may therefore be dominated by a non-targeted clustering via Fc-gamma receptors, depending on the presence of Fc-gamma receptor-expressing cells, which may not necessarily overexpress in the targeted tumor microenvironment as compared to normal tissues.

Thus, there is unmet need for the generation of therapeutics that cluster and activate CD137 with a specific, tumor-targeted mode of action.

To meet this unmet need, the present disclosure provides, among other things, novel approaches for simultaneously engaging CD137 and PD-L1 via one or more fusion proteins having binding specificity for CD137 and binding specificity for PD-L1. Provided fusion proteins are designed to promote CD137 clustering by bridging CD137-positive T cells with PD-L1 expressed in the tumor microenvironment. Such bispecific molecules may combine CD137-induced T-cell activation and expansion with anti-PD-L1 mediated immune checkpoint blockade and thus may overcome certain limitations of single agent therapy and offer benefits to, for example, resistant or non-responsive patients. The fusion proteins are also designed to provide potentials of a combinatorial therapy in one molecule and at the same time allow the localized induction of antigen-specific T cells in the tumor microenvironment, potentially reducing peripheral toxicity.

In some aspects, the present disclosure provides fusion proteins that bind CD137 and PD-L1, as well as methods and useful applications therefor. The disclosure also provides methods of making CD137 and PD-L1 binding fusion proteins described herein as well as compositions comprising such proteins. CD137 and PD-L1 binding fusion proteins of the disclosure as well as compositions thereof may be used in methods of detecting CD137 and/or PD-L1 in a sample, in methods of binding of CD137 and/or PD-L1 in a subject, or in methods of modulating immune responses in a subject. No such fusion proteins having these features attendant to the uses provided by present disclosure have been previously described. In contrast to fusion proteins provided herein, previously known fusion proteins targeting both CD137 and PD-L1 suffered from one or more of poor pharmacokinetics, an unacceptable degree of off-target binding, reduced or otherwise degraded ability to bind to one or both of the targets of a particular fusion protein (e.g., PD-L1 and/or CD137), and/or an unacceptable degree of non-specific (e.g., PD-L1 independent) activation of e.g., the immune system.

### A. Exemplary fusion proteins specific for CD137 and PD-L1 of the disclosure.

The present invention relates to a fusion protein that is capable of binding both CD137 and PD-L1, wherein the fusion protein comprises two subunits, wherein the first subunit comprises a full-length immunoglobulin that is specific for PD-L1, and wherein the second subunit comprises a lipocalin mutein that is specific for CD137, wherein the fusion protein comprises amino acid sequences having at least 90% sequence identity to the amino acid sequences shown in SEQ ID NOs: 90 and 87, wherein the heavy chain of the immunoglobulin comprises the following set of CDR sequences: GFSLSNYD (HCDR1, SEQ ID NO: 60), IWTGGAT (HCDR2, SEQ ID NO: 61), and VRDSNYRYDEPFTY (HCDR3, SEQ ID NO: 62) and the light chain of the immunoglobulin comprises the following set of CDR sequences: QSIGTN (LCDR1, SEQ ID NO: 63), YAS (LCDR2), and QQSNSWPYT (LCDR3, SEQ ID NO: 64), and wherein the amino acid sequence of the lipocalin mutein comprises the following set of mutated amino acid residues in comparison with the linear polypeptide sequence of mature hNGAL (as shown in SEQ ID NO: 2): Gln 28 → His; Leu 36 → Gln; Ala 40 → Ile; lie 41 → Arg; Gln 49 → lie; Tyr 52 → Met; Asn 65 → Asp; Ser 68 → Met; Leu 70 → Lys; Arg 72 → Asp; Lys 73 → Asp; Asp 77 → Met; Trp 79 → Asp; Arg 81 → Trp; Cys 87 → Ser; Asn 96 → Lys; Tyr 100 → Phe; Leu 103 → His; Tyr 106 → Ser; Lys 125 → Phe; Ser 127 → Phe; Tyr 132 → Glu; and Lys 134 → Tyr. A provided fusion protein as set out in the appended set of claims contains at least two subunits: (1) a first subunit that comprises a full-length immunoglobulin specific for PD-L1, and (2) a second subunit that comprises a lipocalin mutein specific for CD137.

In some embodiments, at least one subunit can be linked to another subunit via a linker. In some further embodiments, a linker is a peptide linker, for example, an unstructured glycine-serine (GS) linker, a glycosylated GS linker, or a proline-alanine-serine polymer (PAS) linker. In some embodiments, a GS linker is a (Gly₄Ser)₃ linker ((G₄S)₃) as shown in SEQ ID NO: 13. Other exemplary linkers are shown in SEQ ID NOs: 14-23. In some embodiments, a peptide linker may have from 1 to 50 amino acids, such as 1, 2, 3, 4, 5, 10, 11, 12, 13, 14, 15, 16, 17 18, 19, 20, 25, 30, 35, 40, 45 or 50 amino acids. For example, when a first subunit comprises a full-length immunoglobulin, a second subunit may be linked via a peptide linker between the N-terminus of the second subunit and the C-terminus of a heavy chain constant region (CH) of said immunoglobulin.

Generally, one subunit may be fused at its N-terminus and/or its C-terminus to another subunit.

In some embodiments, with respect to a fusion protein of the disclosure, wherein at least one subunit may be or comprise a full-length immunoglobulin, the Fc function of the Fc region of the full-length immunoglobulin to Fc receptor-positive cell may be preserved at the same time while the fusion protein is simultaneously engaging CD137 and PD-L1.

In some embodiments, wherein at least one subunit of a provided fusion protein may be or comprise a full-length immunoglobulin, the Fc function of the Fc region of the full-length immunoglobulin to Fc receptor-positive cell may be reduced or fully suppressed by protein engineering while the fusion protein is simultaneously engaging CD137 and PD-L1. In some embodiments, this may be achieved, for example, by switching from the IgG1 backbone to IgG4, as IgG4 is known to display reduced Fc-gamma receptor interactions compared to IgG1. In some embodiments, to further reduce the residual binding to Fc-gamma receptors, mutations may be introduced into the IgG4 backbone such as F234A and L235A. In some embodiments, an S228P mutation may also be introduced into the IgG4 backbone to minimize the exchange of IgG4 half-antibody (Silva et al., J Biol Chem, 2015). In some embodiments, F234A and L235A mutations may be introduced for decreased ADCC and ADCP (Glaesner et al., Diabetes Metab Res Rev, 2010) and/or M428L and N434S mutations or M252Y, S254T, and T256E mutations for extended serum half-life (Dall'Acqua et al., J Biol Chem, 2006, Zalevsky et al., Nat Biotechnol, 2010). In some embodiments, an additional N297A mutation may be present in the immunoglobulin heavy chain of the fusion protein in order to remove the natural glycosylation motif.

In some embodiments, the Fc portion of an immunoglobulin included in a fusion protein of the disclosure may contribute to maintaining the serum levels of the fusion protein. For example, when the Fc portion binds to Fc receptors on endothelial cells and phagocytes, the fusion protein may become internalized and recycled back to the bloodstream, enhancing its half-life within the body.

In one aspect, fusion proteins of the disclosure bind CD137 with high affinity. In another aspect, provided fusion proteins bind PD-L1 with high affinity. In some preferred embodiments, provided fusion proteins simultaneously bind CD137 and PD-L1. In some embodiments, the simultaneous binding to CD137 and PD-L1 allows provided fusion proteins to exhibit a durable anti-tumor or anti-infection response.

In some embodiments, a provided fusion protein comprises the amino acid sequences shown in SEQ ID NOs: 90 and 87.

In some embodiments, a provided fusion protein comprises the amino acid sequences having at at least 92%, at least 95%, at least 97%, at least 98%, or even higher sequence identity to the amino acid sequences shown in SEQ ID NOs: 90 and 87.

### B. Exemplary immunoglobulins as included in the fusion proteins.

A fusion protein as set out in the appended set of claims comprises a first subunit comprising a full-length immunoglobulin specific for PD-L1. In some embodiments, an immunoglobulin, for example, may be IgG1, IgG2 or IgG4. In some embodiments, an immunoglobulin is or comprises IgG4.

Illustrative examples of PD-L1-binding antibodies of the disclosure may comprise an antigen-binding region which cross-blocks or binds to the same epitope as a PD-L1-binding antibody comprising the heavy chain variable domain (V_{H}) and light chain variable domain (V_{L}) regions of a known antibody such as atezolizumab (also known as MPDL3280A or RG7446, trade name Tecentriq^{®}), avelumab (also known as MSB0010718C, trade name Bavencio^{®}), durvalumab (previously known as MEDI4736, trade name Imfinzi^{®}), and BMS-936559 (also known as MDX-1105), 5C10 (including humanized 5C10), 5F10 (including humanized 5F10), and 9F6 (including humanized 9F6).

In some embodiments, the heavy chain and light chain pair of a provided PD-L1 antibody or antigen-binding domain thereof are or comprise a HCVR and LCVR, respectively, as follows: SEQ ID NOs: 77 and 82.

In some embodiments, the heavy chain and light chain pair of a provided PD-L1 antibody are or comprise the amino acid sequences as shown in SEQ ID NO: 86 and 87.

The heavy chain variable region of a provided PD-L1 antibody has the three CDRs having following sequences: GFSLSNYD (HCDR1, SEQ ID NO: 60), IWTGGAT (HCDR2, SEQ ID NO: 61), VRDSNYRYDEPFTY (HCDR3; SEQ ID NO: 62).

The light chain variable region of a provided PD-L1 antibody has the three CDRs having following sequences: QSIGTN (LCDR1, SEQ ID NO: 63), YAS (LCDR2), QQSNSWPYT (LCDR3; SEQ ID NO: 64).

A provided PD-L1 antibody or antigen-binding domain thereof comprises a heavy chain variably region that has the three CDRs having following sequences: GFSLSNYD (HCDR1, SEQ ID NO: 60), IWTGGAT (HCDR2, SEQ ID NO: 61), VRDSNYRYDEPFTY (HCDR3; SEQ ID NO: 62), and a light chain variably region that has the three CDRs having following sequences: QSIGTN (LCDR1, SEQ ID NO: 63), YAS (LCDR2), QQSNSWPYT (LCDR3; SEQ ID NO: 64).

Unless otherwise indicated, all CDR sequences disclosed herein are defined according to the IMGT method as described in Lefranc, M.-P., The Immunologist, 7, 132-136 (1999). CDR1 consists of positions 27 to 38, CDR2 consists of positions 56 to 65, CDR3 for germline V-genes consists of positions 105 to 116, CDR3 for rearranged V-J-genes or V-D-J-genes consists of positions 105 to 117 (position preceding J-PHE or J-TRP 118) with gaps at the top of the loop for rearranged CDR3-IMGT with less than 13 amino acids, or with additional positions 112.1, 111.1, 112.2, 111.2, etc. for rearranged CDR3-IMGT with more than 13 amino acids. The positions given in this paragraph are according to the IMGT numbering described in Lefranc, M.-P., The Immunologist, 7, 132-136 (1999).

Antibodies specifically binding to PD-L1 as included in fusion proteins of the disclosure may comprise an Fc part which allows for extending the *in vivo* half-life of the bispecific binding molecule of the disclosure. In some embodiments, such Fc part is preferably from human origin, more preferably a human Fc part of an IgG1 or IgG4 antibody, even more preferably an engineered human Fc part of an IgG1 or IgG4 with activating or silencing effector functions. In some embodiments, silencing effector functions may be preferred over activating effector functions. In some embodiments, such an Fc part is an engineered to silence effector functions with mutation(s) at positions 234 and/or 235, numbering according to EU index of Kabat (Johnson and Wu, Nucleic Acids Res, 2000). In some embodiments, mutations in positions F234 and L235 of a provided anti-PD-L1 antibody may be introduced to silence effector functions. In other embodiments, mutations in positions D265 and P329 of a provided anti-PD-L1 antibody may be introduced to silence effector function. Numbering for both sets of these potential mutations is according to the EU index of Kabat (Shields et al., J Biol Chem, 2001).

Various techniques for the production of antibodies and fragments thereof are well known in the art and described, e.g., in Altshuler et al. (2010). Thus, for example, polyclonal antibodies can be obtained from the blood of an animal following immunization with an antigen in mixture with additives and adjuvants and monoclonal antibodies can be produced by any technique which provides antibodies produced by continuous cell line cultures. Examples of such techniques are described, e.g., Harlow and Lane (1999), (1988), and include the hybridoma technique originally described by Köhler and Milstein, 1975, the trioma technique, the human B cell hybridoma technique (see e.g. Li et al., Proc Natl Acad Sci USA, 2006, Kozbor and Roder, Immunol Today, 1983) and the EBV-hybridoma technique to produce human monoclonal antibodies (Cole et al., Cancer Res, 1984). Furthermore, recombinant antibodies may be obtained from monoclonal antibodies or can be prepared *de novo* using various display methods such as phage, ribosomal, mRNA, or cell display.

### C. Exemplary lipocalin muteins of the disclosure.

Lipocalins are proteinaceous binding molecules that have naturally evolved to bind ligands. Lipocalins occur in many organisms, including vertebrates, insects, plants, and bacteria. The members of the lipocalin protein family (Pervaiz and Brew, FASEB J, 1987) are typically small, secreted proteins and have a single polypeptide chain. They are characterized by a range of different molecular-recognition properties: their binding to various, principally hydrophobic small molecules (such as retinoids, fatty acids, cholesterols, prostaglandins, biliverdins, pheromones, tastants, and odorants), and their binding to specific cell-surface receptors and their formation of macromolecular complexes. Although they have, in the past, been classified primarily as transport proteins, it is now clear that the lipocalins fulfill a variety of physiological functions. These include roles in retinol transport, olfaction, pheromone signaling, and the synthesis of prostaglandins. Lipocalins have also been implicated in the regulation of the immune response and the mediation of cell homeostasis (reviewed, e.g., in Flower et al., Biochim Biophys Acta, 2000, Flower, Biochem J, 1996).

Lipocalins share unusually low levels of overall sequence conservation, often with sequence identities of less than 20%. In strong contrast, their overall folding pattern is highly conserved. The central part of the lipocalin structure consists of a single eight-stranded anti-parallel β-sheet closed back on itself to form a continuously hydrogen-bonded β-barrel. This β-barrel forms a central cavity. One end of the barrel is sterically blocked by the N-terminal peptide segment that runs across its bottom as well as three peptide loops connecting the β-strands. The other end of the β-barrel is open to the solvent and encompasses a target-binding site, which is formed by four flexible peptide loops (AB, CD, EF, and GH). It is the diversity of the loops in the otherwise rigid lipocalin scaffold that gives rise to a variety of different binding modes each capable of accommodating targets of different size, shape, and chemical character (reviewed, e.g., in Skerra, Biochim Biophys Acta, 2000, Flower et al., Biochim Biophys Acta, 2000, Flower, Biochem J, 1996).

A lipocalin mutein according to the present disclosure may be a mutein of any lipocalin. Examples of suitable lipocalins (also sometimes designated as "reference lipocalin," "wild-type lipocalin," "reference protein scaffolds," or simply "scaffolds") of which a mutein may be used include, but are not limited to, tear lipocalin (lipocalin-1, Tlc, or von Ebner's gland protein), retinol binding protein, neutrophil lipocalin-type prostaglandin D-synthase, β-lactoglobulin, bilin-binding protein (BBP), apolipoprotein D (APOD), neutrophil gelatinase-associated lipocalin (NGAL), α2-microglobulin-related protein (A2m), 24p3/uterocalin (24p3), von Ebner's gland protein 1 (VEGP 1), von Ebner's gland protein 2 (VEGP 2), and Major allergen Can f 1 (ALL-1).

The amino acid sequence of a lipocalin mutein according to the disclosure may have a high sequence identity as compared to the reference (or wild-type) lipocalin from which it is derived, for example, hTlc or hNGAL, when compared to sequence identities with another lipocalin (see also above). In this general context the amino acid sequence of a lipocalin mutein according to the disclosure is at least substantially similar to the amino acid sequence of the corresponding reference (wild-type) lipocalin, with the proviso that there may be gaps (as defined herein) in an alignment that are the result of additions or deletions of amino acids.

Typically, a lipocalin mutein contains one or more mutated amino acid residues - relative to the amino acid sequence of the wild-type or reference lipocalin, for example, hTlc and hNGAL - in the four loops at the open end that comprise a ligand-binding pocket and define the entrance of ligand-binding pocket (cf. above). As explained above, these regions are essential in determining the binding specificity of a lipocalin mutein for the desired target.

### D. Exemplary CD137-specific lipocalin muteins of the disclosure.

As noted above, a lipocalin is a polypeptide defined by its supersecondary structure, namely cylindrical β-pleated sheet supersecondary structural region comprising eight β-strands connected pair-wise by four loops at one end to define thereby a binding pocket. The present disclosure is not limited to lipocalin muteins specifically disclosed herein. In this regard, the disclosure relates to a lipocalin mutein having a cylindrical β-pleated sheet supersecondary structural region comprising eight β-strands connected pair-wise by four loops at one end to define thereby a binding pocket, wherein at least one amino acid of each of at least three of said four loops has been mutated and wherein said lipocalin is effective to bind CD137 with detectable affinity.

In one aspect, the present disclosure includes any number of lipocalin muteins derived from a reference (wild-type) lipocalin, preferably derived from mature hTIc or mature hNGAL, that bind CD137 with detectable affinity. In a related aspect, the disclosure includes various lipocalin muteins that are capable of activating the downstream signaling pathways of CD137 by binding to CD137. In this sense, CD137 can be regarded as a non-natural target of the reference (wild-type) lipocalin, preferably hTIc or hNGAL, where "non-natural target" refers to a substance that does not bind to the reference (wild-type) lipocalins under physiological conditions.

In some aspects, the present disclosure provides CD137-binding hNGAL muteins. In this regard, the disclosure provides one or more hNGAL muteins that are capable of binding CD137 with an affinity measured by a K_{D} of about 800 nM, 700 nM, 200 nM, 140 nM, 100 nM or lower, preferably about 70 nM, 50 nM, 30 nM, 10 nM, 5 nM, 2 nM, or even lower.

A CD137-binding hNGAL mutein comprised in the fusion protein of the invention comprises the following set of mutated amino acid residues in comparison with the linear polypeptide sequence of mature hNGAL as shown in SEQ ID NO: 2: Gln 28 → His; Leu 36 → Gln; Ala 40 → Ile; Ile 41 → Arg; Gln 49 → Ile; Tyr 52 → Met; Asn 65 → Asp; Ser 68 → Met; Leu 70 → Lys; Arg 72 → Asp; Lys 73 → Asp; Asp 77 → Met; Trp 79 → Asp; Arg 81 → Trp; Cys 87 → Ser; Asn 96 → Lys; Tyr 100 → Phe; Leu 103 → His; Tyr 106 → Ser; Lys 125 → Phe; Ser 127 → Phe; Tyr 132 → Glu; and Lys 134 → Tyr.ln some embodiments, an hNGAL mutein comprised in the fusion protein of the invention comprises the amino acid sequence as set forth in SEQ ID NO: 42.

In some embodiments, an hNGAL mutein comprised in the fusion protein of the invention has at least 90%, at least 95%, at least 98%, or higher sequence identity to the amino acid sequence of SEQ ID NO: 42.

In some embodiments, the fusion protein of the invention comprises a lipocalin mutein that binds CD137 with an affinity measured by a K_{D} of about 5 nM or lower, wherein the lipocalin mutein has at least 90%, at least 95%, at least 98%, or higher sequence identity to the amino acid sequence of SEQ ID NO: 42.

In some embodiments, a fusion protein of the invention comprises a lipocalin mutein that can comprise a heterologous amino acid sequence at its N-or C-Terminus, preferably C-terminus, such as a Strep II tag (SEQ ID NO: 12) or a cleavage site sequence for certain restriction enzymes, without affecting the biological activity (binding to its target, e.g., CD137) of the lipocalin mutein.

In some embodiments, further modifications of a lipocalin mutein may be introduced in order to modulate certain characteristics of the mutein, such as to improve folding stability, serum stability, protein resistance or water solubility or to reduce aggregation tendency, or to introduce new characteristics to the mutein. In some embodiments, modification(s) may result in two or more (e.g., 2, 3, 4, 5, 6, 7, 8, 9, or 10) characteristics of a provided mutein being modulated.

For example, it is possible to mutate one or more amino acid sequence positions of a lipocalin mutein to introduce new reactive groups, for example, for the conjugation to other compounds, such as polyethylene glycol (PEG), hydroxyethyl starch (HES), biotin, peptides or proteins, or for the formation of non-naturally occurring disulphide linkages. The conjugated compound, for example, PEG and HES, can in some cases increase the serum half-life of the corresponding lipocalin mutein.

In some embodiments, a reactive group of a lipocalin mutein may occur naturally in its amino acid sequence, such as naturally occurring cysteine residues in said amino acid sequence. In some other embodiments, such reactive group may be introduced via mutagenesis. In case a reactive group is introduced via mutagenesis, one possibility is the mutation of an amino acid at the appropriate position by a cysteine residue. Exemplary possibilities of such a mutation to introduce a cysteine residue into the amino acid sequence of an hTIc mutein include the substitutions Thr 40→ Cys, Glu 73→ Cys, Arg 90→ Cys, Asp 95→ Cys, and Glu 131→ Cys of the wild-type sequence of hTIc (SEQ ID NO: 1). Exemplary possibilities of such a mutation to introduce a cysteine residue into the amino acid sequence of an hNGAL mutein include the introduction of a cysteine residue at one or more of the sequence positions that correspond to sequence positions 14, 21, 60, 84, 88, 116, 141, 145, 143, 146 or 158 of the wild-type sequence of hNGAL (SEQ ID NO: 2).The generated thiol moiety may be used to PEGylate or HESylate the mutein, for example, in order to increase the serum half-life of a respective lipocalin mutein

In some embodiments, in order to provide suitable amino acid side chains as new reactive groups for conjugating one of the above compounds to a lipocalin mutein, artificial amino acids may be introduced to the amino acid sequence of a lipocalin mutein. Generally, such artificial amino acids are designed to be more reactive and thus to facilitate the conjugation to the desired compound. Such artificial amino acids may be introduced by mutagenesis, for example, using an artificial tRNA is para-acetyl-phenylalanine.

### E. Exemplary uses and applications of fusion proteins specific for CD137 and PD-L1

The references to the methods of treatment by therapy or surgery or *in vivo* diagnosis methods or uses of compounds or compositions for methods of treatment by therapy or surgery or *in vivo* diagnosis methods in this chapter of the description are to be interpreted as references to compounds, pharmaceutical compositions and medicaments of the present invention for use in those methods

In some embodiments, fusion proteins of the disclosure may produce synergistic effect through dual-targeting of CD137 and PD-L1. In some embodiments, fusion proteins of the disclosure may produce synergistic effect through CD137 co-stimulation and PD-1/PD-L1 pathway blockade. In some embodiments, fusion proteins of the disclosure may produce localized anti-tumor effect through dual-targeting of CD137 and PD-L1. Numerous possible applications for the fusion proteins of the disclosure, therefore, exist in medicine.

In some embodiments, the present disclosure encompasses the use of one or more fusion proteins disclosed herein or of one or more compositions comprising such fusion proteins for simultaneously binding of CD137 and PD-L1.

The present disclosure also involves the use of one or more fusion proteins as described for complex formation with CD137 and/or PD-L1.

Therefore, in one aspect of the disclosure, provided fusion proteins may be used for the detection of CD137 and PD-L1. Such use may include the steps of contacting one or more said fusion proteins, under suitable conditions, with a sample suspected of containing CD137 and/or PD-L1, thereby allowing formation of a complex between the fusion proteins and CD137 and/or PD-L1, and detecting the complex by a suitable signal. The detectable signal can be caused by a label, as explained above, or by a change of physical properties due to the binding, i.e., the complex formation, itself. One example is surface plasmon resonance, the value of which is changed during binding of binding partners from which one is immobilized on a surface such as a gold foil.

Fusion proteins of the disclosure may also be used for the separation of CD137 and/or PD-L1. Such use may include the steps of contacting one or more said fusion proteins, under suitable conditions, with a sample supposed to contain CD137 and/or PD-L1, thereby allowing the formation of a complex between the fusion proteins and CD137 and/or PD-L1 and separating the complex from the sample.

In some aspects, the present disclosure provides diagnostic and/or analytical kits comprising one or more fusion proteins according to the disclosure.

In addition to their use in diagnostics, in yet another aspect, the disclosure contemplates pharmaceutical compositions comprising one or more fusion proteins of the disclosure and a pharmaceutically acceptable excipient.

Furthermore, in some embodiments, the present disclosure provides fusion proteins that simultaneously bind CD137 and/or PD-L1 for use such as anti-tumor and/or anti-infection agents, and immune modulators. In some embodiments, fusion proteins of the present invention are envisaged to be used in a method of prevention, amelioration, or treatment of human diseases, such as a variety of cancers, including PD-L1-positive cancers. Accordingly, also provided are methods of preventing, ameliorating, or treating human diseases such as a variety of cancers, including PD-L1-positive cancer, in a subject in need thereof, comprising administering to said subject a therapeutically effective amount of one or more fusion proteins of the disclosure.

Examples of cancers that may be treated using the fusion proteins of the disclosure, include liver cancer, bone cancer, pancreatic cancer, skin cancer, head and neck cancer, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T cell lymphoma, and precursor T-lymphoblastic lymphoma, and any combinations of said cancers. In some embodiments, the present invention is also applicable to the treatment of metastatic cancers.

In some embodiments, fusion proteins of the invention may simultaneously target tumor cells where PD-L1 is expressed and activate lymphocytes of the host immune system adjacent to such tumor cells. In some embodiments, fusion proteins of the invention may increase targeted anti-tumor T cells activity, enhance anti-tumor immunity and, and/or have a direct inhibiting effect on tumor growth, thereby produce synergistic anti-tumor results. In some embodiments, fusion proteins of the invention may activate immune responses in a tumor microenvironment. In some embodiments, fusion proteins of the invention may reduce side effects of effector lymphocytes towards healthy cells, i.e. off-target toxicity, for example, via locally inhibiting oncogene activity and inducing lymphocyte activation.

In some embodiments, the present disclosure encompasses the use of a fusion protein of the invention, or a composition comprising a provided fusion protein, for inducing a localized lymphocyte response in the vicinity of PD-L1-positive tumor cells. Accordingly, in some embodiments, the present disclosure provides methods of inducing a localized lymphocyte response in the vicinity of PD-L1-positive tumor cells, comprising applying one or more fusion proteins of the invention or of one or more compositions comprising such fusion proteins. "Localized" means that upon simultaneous binding T-cells via CD137 and engaging PD-L1-positive tumor cells, T-cells produce cytokines, particularly IL-2 and/or IFN gamma in vicinity of the PD-L1-positive cells. Such cytokines reflect activation of T-cells which may then be able to kill PD-L1-positive cells, either directly or indirectly by attracting other killer cells, such as T-cells or NK cells.

In some embodiments, the present disclosure encompasses the use of a fusion protein of the invention, or a composition comprising such fusion protein, for co-stimulating T-cells, and/or activating downstream signaling pathways of CD137. Preferably, a proivided fusion protein co-stimulates T-cells and/or activating downstream signaling pathways of CD137 when engaging tumor cells where PD-L1 is expressed. Accordingly, the present disclosure provides methods of inducing T lymphocyte proliferation and/or activating downstream signaling pathways of CD137, preferably when engaging tumor cells where PD-L1 is expressed, comprising applying one or more fusion proteins of the disclosure and/or one or more compositions comprising such fusion proteins.

In some embodiments, the present disclosure encompasses the use of a fusion protein of the invention, or a composition comprising such fusion protein, for inducing CD137 clustering and activation on T-cells and directing such T-cells to tumor cells where PD-L1 is expressed.

### F. Production of exemplary provided fusion proteins specific for CD137 and PD-L1.

In some embodiments, the present disclosure provides nucleic acid molecules (DNA and RNA) that include nucleotide sequences encoding a fusion protein as set out in the appended set of claims. In some embodiments, the disclosure encompasses a host cell containing a provided nucleic acid molecule. Since the degeneracy of the genetic code permits substitutions of certain codons by other codons specifying the same amino acid, the disclosure is not limited to a specific nucleic acid molecule encoding a fusion protein as described herein, rather, encompassing all nucleic acid molecules that include nucleotide sequences encoding a functional fusion protein. In this regard, the present disclosure also relates to nucleotide sequences encoding provided fusion proteins.

A nucleic acid molecule, such as DNA, is referred to as "capable of expressing a nucleic acid molecule" or "able to allow expression of a nucleotide sequence" if it includes sequence elements that contain information regarding to transcriptional and/or translational regulation, and such sequences are "operably linked" to the nucleotide sequence encoding the protein. An operable linkage is a linkage in which the regulatory sequence elements and the sequence to be expressed are connected in a way that enables gene expression. The precise nature of the regulatory regions necessary for gene expression may vary among species, but in general these regions include a promoter, which, in prokaryotes, contains both the promoter per se, i.e., DNA elements directing the initiation of transcription, as well as DNA elements which, when transcribed into RNA, will signal the initiation of translation. Such promoter regions normally include 5' non-coding sequences involved in initiation of transcription and translation, such as the -35/-10 boxes and the Shine-Dalgarno element in prokaryotes or the TATA box, CAAT sequences, and 5'-capping elements in eukaryotes. These regions can also include enhancer or repressor elements as well as translated signal and leader sequences for targeting the native protein to a specific compartment of a host cell.

In addition, 3' non-coding sequences may contain regulatory elements involved in transcriptional termination, polyadenylation or the like. If, however, these termination sequences are not satisfactorily functional in a particular host cell, then they may be substituted with signals functional in that cell.

Therefore, a nucleic acid molecule of the disclosure may be "operably linked" to one or more regulatory sequences, such as a promoter sequence, to allow expression of this nucleic acid molecule. In some embodiments, a nucleic acid molecule of the disclosure includes a promoter sequence and a transcriptional termination sequence. Suitable prokaryotic promoters are, for example, the tet promoter, the lacUV5 promoter or the T7 promoter. Examples of promoters useful for expression in eukaryotic cells are the SV40 promoter or the CMV promoter.

In some embodiments, provided nucleic acid molecules can also be part of a vector or any other kind of cloning vehicle, such as a plasmid, a phagemid, a phage, a baculovirus, a cosmid or an artificial chromosome.

In some embodiments, a provided nucleic acid molecule may be included in a phagemid. As used in this context, a phagemid vector denotes a vector encoding the intergenic region of a temperate phage, such as M13 or f1, or a functional part thereof fused to the cDNA of interest. For example, in some embodiments, after superinfection of bacterial host cells with such a provided phagemid vector and an appropriate helper phage (e.g., M13K07, VCS-M13 or R408) intact phage particles are produced, thereby enabling physical coupling of the encoded heterologous cDNA to its corresponding polypeptide displayed on the phage surface (Lowman, Annu Rev Biophys Biomol Struct, 1997, Rodi and Makowski, Curr Opin Biotechnol, 1999).

In accordance with various embodiments, cloning vehicles can include, aside from the regulatory sequences described above and a nucleic acid sequence encoding a fusion protein as described herein, replication and control sequences derived from a species compatible with the host cell that is used for expression as well as selection markers conferring a selectable phenotype on transformed or transfected cells. Large numbers of suitable cloning vectors are known in the art and are commercially available.

The disclosure also relates, in some embodiments, to methods for the production of fusion proteins of the disclosure starting from a nucleic acid coding for a fusion protein or any subunits therein using genetic engineering methods as set out in the appended set of claims. In some embodiments, a provided method can be carried out *in vivo,* wherein a provided fusion protein can, for example, be produced in a bacterial or eukaryotic host organism, and then isolated from this host organism or its culture. It is also possible to produce a fusion protein of the disclosure *in vitro,* for example, using an *in vitro* translation system.

When producing a fusion protein *in vivo,* a nucleic acid encoding such fusion protein may be introduced into a suitable bacterial or eukaryotic host organism using recombinant DNA technology well known in the art. In some embodiments, a DNA molecule encoding a fusion protein of the invention, and in particular a cloning vector containing the coding sequence of such a fusion protein can be transformed into a host cell capable of expressing the gene. Transformation can be performed using standard techniques. Thus, the disclosure is also directed to host cells containing a nucleic acid molecule as disclosed herein.

In some embodiments, transformed host cells may be cultured under conditions suitable for expression of the nucleotide sequence encoding a fusion protein of the invention. In some embodiments, host cells can be prokaryotic, such as *Escherichia coli* (*E. coli*) or *Bacillus subtilis,* or eukaryotic, such as *Saccharomyces cerevisiae, Pichia pastoris,* SF9 or High5 insect cells, immortalized mammalian cell lines (e.g., HeLa cells or CHO cells) or primary mammalian cells.

In some embodiments, where a lipocalin mutein of the disclosure as comprised in a fusion protein as set out in the appended set of claims, includes intramolecular disulfide bonds, it may be preferred to direct the nascent protein to a cell compartment having an oxidizing redox milieu using an appropriate signal sequence. Such an oxidizing environment may be provided by the periplasm of Gram-negative bacteria such as *E*. *coli,* in the extracellular milieu of Gram-positive bacteria or the lumen of the endoplasmic reticulum of eukaryotic cells and usually favors the formation of structural disulfide bonds.

In some embodiments, it is also possible to produce a fusion protein of the invention in the cytosol of a host cell, preferably *E*. *coli.* In this case, a provided fusion protein can either be directly obtained in a soluble and folded state or recovered in the form of inclusion bodies, followed by renaturation *in vitro.* A further option is the use of specific host strains having an oxidizing intracellular milieu, which may thus allow the formation of disulfide bonds in the cytosol (Venturi et al., J Mol Biol, 2002).

The skilled worker will appreciate methods useful to prepare fusion proteins contemplated by the present disclosure but whose protein or nucleic acid sequences are not explicitly disclosed herein. As an overview, such modifications of the amino acid sequence include, e.g., directed mutagenesis of single amino acid positions to simplify sub-cloning of a protein gene or its parts by incorporating cleavage sites for certain restriction enzymes. Also, these mutations can be incorporated to further improve the affinity of a fusion protein for its targets (e.g., CD137 and PD-L1). Furthermore, mutations can be introduced to modulate one or more characteristics of the protein such as to improve folding stability, serum stability, protein resistance or water solubility or to reduce aggregation tendency, if necessary.

### V. EXAMPLES

### Example 1: Expression and analysis of representative fusion proteins

In this Example, representative antibody-lipocalin mutein fusion proteins were generated by fusing together a PD-L1 specific antibody having the heavy chain provided by SEQ ID NO: 86, or comprise a heavy chain variable domain of SEQ ID NO: 77, or comprising the CDRs of GFSLSNYD (HCDR1, SEQ ID NO: 60), IWTGGAT (HCDR2, SEQ ID NO: 61), VRDSNYRYDEPFTY (HCDR3; SEQ ID NO: 62), and light chains provided by SEQ ID NO: 87, or comprise a heavy chain variable domain of SEQ ID NO: 82, or comprising the CDRs of QSIGTN (LCDR1, SEQ ID NO: 63), YAS (LCDR2), QQSNSWPYT (LCDR3; SEQ ID NO: 64), and the CD137-specific lipocalin mutein of SEQ ID NO: 42, via a linker, such as an unstructured (G₄S)₃ linker of SEQ ID NO: 13, to engage PD-L1 and CD137 at the same time. The different formats that were generated are depicted in **Figure 1**. For example, such fusion proteins, e.g., SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91, were generated via fusing the one or more of lipocalin mutein of SEQ ID NO: 42 to either one or more of the four termini of an antibody comprising of the heavy chain provided by the heavy chain provided by SEQ ID NO: 86, or comprise a heavy chain variable domain of SEQ ID NO: 77, or comprising the CDRs of GFSLSNYD (HCDR1, SEQ ID NO: 60), IWTGGAT (HCDR2, SEQ ID NO: 61), VRDSNYRYDEPFTY (HCDR3; SEQ ID NO: 62), and light chains provided by SEQ ID NO: 87, or comprise a heavy chain variable domain of SEQ ID NO: 82, or comprising the CDRs of QSIGTN (LCDR1, SEQ ID NO: 63), YAS (LCDR2), QQSNSWPYT (LCDR3; SEQ ID NO: 64). The generated fusion proteins can be bivalent to CD137 (e.g., as depicted in **Figure 1A-1D**) or tetravalent to CD137 (e.g., as depicted in **Figure 1E-1H**), or have even higher valency to CD137 (e.g., as depicted in **Figure 1I**).

The PD-L1 specific antibodies as well as all antibody lipocalin mutein fusion proteins described in this Example had an engineered IgG4 backbone, which contained a S228P mutation to minimize IgG4 half-antibody exchange in-vitro and in-vivo (Silva et al., J Biol Chem, 2015). Additional mutations in the IgG4 backbones may also exist in all antibodies and fusion proteins described here, including any one or more of mutations F234A, L235A, M428L, N434S, M252Y, S254T, and T256E. F234A and L235A mutations may be introduced to decrease ADCC and ADCP (Glaesner et al., Diabetes Metab Res Rev, 2010). M428L and N434S mutations or M252Y, S254T, and T256E mutations may be introduced for extended serum half-life (Dall'Acqua et al., J Biol Chem, 2006, Zalevsky et al., Nat Biotechnol, 2010). All antibodies were expressed without the carboxy-terminal lysine to avoid heterogeneity.

In addition, monospecific lipocalin mutein Fc fusions were generated by fusing one or more of the CD137 specific lipocalin mutein of SEQ ID NO: 42, via a linker, e.g., an unstructured (G4S)3 linker of SEQ ID NO: 13, to the C-terminus of the Fc region of an antibody provided in SEQ ID NO: 30 as depicted in **Figure 1J-1K**. The resulting construct is provided in SEQ ID NOs: 88-89.

The constructs of the fusion proteins were generated by gene synthesis and cloned into a mammalian expression vector. They were then transiently expressed in Expi293FTM cells (Life Technologies). The concentration of fusion proteins in the cell culture medium was measured by HPLC (Agilent Technologies) employing a POROS^{®} protein A affinity column (Applied Biosystems). The titers of the fusion proteins were summarized in Table 1.

The fusion proteins were purified using Protein A chromatography followed by size-exclusion chromatography (SEC) in phosphate-buffered saline (PBS). After SEC purification, the fractions containing monomeric protein are pooled and analyzed again using analytical SEC.

**Table 1: Transient expression titers**

| **SEQ ID NO** | **Expression titer [mg/mL]** |
|---|---|
| SEQ ID NOs: 90 and 87 | 0.05 |
| SEQ ID NOs: 86 and 91 | 0.07 |
| SEQ ID NOs: 92 and 87 | 0.06 |
| SEQ ID NOs: 86 and 93 | 0.09 |
| SEQ ID NOs: 94 and 87 | 0.06 |
| SEQ ID NOs: 90 and 91 | 0.10 |
| SEQ ID NO: 88 | 0.24 |
| SEQ ID NOs: 86 and 87 | 0.07 |

### Example 2: Expression of the fusion proteins

The constructs of exemplary fusion proteins were generated by gene synthesis including codon optimization and cloned into a mammalian expression vector. They were then stably expressed in Chinese hamster ovary (CHO) cells. The concentration of fusion proteins in the cell culture medium was measured using Octet (ForteBio, Pall Corp.) with Protein-A sensors and quantified using human IgG1 standard. The titers of the fusion proteins were summarized in **Table 2.** The data suggest that the geometry of the fusion proteins may have an influence on product yield and cell productivity.

**Table 2: Stable expression titers**

| **SEQ ID NO** | **Expression titer [mg/mL]** |
|---|---|
| SEQ ID NOs: 90 and 87 | 1.423 |
| SEQ ID NOs: 86 and 91 | 1.502 |
| SEQ ID NOs: 92 and 87 | 0.456 |
| SEQ ID NOs: 86 and 93 | 0.294 |
| SEQ ID NOs: 94 and 87 | 0.293 |
| SEQ ID NOs: 90 and 91 | 1.297 |
| SEQ ID NO: 88 | 0.150 |
| SEQ ID NOs: 86 and 87 | 1.018 |

### Example 3: Binding of fusion proteins towards PD-L1 or CD137 determined by surface plasmon resonance (SPR)

The binding kinetics and affinity of exemplary fusion proteins to huPD-L1-His or huCD137-His (human PD-L1 or human CD137 with a C-terminal polyhistidine tag, R&D Systems) were determined by surface plasmon resonance (SPR) using a Biacore 8K or a Biacore T200 (GE Healthcare).

The anti-human IgG Fc antibody (GE Healthcare) was immobilized on a CM5 sensor chip using standard amine chemistry: the carboxyl groups on the chip were activated using 1-ethyl-3-(3-dimethylaminopropyl)-carbodiimide (EDC) and N-hydroxysuccinimide (NHS). Subsequently, anti-human IgG Fc antibody solution (GE Healthcare) at a concentration of 25 µg/mL in 10 mM sodium acetate (pH 5.0) was applied at a flow rate of 5 µL/min until an immobilization level of 6000-10000 resonance units (RU) was achieved. Residual non-reacted NHS-esters were blocked by passing a solution of 1M ethanolamine across the surface. The reference channel was treated in an analogous manner. Subsequently, testing fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, SEQ ID NOs: 90 and 91, SEQ ID NO: 88, and SEQ ID NO: 89) at 0.25 µg/ml or 0.5 µg/mL in HBS-EP+ buffer was captured by the anti-human IgG-Fc antibody at the chip surface for 180 s at a flow rate of 10 µL/min. After each capture step, the needle was washed. Anti-PD-L1 antibodies, including a reference antibody (SEQ ID NOs: 26 and 27) and an antibody as included in the fusion proteins (SEQ ID NOs: 86 and 87), and a reference anti-CD137 antibody (SEQ ID NOs: 28 and 29) were also tested as controls.

For affinity determination, dilutions of huPD-L1-His (10 nM, 5 nM and 2.5 nM or huCD137-His (900 nM, 300 nM, and 100 nM) were prepared in HBS-EP+ buffer and applied to the prepared chip surface. The binding assay was carried out with a contact time of 180 s, a dissociation time of 900 s and a flow rate of 30 µL/min. All measurements were performed at 25°C. Regeneration of the chip surface was achieved with injections of 3 M MgCl₂for 120 s. Prior to the protein measurements, three startup cycles were performed for conditioning purposes. Data were evaluated with Biacore T200 Evaluation software (v2.0) or with Biacore 8K Evaluation software (V1.1.1). Double referencing was used and the 1:1 binding model was used to fit the raw data.

The values determined for kₒₙ, k_{off}, and the resulting equilibrium dissociation constant (K_{D}) for representative fusion proteins are summarized in **Table 3.** All bispecific fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91) bind PD-L1 as well as CD137 with subnanomolar to low nanomolar affinity. Monospecific CD137-specific lipocalin mutein-Fc fusions (SEQ ID NO: 88 and SEQ ID NO: 89) only bind CD137 with low nanomolar affinity.

**Table 3: Kinetic constants and affinities of fusion proteins determined by SPR**

| **SEQ ID NO** | **huPD-L1** | | | **huCD137** | | |
|---|---|---|---|---|---|---|
| | **kₒₙ [M⁻¹ x s⁻¹]** | **k_{off} [s⁻¹]** | **K_{D} [nM]** | **kₒₙ [M⁻¹ x s⁻¹]** | **k_{off} [s⁻¹]** | **K_{D} [nM]** |
| 90 and 87 | 1.30E+06 | 7.69E-04 | 0.592 | 3.93E+04 | 2.55E-04 | 6.484 |
| 86 and 91 | 1.32E+06 | 7.48E-04 | 0.568 | 3.42E+04 | 1.75E-04 | 5.106 |
| 92 and 87 | 7.87E+05 | 8.03E-04 | 1.021 | 3.48E+04 | 2.08E-04 | 5.966 |
| 86 and 93 | 5.46E+05 | 7.54E-04 | 1.381 | 3.68E+04 | 1.30E-04 | 3.548 |
| 94 and 87 | 1.51E+06 | 9.16E-04 | 0.608 | 4.07E+04 | 2.24E-04 | 5.504 |
| 90 and 91 | 1.60E+06 | 8.61E-04 | 0.537 | 3.91E+04 | 2.24E-04 | 5.726 |
| 88 | N.A. | N.A. | N.A. | 3.94E+04 | 1.63E-04 | 4.155 |
| 86 and 87 | 1.09E+06 | 6.49E-04 | 0.593 | N.A. | N.A. | N.A. |
| 89 | N.A. | N.A. | N.A. | 3.66E+04 | 1.32E-04 | 3.595 |
| 26 and 27 | 6.38E+05 | 2.01E-04 | 0.314 | N.A. | N.A. | N.A. |
| 28 and 29 | N.A. | N.A. | N.A. | 4.72E+05 | 2.94E-03 | 6.237 |

### Example 4. Binding of fusion proteins towards PD-L1 or CD137 in enzyme-linked immunosorbent assay (ELISA)

An enzyme-linked immunosorbent assay (ELISA) was employed to determine the binding potency of exemplary fusion proteins to human PD-L1 and cynomolgus PD-L1.

Recombinant huPD-L1-His or cyPD-L1-His (human or cynomolgus PD-L1 with a C-terminal polyhistidine tag, R&D Systems or Sino Biologics) at the concentration of 1 µg/mL in PBS was coated overnight on microtiter plates at 4°C. After washing with PBS-0.05%T (PBS supplemented with 0.05% (v/v) Tween 20), the plates were blocked with 2% BSA (w/v) in PBS-0.1%T (PBS supplemented with 0.1% (v/v) Tween 20) for 1 h at room temperature. After washing with 100 µL PBS-0.05%T five times, exemplary fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, SEQ ID NOs: 90 and 91), Fc fusions a CD137-specific lipocalin mutein (SEQ ID NOs: 88 and SEQ ID NO: 89), and anti-PD-L1 antibodies (SEQ ID NOs: 26 and 27, SEQ ID NOs: 86 and 87), at different concentrations were added to the wells and incubated for 1 h at room temperature, followed by another wash step. Bound molecules under study were detected by incubation with 1:5000 diluted anti-human IgG Fc-HRP (Jackson Laboratory) in PBS-0.1%T-2%BSA. After an additional wash step, fluorogenic HRP substrate (QuantaBlu, Thermo) was added to each well and the fluorescence intensity was detected using a fluorescence microplate reader.

The same ELISA setup was also employed to determine the binding potency of fusion proteins to CD137, where huCD137-His (human CD137 with C-terminal polyhistidine tag, R&D Systems) or cyCD137-Fc (cynomolgus CD137 C-terminally fused to Fc) was instead coated on a microtiter plate. The testing agents were similarly titrated and bound agents were detected via anti-NGAL-HRP.

The results of exemplary experiments are depicted in **Figure 2A-2D****,** together with the fit curves resulting from a 1:1 binding sigmoidal fit, where the EC₅₀ value and the maximum signal were free parameters, and the slope was fixed to unity. The resulting EC₅₀ values are provided in **Table 4.**

The observed EC₅₀ values toward the two human targets of provided fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, SEQ ID NOs: 90 and 91, SEQ ID NO: 88, and SEQ ID NO: 89) were very similar or comparable to tested PD-L1 antibodies (reference PD-L1 antibody of SEQ ID NOs: 26 and 27 and PD-L1 antibody of SEQ ID NOs: 86 and 87 as included in the fusion proteins), and/or the CD137-specific lipocalin mutein as included in the fusion proteins (SEQ ID NO: 42).

All tested fusion proteins show cross-reactivity to cynomolgus PD-L1, with comparable EC₅₀ values to the reference PD-L1 antibody (SEQ ID NOs: 26 and 27) or the PD-L1 antibody included in the fusion proteins (SEQ ID NOs: 86 and 87). Only fusion proteins that are tetravalent to CD137 showed (SEQ ID NOs: 94 and 87, SEQ ID NOs: 90 and 91, and SEQ ID NO: 88) show cross-reactivity to cynomolgus CD137 at a comparable level to human CD137, i.e, bind cynomolgus CD137 with EC₅₀ values in the same range as the corresponding EC₅₀s for human CD137.

**Table 4. ELISA data for PD-L1 or CD137 binding**

| **SEQ ID NO** | **EC₅₀ [nM] Binding to huPD-L1** | **EC₅₀ [nM] Binding to cyPD-L1** | **EC₅₀ [nM] Binding to huCD137** | **EC₅₀ [nM] Binding to cyCD137** |
|---|---|---|---|---|
| 90 and 87 | 0.15 | 0.13 | 0.28 | 5.9 |
| 86 and 91 | 0.23 | 0.18 | 0.57 | 13 |
| 92 and 87 | 0.16 | 0.15 | 0.41 | 9.8 |
| 86 and 93 | 0.19 | 0.17 | 0.35 | 8.1 |
| 94 and 87 | 0.18 | 0.15 | 0.16 | 0.19 |
| 90 and 91 | 0.20 | 0.17 | 0.14 | 0.21 |
| 88 | N.A. | N.A. | 0.15 | 0.12 |
| 86 and 87 | 0.12 | 0.1 | N.A. | N.A. |
| 89 | N.A. | N.A. | 0.29 | 0.82 |
| 26 and 27 | 0.09 | 0.09 | N.A. | N.A. |
| 42 | N.A. | N.A. | 0.27 | N.A. |

### Example 5. Simultaneous binding of fusion proteins to PD-L1 and CD137 in ELISA

In order to demonstrate the simultaneous binding of exemplary fusion proteins to PD-L1 and CD137, a dual-binding ELISA format was used.

Recombinant huPD-L1-His (R&D Systems) in PBS (1 µg/mL) was coated overnight on microtiter plates at 4°C. The plates were washed five times after each incubation step with 100 µL PBS-0.05%T. The plates were blocked with 2% BSA (w/v) in PBS-0.1%T for 1 h at room temperature and subsequently washed again. Different concentrations of tested fusion proteins were added to the wells and incubated for 1 h at room temperature, followed by a wash step. Subsequently, biotinylated huCD137-His (huCD137-His-Bio, Sino Biological) was added at a constant concentration of 1 µg/mL in PBS-0.1%T-2%BSA for 1 h. After washing, a 1:5000 dilution of ExtrAvidin-HRP (Sigma-Aldrich) in PBS-0.1%T-2%BSA was added to the wells and incubated for 1 h. After an additional wash step, fluorogenic HRP substrate (QuantaBlu, Thermo) was added to each well, and the fluorescence intensity was detected using a fluorescence microplate reader.

The dual binding of exemplary fusion proteins was also tested with a reverse set-up where recombinant 1 µg/ml huCD137-His (R&D Systems) was coated on microtiter plates and the bound fusion proteins were detected via the addition of biotinylated huPD-L1-His (R&D Systems).

Dual binding data of fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91) are shown in **Figure 3A** and **3B**, together with the fit curves resulting from a 1:1 sigmoidal binding fit, where the EC₅₀ value and the maximum signal were free parameters, and the slope was fixed to unity. The EC₅₀ values are summarized in **Table 5**. All bispecific fusion proteins show clear binding signals, demonstrating that the fusion proteins are able to engage PD-L1 and CD137 simultaneously. The data further suggested fusing CD137-specific lipocalin muteins to C-termini of the PD-L1-specific antibodies may be more advantageous than to the N-termini.

**Table 5. ELISA data for simultaneous target binding of both PD-L1 and CD37**

| **SEQ ID NO** | **EC₅₀ [nM] PD-L1 capture_CD137 detection** | **EC₅₀ [nM] CD137 capture_PD-L1 detection** |
|---|---|---|
| 90 and 87 | 0.58 | 2.9 |
| 86 and 91 | 0.59 | 3.3 |
| 92 and 87 | 1.2 | 7.2 |
| 86 and 93 | 0.74 | 6.7 |
| 94 and 87 | 0.48 | 2.6 |
| 90 and 91 | 0.49 | 2.3 |

### Example 6. Flow cytometric analysis of fusion proteins binding to cells expressing human and cynomolgus CD137 and PD-L1

Target specific binding of fusion proteins to human and cynomolgus PD-L1-expressing cells and human and cynomolgus CD137-expressing cells was assessed by flow cytometry.

CHO cells were stably transfected with human PD-L1, cynomolgus PD-L1, human CD137, cynomolgus CD137 or a mock control using the Flp-In system (Life technologies) according to the manufacturer's instructions.

Transfected CHO cells were maintained in Ham's F12 medium (Life technologies) supplemented with 10% Fetal Calf Serum (Biochrom) and 500 µg/ml Hygromycin B (Roth). Cells were cultured in cell culture flasks according to manufacturer's instruction (37°C, 5% CO2 atmosphere).

For flow cytometric analysis, respective cell lines were incubated with fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, SEQ ID NOs: 90 and 91, SEQ ID NO: 88, and SEQ ID NO: 89) and detected using a fluorescently labeled anti-human IgG antibody in FACS analysis as described in the following:

5 × 10⁴ cells per well were incubated for 1 h in ice-cold PBS containing 5% fetal calf serum (PBS-FCS). A dilution series of the fusion proteins and control antibodies were added to the cells and incubated for 1 h on ice. Cells were washed twice with PBS and then incubated with a goat anti-hlgG Alexa647-labeled antibody for 30 min on ice. Cells were subsequently washed and analyzed using iQue Flow cytometer (Intellicyte Screener). Mean geometric fluorescent signals were plotted and fitted with Graphpad software using nonlinear regression (shared bottom, SLOPE =1).

The ability of fusion proteins to bind human and cynomolgus PD-L1 and CD137 is depicted in **Figure 4****.** Binding affinities (EC₅₀s) of bispecific fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91) to human and cynomolgus PD-L1 expressing cells are in the single digit nanomolar range demonstrating full cyno-crossreactivity (summarized in **Table 6**). Binding affinities of fusion proteins to human CD137 expressing cells are in the low nanomolar range. Tested fusion proteins are fully cross-reactive to cynomolgus CD137 (SEQ ID NOs: 94 and 87, SEQ ID NOs: 90 and 91, and SEQ ID NO: 88), bind cynomolgus CD137 with 6-13-fold decreased affinities compared to the corresponding binding affinities to human CD137 (SEQ ID NOs: 90 and 87, SEQ ID NOs: 86 and 91, and SEQ ID NO: 89), or do not bind cynomolgus CD137 (SEQ ID NO: 92 and 87 and 86 and 93). None of the fusion proteins bind to mock transfected cells.

**Table 6. Binding affinities of the fusion proteins to cells expressing human and cynomolgus PD-L1 or CD137**

| **SEQ ID NO** | **EC₅₀ [nM] Flp-In-CHO::huCD137** | **EC₅₀ [nM] Flp-In-CHO::cynoCD137** | **EC₅₀ [nM] Flp-In-CHO::huPDL-1** | **EC₅₀ [nM] Flp-In-CHO::cynoPDL-1** |
|---|---|---|---|---|
| 90 and 87 | 3.74 | 51.35 | 3.64 | 4.48 |
| 86 and 91 | 6.48 | 39.15 | 1.43 | 1.41 |
| 92 and 87 | 11.91 | -- | 2.95 | 2.03 |
| 86 and 93 | 12.73 | -- | 4.63 | 2.03 |
| 94 and 87 | 4.15 | 6.92 | 6.99 | 5.89 |
| 90 and 91 | 4.69 | 3.66 | 4.09 | 3.37 |
| 86 and 87 | -- | -- | 2.96 | 3.55 |
| 89 | 5.74 | 26.70 | -- | -- |
| 88 | 4.33 | 3.81 | -- | -- |
| 28 and 29 | 1.31 | -- | -- | -- |

### Example 7. Binding affinities of the fusion proteins to PD-L1-positive tumor cells

Binding of fusion proteins to tumor cells expressing PD-L1 was assessed by flow cytometry.

PD-L1 expressing colorectal cancer cell line RKO was maintained in RPMI1640 (Life technologies) supplemented with 10% FCS at 37°C in a humidified 5% CO₂ atmosphere.

For flow cytometric analysis, RKO cells were incubated with fusion proteins and detected using a fluorescently labeled anti-human IgG antibody as described in **Example 6**.

The ability of fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91) to bind PD-L1-positive tumor cells is depicted in **Figure 5** and the corresponding binding affinities (EC₅₀s) are summarized in **Table 7**. Binding affinities of fusion proteins to PD-L1-expressing RKO cells were in the low nanomolar or subnanomolar range, comparable to the PD-L1 antibody included in the fusion proteins (SEQ ID NOs: 86 and 87).

**Table 7. Binding affinities of the fusion proteins to PD-L1-positive tumor cells**

| **SEQ ID NO** | **EC₅₀ [nM] RKO** |
|---|---|
| 90 and 87 | 0.51 |
| 86 and 91 | 0.45 |
| 92 and 87 | 1.00 |
| 86 and 93 | 1.38 |
| 94 and 87 | 0.69 |
| 90 and 91 | 0.53 |
| 86 and 87 | 0.32 |

### Example 8. Determination of the competition between CD137L and fusion proteins in binding to CD137 by SPR

An SPR assay was utilized to investigate the competition between human CD137L (huCD137L-His, R&D Systems) and exemplary fusion proteins to the human CD137. The competition assay was performed at 25°C on a Biacore T200 instrument (GE Healthcare).

BiotinCAPture reagent (GE Healthcare) was immobilized on a CAP sensor chip at a concentration of 50 µg/ml and a flow rate of 2 µL/min for 300 s. The reference channel was treated in an analogous manner. Biotinylated huCD137-Fc (R&D systems) was captured on the chip surface for 300 s at a concentration of 1 µg/mL and at a flow rate of 5 µL/min on another channel.

To analyze whether the testing fusion proteins (SEQ ID NOs: 90 and 87, SEQ ID NOs: 86 and 91, SEQ ID NOs: 92 and 87, SEQ ID NOs: 86 and 93, SEQ ID NOs: 90 and 91, SEQ ID NO: 88, and SEQ ID NO: 89) compete with CD137L to bind CD137, either running buffer (HBS-EP+ buffer) or 500 nM huCD137L-His was applied to the chip surface for 180 s with a flow rate of 30 µL/min. Subsequently, the testing fusion proteins were applied to the prepared chip surface in HBS-EP+ buffer at a fixed concentration of 1 µM. The binding assay was carried out with a contact time of 180 s, a dissociation time of 15 s and a flow rate of 30 µL/min. As a control, buffer injections were included with the same parameters. Regeneration of the chip surface was achieved with injections of 6M Gua-HCl, 0,25M NaOH for 120 s at a flow rate of 10 µL/min, followed by an additional wash step with H₂O (120 s, 10 µl/min).

Representative examples for the relevant segment of the resulting sensorgrams are provided in **Figure 6** for the fusion proteins of SEQ ID NOs: 90 and 87, SEQ ID NOs: 86 and 91, SEQ ID NOs: 92 and 87, SEQ ID NOs: 86 and 93, SEQ ID NOs: 90 and 91, SEQ ID NO: 88, and SEQ ID NO: 89. The SPR trace for the binding of the respective fusion protein to huCD137-Fc alone is marked with an arrow with a solid stem. The SPR trace for the binding of the fusion protein to huCD137-Fc that has been saturated with huCD137L-His is marked with an arrow with a broken stem. The data shows that all fusion proteins bind to huCD137 in the presence of huCD137L, but with slightly reduced signals as compared to their binding to CD137 in the absence of CD137L. This suggests tested fusion proteins may be sterically hindered by the binding of CD137L to CD137 to some extent. This binding behavior of the fusion proteins (SEQ ID NOs: 90 and 87, SEQ ID NOs: 86 and 91, SEQ ID NOs: 92 and 87, SEQ ID NOs: 86 and 93, SEQ ID NOs: 90 and 91, SEQ ID NO: 88, and SEQ ID NO: 89) is similar to that of the anti-CD137 antibody SEQ ID NOs: 28 and 29.

### Example 9. Competition of the fusion proteins with PD-L1 in binding to PD-1 determined using ELISA

In order to demonstrate the ability of the fusion proteins to inhibit the interaction between PD-1 and PD-L1, a competitive ELISA format was used.

Recombinant huPD-1-His (Acrobiosystems) in PBS (1 µg/mL) was coated overnight on microtiter plates at 4°C. The plates were washed five times after each incubation step with 100 µL PBS-0.05%T (PBS supplemented with 0.05% (v/v) Tween 20). The plates were blocked with 2% BSA (w/v) in PBS-0.1%T (PBS supplemented with 0.1% (v/v) Tween 20) for 1 h at room temperature and subsequently washed again. Fusion proteins at different concentrations were mixed with 15 nM of recombinant huPD-L1-Fc (R&D systems) as a tracer and incubated for 1 h at room temperature. The mixtures of fusion proteins and the tracer were added to the plates and incubated for 20 min at room temperature following by five washing steps with 100 µL PBS-0.05%T. Subsequently, a 1:5000 dilution of goat-anti human IgG-Fc HRP (Jackson) was added to the wells and incubated for 1h. After an additional wash step, fluorogenic HRP substrate (QuantaBlu, Thermo) was added to each well, and the fluorescence intensity was detected using a fluorescence microplate reader.

Competition data of exemplary fusion proteins (SEQ ID NOs: 90 and 87, SEQ ID NOs: 86 and 91, SEQ ID NOs: 92 and 87, SEQ ID NOs: 86 and 93, and SEQ ID NOs: 90 and 91) are shown in **Figure 7****,** together with the fit curves resulting from a 1:1 sigmoidal binding fit, where the IC₅₀ value and the maximum signal were free parameters, and the slope was fixed to unity. The IC₅₀ values are summarized in **Table 9**. All bispecific fusion proteins showed clear inhibition of the PD-1/PD-L1 interaction with IC₅₀ values comparable to the antibody building block (SEQ ID NOs: 86 and 87) and a reference PD-L1 antibody (SEQ ID NOs: 26 and 27).

**Table 8. Competition of fusion proteins with PD-L1 for binding to PD-1**

| **SEQ ID NO** | **IC₅₀ [nM]** |
|---|---|
| 90 and 87 | 2.3 |
| 86 and 91 | 3.0 |
| 92 and 87 | 3.4 |
| 86 and 93 | 3.2 |
| 94 and 87 | 2.4 |
| 90 and 91 | 2.8 |
| 86 and 87 | 3.5 |
| 26 and 27 | 3.8 |

### Example 10. PD-L1 dependent T-cell co-stimulation using a CD137 Bioassay

The potential of selected fusion proteins to induce activation of CD137 signaling pathway in the presence of PD-L1 was assessed using a commercially available double stable transfected Jurkat cell line expressing CD137 and the *luc2* gene (humanized version of firefly luciferase) whereas *luc2* expression is driven by a NFκB-responsive element. In this bioassay, CD137 engagement results in CD137 intracellular signaling, leading to NFκB-mediated luminescence.

PD-L1 expressing colorectal cancer cell line RKO was cultured as described in **Example 7.** One day prior to the assay, RKO cells were plated at 1.25 × 10⁴ cells per well and allowed to adhere overnight at 37°C in a humidified 5% CO₂ atmosphere.

The next day, 3.75 × 10⁴ NF-kB-Luc2/CD137 Jurkat cells were added to each well, followed by the addition of various concentration, typically ranging from 0.001 nM to 5 nM, of fusion proteins or a reference CD137 antibody (SEQ ID NOs: 28 and 29). Plates were covered with a gas permeable seal and incubated at 37°C in a humidified 5% CO₂ atmosphere. After 4h, 30 µL Bio-Gio^{™} Reagent was added to each well and the bioluminescent signal was quantified using a luminometer (PHERAstar). Four-parameter logistic curve analysis was performed with GraphPad Prism^{®} to calculate EC₅₀ values (shared bottom, fixed slope) which are summarized in **Table 9**. To demonstrate the PD-L1 dependency of CD137 engagement by fusion proteins, the same experiment was performed in parallel in the absence of RKO cells. The assay was performed in triplicates.

The results of a representative experiment are depicted in **Figure 8A-8D****.** The data demonstrate that all tested fusion proteins (SEQ ID NOs: 90 and 87, SEQ ID NOs: 86 and 91, SEQ ID NOs: 92 and 87, and SEQ ID NOs: 86 and 93) induced a strong CD137 mediated T-cell co-stimulation. **Figure 8B** **and** **8D** shows that the activation of CD137 by fusion proteins is PD-L1 dependent, because no activation of the NF-kB-Luc2/CD137 Jurkat cells was detected in absence of PD-L1 expressing tumor cells. In contrast, the reference anti-CD137 mAb (SEQ ID NOs: 28 and 29) showed CD137 mediated T-cell co-stimulation regardless of the presence or absence of target cells.

**Table 9. Assessment of T-cell activation using a CD137 Bioassay**

| **SEQ ID NO** | **EC₅₀ [nM] With RKO cells** |
|---|---|
| 90 and 87 | 0.0809 |
| 86 and 91 | 0.0889 |
| 92 and 87 | 0.1811 |
| 86 and 93 | 0.2636 |
| 28 and 29 | 0.8135 |

### Example 11. Assessment of T-cell activation using human peripheral blood mononuclear cells (PBMCs)

A T cell assay was employed to assess the ability of the selected fusion proteins to co-stimulate T-cell responses as well as prevent co-inhibition mediated by PD-L1 binding to PD-1. For this purpose, fusion proteins at different concentrations were added to staphylococcal enterotoxin B (SEB) stimulated human peripheral blood mononuclear cells (PBMCs) and incubated for 4 days at 37°C. IL-2 secretion levels were measured in the supernatants.

PBMCs from healthy volunteer donors were isolated from buffy coats by centrifugation through a polysucrose density gradient (Biocoll, 1.077 g/mL, Biochrom), following Biochrom's protocols. The purified PBMCs were resuspended in a buffer consisting of 90% FCS and 10% DMSO, immediately frozen down and stored in liquid nitrogen until further use. For the assay, PBMCs were thawed and rested in culture media (RPMI 1640, Life Technologies) supplemented with 10% FCS and 1% Penicillin-Streptomycin (Life Technologies) for 16 h at 37°C in a humidified 5% CO₂ atmosphere.

The following procedure was performed using triplicates for each experimental condition: 2.5×10⁴ PBMCs were incubated in each well of a 384 well flat-bottom tissue culture plates in culture media. A dilution series of fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91), the building block PD-L1 antibody (SEQ ID NOs: 86 and 87), reference PD-L1 antibody (SEQ ID NOs: 26 and 27), reference CD137 antibody (SEQ ID NO: 28 and 29), a cocktail of the reference CD137 antibody (SEQ ID NOs: 28 and 29) and a PD-L1 antibody (SEQ ID NOs: 86 and 87 or SEQ ID NOs: 26 and 27), or an isotype control (SEQ ID NOs: 24 and 25), typically ranging from 10 to 0.002 nM, and SEB at 0.1 ng/ml were added to the respective wells. Plates were covered with a gas permeable seal (4titude) and incubated at 37°C in a humidified 5% CO₂ atmosphere for four days. Subsequently, IL-2 levels in the supernatant were assessed using the human IL-2 DuoSet kit (R&D Systems) as described in the following procedures.

384 well plates were coated for 2 h at room temperature with 1 µg/mL "Human IL-2 Capture Antibody" in PBS. Subsequently, wells were washed 5 times with 80 µl PBS supplemented with 0.05 % Tween (PBS-T). After 1 h blocking in PBS-0.05%T containing 1% casein (w/w), assay supernatants and a concentration series of IL-2 standard diluted in culture medium was transferred to respective wells and incubated overnight at 4°C. The next day, a mixture of 100 ng/mL goat anti-hlL-2-Bio detection antibody (R&D Systems) and 1µg/mL Sulfotag-labelled streptavidin (Mesoscale Discovery) in PBS-T containing 0.5% casein were added and incubated at room temperature for 1 h. After washing, 25 µL reading buffer (Mesoscale Discovery) was added to each well and the resulting electrochemiluminescence (ECL) signal was detected by a Mesoscale Discovery reader. Analysis and quantification were performed using Mesoscale Discovery software.

The result of a representative experiment is depicted in **Figure 9****.** Bispecific fusion proteins of SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91 are capable of inducing T-cell activation, which is demonstrated by increased IL-2 secretion levels compared to isotype control (hlgG4, Sigma). The strongest increase in IL-2 secretion is observed by the fusion proteins tetravalent to CD137 (SEQ ID NOs: 94 and 87 and SEQ ID NOs: 90 and 91), followed by the fusion protein bivalent to CD137 with the lipocalin mutein fused to the C-terminus of PD-L1-specific antibody (SEQ ID NOs: 90 and 87 and SEQ ID NOs: 86 and 91). The lowest increase is observed with the fusion proteins bivalent to CD137 with the lipocalin mutein fused to the N-terminus of PD-L1-specific antibody (SEQ ID NOs: 92 and 87 and SEQ ID NOs: 86 and 93), however, still comparable to the cocktail of a reference CD137 antibody (SEQ ID NOs: 28 and 29) and a reference PD-L1 antibody (SEQ ID NOs: 26 and 27). All fusion proteins show higher IL-2 secretion levels than the single building blocks, i.e., CD137-specific lipocalin mutein-Fc (SEQ ID NO: 88 or SEQ ID NO: 89) or the building block PD-L1 mAb (SEQ ID NOs: 86 and 87).

### Example 12. Assessment of T-cell activation in presence of tumor cells expressing different level of PD-L1

A further T cell assay was employed to assess the ability of the fusion proteins to co-stimulate T-cell activation in a PD-L1 target dependent manner. Fusion proteins were applied at different concentrations to anti-CD3 stimulated T cells, in the presence of tumor cell lines with different PD-L1 expression levels. Tested tumor cell lines include RKO (PD-L1 high), HCC827 (PD-L1 moderate) and Hep-G2 (PD-L1 negative). IL-2 secretion levels were measured in the supernatants.

PBMC from healthy volunteer donors were isolated from buffy coats as described in **Example 11.** T lymphocytes were further purified from PBMC by magnetic cell sorting using a Pan T cell purification Kit (Miltenyi Biotec GmbH) following the manufacturer's instructions. Purified Pan T cells were resuspended in a buffer consisting of 90% FCS and 10% DMSO, immediately frozen down and stored in liquid nitrogen until further use.

For the assay, T cells were thawed and rested in culture media (RPMI 1640, Life Technologies) supplemented with 10% FCS and 1% Penicillin-Streptomycin (Life Technologies) for 16 h at 37°C in a humidified 5% CO₂ atmosphere.

The following procedure was performed using triplicates for each experimental condition: flat-bottom tissue culture plates were pre-coated with 0.25 µg/mL anti-CD3 antibody for 1 h at 37°C and then washed twice with PBS. Tumor cell line RKO, HCC827 or Hep-G2 were treated for 30 min with 30 µg/ml mitomycin C (Sigma Aldrich) in order to block proliferation. Mitomycin treated tumor cells were then washed twice with PBS and plated at 2.5 × 10⁴ cells per well in culture medium to allow adhesion overnight at 37°C in a humidified 5% CO₂ atmosphere. The target cells had before been grown under standard conditions, detached using Accutase (PAA Laboratories), and resuspended in culture media.

On the next days, after washing the plates twice with PBS, 1.25 × 10⁴ T cells per well were added to the tumor cells. A dilution series of fusion proteins (SEQ ID NOs: 90 and 87, SEQ ID NOs: 86 and 91, SEQ ID NOs: 92 and 87, and SEQ ID NOs: 86 and 93), reference PD-L1 antibody (SEQ ID NOs: 26 and 27) and reference CD137 antibody (SEQ ID NO: 28 and 29) used alone or in combination, or an isotype control (SEQ ID NOs: 24 and 25), typically ranging from 0.005 nM to 10nM, were added to corresponding wells. Plates were covered with a gas permeable seal and incubated at 37°C in a humidified 5% CO₂ atmosphere for 3 days.

After 3 days of co-culturing, IL-2 level in the supernatant were assessed as described in **Example 11.**

Exemplary data are shown in **Figure 10****.** Co-culturing of Pan T cells with RKO cells (PD-L1 high) or HCC827 (PD-L1 moderate) in presence of the fusion proteins with the lipocalin mutein fused to the C-terminus of the PD-L1-specific antibody (SEQ ID NOs: 90 and 87 and SEQ ID NOs: 86 and 91) lead to a clear increase in IL-2 secretion compared to hIgG4 isotype control. The increase of IL-2 secretion induced by fusion proteins with the lipocalin mutein fused to the N-terminus of the PD-L1-specific antibody (SEQ ID NOs: 92 and 87 and SEQ ID NOs: 86 and 93) was weaker but still higher than a cocktail of a reference CD137 antibody (SEQ ID NOs: 28 and 29) and a reference PD-L1 antibody (SEQ ID NO: 26 and 27). No increase of IL-2 secretion was observed with a cocktail of the building blocks, CD137-specific lipocalin mutein (Fc fusion, SEQ ID NO: 89) and PD-L1 antibody (SEQ ID NO: 86 and 87). Additionally, co-culturing with Hep-G2 (PD-L1 negative) did no increase IL-2 secretion levels with any fusion proteins, but with the cocktail of a reference CD137 antiobdy (SEQ ID NO: 28 and 29) and a reference PD-L1 antibody (SEQ ID NO: 26 and 27).

The data indicate that the functional activity of fusion proteins, measured by their ability to activate T cells or increase IL-2 secretion, is PD-L1 dependent. In contrast, the T-cell activation or IL-2 secretion induced by the reference CD137 antibody (SEQ ID NOs: 28 and 29), when used in combination with the reference PD-L1 antibody (SEQ ID NOs: 26 and 27), is not necessarily PD-L1 dependent and hard to predict. Furthermore, the data show that the bispecific format of targeting PD-L1 and CD137 is superior to a cocktail of two separate molecules targeting CD137 and PD-L1 in presence of PD-L1 expressing target cells.

### Example 13. Storage stability assessment of fusion proteins

To assess storage stability, exemplary fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, SEQ ID NO: 88, and SEQ ID NO: 89) were incubated for 1 week at 37°C at a concentration of 1 mg/ml in PBS. Monomeric fusion proteins were subsequently determined using analytical size exclusion by applying 20 µg of sample onto a Superdex 200, 3.2/300 Increase (GE Healthcare) column at a flow rate of 0.15 ml/min and PBS as running buffer. All testing fusion peptides were stable after incubation of 1 week in PBS at 37 °C. Exemplary results are shown in **Figure 11A****.**

Further storage stability assessments were performed for a selected fusion protein of SEQ ID NOs: 90 and 87. The fusion protein at 20 mg/ml was incubated for four weeks at 40°C in 25 mM histidine, 60 mM NaCl, 200 mM arginine pH 6. Functional fusion protein content was measured in a quantitative ELISA setting, utilizing the simultaneous binding assay as described **Example 5.** Exemplary results are shown in **Figure 11B****.**

### Example 14. Mixed lymphocyte reaction (MLR) assessment with CD4⁺ T cells

A mixed lymphocyte reaction (MLR) assay was utilized to assess the ability of an exemplary fusion protein to induce CD4⁺ T-cell activation in the presence of antigen presenting cells. The fusion protein (SEQ ID NOs: 90 and 87) at various concentrations was tested in presence of monocyte derived dendritic cells (moDCs) and CD4⁺ T cells from mismatching healthy donors. After 6 days of culturing in the presence of tested molecules, the secretion of IL-2 and IFN-gamma were quantified in the supernatants.

PBMCs were purified from platelet apheresis blood pack using a Lymphoprep solution following manufacturer instructions (StemCell). Total CD4⁺ T lymphocytes were purified from PBMC using a Miltenyi kit and frozen in a solution of 90% FBS 10% DMSO. CD14⁺ monocytes were purified using CD14⁺ beads kit (Miltenyi) and used fresh.

MoDCs were obtained by culturing CD14⁺ monocytes in RPMI1640 plus 10% FBS and Pen/Strep (LifeTech) in the presence of 50 ng/mL of IL-4 and 100 ng/mL of GMCSF (Miltenyi) for 6 days at 2×10⁶ cells/mL. At day 3, 10 ml of fresh medium containing cytokines was added. Phenotype (CD14, CD1a, HLADR, PD-L1) was assessed at day 7 of differentiation by FACS.

10000 moDCs were cultured in presence of 50000 CD4 T⁺ cells in U bottom 96 wells in complete RPMI medium, in the presence of tested molecules for 6 days in RPMI in triplicate wells. At the end of the culture, supernatants were immediately frozen and stored for cytokine quantification.

IL-2 level was measured in the supernatants by using Luminex technology and exemplary data are shown in **Figure 12****.** **Figure 12A** shows the fusion protein (SEQ ID NOs: 90 and 87) was significantly better in IL-2 induction at 10 and 0.1 µg/mL as compared to the corresponding building blocks (SEQ ID NO: 89 and SEQ ID NOs: 86 and 87) alone or a reference CD137 or PD-L1 antibody (SEQ ID NOs: 28 and 29 or SEQ ID NOs: 26 and 27) in several sets of MLR experiments (N=8). **Figure 12B** indicates that the fusion protein (SEQ ID NOs: 90 and 87) induced a dose-dependent secretion of IL-2 as compared to an isotype antibody control. IL-2 levels induced by the fusion protein SEQ ID NOs: 90 and 87 were higher as compared to equimolar concentrations of the cocktail of a reference PD-L1 antibody (SEQ ID NOs: 26 and 27) and a reference CD137 antibody (SEQ ID NOs: 28 and 29), over concentrations ranging from 0.001 to 20 µg/mL.

### Example 15. Mixed lymphocyte reaction assessment with CD8⁺ T cells

Given reports of CD137 expression and activity in human CD8⁺ T cells, the ability of an exemplary fusion protein to induce CD8⁺ T-cell activation in the presence of antigen presenting cells was assessed in an MLR assay. The fusion protein (SEQ ID NOs: 90 and 87) was tested in presence of moDCs and total CD8⁺ T cells from mismatching healthy donors. After 6 days of culturing in presence of tested molecules, secretion of IL-2 and CD8 effector molecules (perforin, granzyme B, and granzyme A) were quantified in the supernatants.

PBMCs were purified from platelet apheresis blood pack using a Lymphoprep solution following manufacturer instructions (StemCell). Total CD8⁺ T lymphocytes were purified from PBMC using a Miltenyi kit and used fresh. CD14 positive monocytes were purified using CD14⁺ beads kit (Miltenyi) and used fresh.

MoDCs were obtained by culturing CD14⁺ monocytes in RPMI1640 plus 10% FBS and Pen/Strep (LifeTech) in the presence of 50 ng/mL of IL-4 and 100 ng/mL of GMCSF (Miltenyi) for 6 days at 2×10⁶ cells/mL. At day 3, 10 mL of fresh medium containing cytokines was added. Phenotype (CD14, CD1a, HLADR, PD-L1) was assessed at day 7 of differentiation by FACS.

10000 moDCs were cultured with 50000 CD8⁺ T cells, in the presence of tested molecules, in U bottom 96 wells (in triplet wells) in complete RPMI medium for 6 days. At the end of the culture, supernatants were immediately frozen and stored for secreted factor quantification.

IL-2, perforin, granzyme A, and granzyme B were quantified in the supernatants using Luminex technology. Exemplary data are shown in **Figure 13****.**

**Figure 13** indicates that the fusion protein (SEQ ID NOs: 90 and 87) showed increase in the secretion of IL-2, perforin, granzyme B, and granzyme A as compared to an equimolar concentration of a reference PD-L1 antibody (SEQ ID NOs: 26 and 27), a reference CD137 antibody (SEQ ID NOs: 28 and 29), or the cocktail of the two at 10 µg/mL (N=4). The data further suggest that the fusion protein (SEQ ID NOs: 90 and 87) has activity on cytotoxic CD8⁺ T cells.

### Example 16. Assessment of functional in vivo activity in a xenograft mouse model engrafted with human PBMCs

In order to investigate the *in vivo* activity of provided fusion proteins, cell line-derived xenograft mouse model will be used. Accordingly, a human cancer cell line will be implanted subcutaneously in immune deficient female NOG mice, delivered at the age of 4-6 weeks with at least 1 week of quarantine. After the tumors have been reaching volumes of approximately 80-100 mm³, mice will be substitutes with human PBMCs. Test compounds will be injected at least three times and tumor growth and activity will be constantly measured. After reaching study end, mice will be sacrificed. Intratumoral infiltration of CD3-, CD4- and CD8-positive cells will be assessed via immunohistochemistry. IFN-gamma RNAscope will be conducted as further read-out.

### Example 17. Epitope analysis of the fusion proteins

In order to evaluate epitopes the fusion proteins recognize, and whether they are clinically relevant, a competitive ELISA format was used to determine the competition between the fusion proteins and a reference CD137 antibody.

Microtiter plates were coated with the reference CD137 antibody SEQ ID NOs: 28 and 29 in PBS (4 µg/mL) at 4°C overnight. The plates were washed five times after each incubation step with 100 µL PBS-0.05%T (PBS supplemented with 0.05% (v/v) Tween 20). The plates were blocked with 2% BSA (w/v) in PBS-0.1%T (PBS supplemented with 0.1% (v/v) Tween 20) for 1 h at room temperature and subsequently washed again. Fusion proteins (SEQ ID NOs: 90 and 87 and SEQ ID NOs: 86 and 91), the CD137 specific lipocalin mutein (SEQ ID NO: 42), the reference CD137 antibody (SEQ ID NOs: 28 and 29), and a control antibody (SEQ ID NOs: 86 and 87) at different concentrations were mixed with 1 nM of biotinylated human CD137 Fc fusion (huCD137-Fc-bio) as a tracer and incubated for 1 h at room temperature. The mixtures of testing molecules and the tracer were added to the plates and incubated for 20 min at room temperature following by five washing steps with 100 µL PBS-0.05%T. Subsequently, a 1:5000 dilution of ExtrAvidin-HRP (Sigma-Aldrich) in PBS-0.1%T-2%BSA was added to the wells and incubated for 1 h. After an additional wash step, fluorogenic HRP substrate (QuantaBlu, Thermo) was added to each well, and the fluorescence intensity was detected using a fluorescence microplate reader.

Competition data for an exemplary experiment are shown in **Figure 14****,** where the x-axis represents testing molecule concentration and the y-axis represents the measured trace molecule concentration. The data were fit to a 1:1 sigmoidal curve, where the IC₅₀ value and the maximum signal were free parameters, and the slope was fixed to unity. The results demonstrate that exemplary fusion proteins (SEQ ID NOs: 90 and 87 and SEQ ID NOs: 86 and 91) compete with the CD137 antibody SEQ ID NOs: 28 and 29 for binding to CD137, suggesting the fusion proteins bind overlapping epitopes with the antibody.

**Table 10. Competition of fusion proteins**

| **SEQ ID NO** | **IC₅₀ [nM]** |
|---|---|
| 90 and 87 | 1.10 |
| 86 and 91 | 0.99 |
| 28 and 29 | 0.20 |
| 42 | 2.60 |

### Example 18. Assessment of T cell activation using a PD-1/PD-L1 blockade bioassay

The potential of selected fusion proteins to block PD-1/PD-L1 mediated suppression was assessed using PD-1-NFAT-luc Jurkat T cells (a Jurkat cell line engineered to express PD-1 and the *luc* gene (firefly luciferase gene) driven by an NFAT response element (NFAT-RE)), co-cultured with PD-L1 aAPC/CHO-K1 cells (CHO-K1 cells expressing human PD-L1 and an engineered cell surface protein designed to activate cognate TCRs in an antigen-independent manner). In this bioassay, when PD-1-NFAT-luc Jurkat T cells and PD-L1 aAPC/CHO-K1 cells are co-cultured, the PD-1/PD-L1 interaction inhibits TCR signaling and NFAT-RE-mediated luminescence. Addition of a PD-1/PD-L1 blocking agent, such as fusion proteins specific for CD137 and PD-L1 as described herein, releases the inhibitory signal and results in TCR activation and NFAT-RE-mediated luminescence.

PD-L1 aAPC/CHO-K1 cells were grown in Ham's F12 medium supplemented with 10% FCS and plated at 8.00 × 10³ cells per well and allowed to adhere overnight at 37°C in a humidified 5% CO₂ atmosphere. On the next day, the culture media was discarded. 1.00 × 10⁴ PD-1-NFAT-luc Jurkat T cells were added to each well, followed by the addition of various concentrations, typically ranging from 0.005 nM to 50 nM. of a fusion protein (SEQ ID NOs: 90 and 87) or a PD-L1 antibody (SEQ ID NOs: 86 and 87 or SEQ ID NOs: 26 and 27). Plates were covered with a gas permeable seal and incubated at 37°C in a humidified 5% CO₂ atmosphere. After 6h, 30 µL Bio-Glo^{™} Reagent was added to each well and the bioluminescent signal was quantified using a luminometer. Four-parameter logistic curve analysis was performed with GraphPad Prism^{®} to calculate EC₅₀ values which are summarized in **Table 11.** The assay was performed in triplicates.

The results of a representative experiment are depicted in **Figure 15****.** The data demonstrate the tested fusion protein inhibits PD-1/PD-L1 blockade and activates T cells in a dose-dependent manner, with an EC50 value comparable to that of a PD-L1 antibody (SEQ ID NOs: 86 and 87 or SEQ ID NOs: 26 and 27). As negative controls, neither the reference CD137 antibody (SEQ ID NOs: 28 and 29) or and the isotype control antibody (SEQ ID NOs: 24 and 25) leads to an increase in luminescence signal.

**Table 11. Assessment of T-cell activation using a PD-1/PD-L1 blockade bioassay**

| **SEQ ID NO** | **EC₅₀ [nM]** |
|---|---|
| 90 and 87 | 0.49 |
| 86 and 91 | 0.58 |
| 28 and 29 | 0.46 |

### Example 19. Assessment of T-cell activation using human PBMCs

An additional T cell assay was employed to assess the ability of the selected fusion proteins to co-stimulate T-cell responses, where fusion proteins at different concentrations were added to SEB stimulated human PBMCs and incubated for 3 days at 37°C. IL-2 secretion levels were measured in the supernatants.

PBMCs from healthy volunteer donors were isolated and stored as described in Example 11. For the assay, PBMCs were thawed and rested in culture media (RPMI 1640, Life Technologies) supplemented with 10% FCS and 1% Penicillin-Streptomycin (Life Technologies) for 24 h at 37°C in a humidified 5% CO₂ atmosphere.

The following procedure was performed using triplicates for each experimental condition: 2.5×10⁴ PBMCs were incubated in each well of a 384 well flat-bottom tissue culture plates in culture media. A dilution series of a selected fusion protein (SEQ ID NOs: 90 and 87), the building block PD-L1 antibody (SEQ ID NOs: 86 and 87), a reference CD137 antibody (SEQ ID NO: 28 and 29) used alone or in combination with a reference PD-L1 antibody (SEQ ID NOs: 26 and 27), or an isotype control (SEQ ID NOs: 24 and 25), typically ranging from 0.0002 to 10 nM, and 0.1 ng/ml SEB were added to the respective wells. Plates were covered with a gas permeable seal (4titude) and incubated at 37°C in a humidified 5% CO₂ atmosphere for three days. Subsequently, IL-2 in the supernatant were assessed as described in **Example 11.**

The results of a representative experiment are depicted in **Figure 16****.** The EC₅₀ values of the testing molecules for inducing IL-2 secretion are summarized in **Table 12.** The bispecific fusion protein of SEQ ID NOs: 90 and 87 induces a strong dose-dependent increase in IL-2 secretion, to higher levels as compared to the building block PD-L1 antibody, the reference CD137 antibody and the cocktail of the reference PD-L1 and CD137 antibodies, as well as decreases the effective EC₅₀ value relative to the PD-L1 and CD137 antibodies used alone or in combination..

**Table 12. Assessment of T-cell activation using human PBMCs**

| **Donor** | **EC₅₀ [nM]** | | | |
|---|---|---|---|---|
| | **SEQ ID NOs: 90 and 87** | **SEQ ID NOs: 86 and 87** | **SEQ ID NOs: 26 and 27** | **SEQ ID NOs: 28 and 29 + SEQ ID NOs: 86 and 87** |
| **#1** | 0.019 | 0.061 | 0.250 | 0.279 |
| **#2** | 0.026 | 0.057 | 0.134 | 0.089 |

### Example 20. Assessment of PD-L1 dependent T-cell activation induced by the fusion proteins

The PD-L1 target dependent T-cell costimulation by the fusion proteins was further analyzed using a T-cell activation assay. Fusion proteins were applied at different concentrations to anti-CD3 stimulated T cells, co-cultured with human PD-L1 transfected or mock transfected Flp-In-CHO cells. IL-2 secretion levels were measured in the supernatants.

PBMC from healthy volunteer donors were isolated from buffy coats as described in **Example 11.** T lymphocytes were further purified and stored as described in **Example 12.**

For the assay, T cells were thawed and rested in culture media (RPMI 1640, Life Technologies) supplemented with 10% FCS and 1% Penicillin-Streptomycin (Life Technologies) for overnight at 37°C in a humidified 5% CO₂ atmosphere.

The following procedure was performed using triplicates for each experimental condition: flat-bottom tissue culture plates were pre-coated with 0.25 µg/mL anti-CD3 antibody for 2 h at 37°C and then washed twice with PBS. CHO cells, transfected with human PD-L1 or mock transfected, were treated for 30 min with 30 µg/ml mitomycin C (Sigma Aldrich) in order to block proliferation. Mitomycin treated cells were then washed twice with PBS and plated at 1.0 × 10⁷ cells per well in culture medium to allow adhesion overnight at 37°C in a humidified 5% CO₂ atmosphere. The CHO cells had before been grown under standard conditions, detached using Accutase (PAA Laboratories), and resuspended in culture media.

On the next days, 8.33 × 10³ T cells per well were added to the CHO cells. A dilution series of an exemplary fusion protein SEQ ID NOs: 90 and 87, the building block PD-L1 antibody (SEQ ID NOs: 86 and 87), reference CD137 antibody (SEQ ID NOs: 28 and 29), and a cocktail of reference PD-L1 antibody (SEQ ID NOs: 26 and 27) and reference CD137 antibody (SEQ ID NO: 28 and 29), or an isotype control (SEQ ID NOs: 24 and 25), typically ranging from 0.003 nM to 50nM, were added to corresponding wells. Plates were covered with a gas permeable seal and incubated at 37°C in a humidified 5% CO₂ atmosphere for 2 days.

After 2 days of co-culturing, IL-2 levels in the supernatant were assessed as described in **Example 11.**

Exemplary data are shown in **Figure 17****.** Co-culturing of Pan T cells with CHO cells transfected with human PD-L1 in presence of the fusion protein (SEQ ID NOs: 90 and 87 and SEQ ID NOs: 86 and 91) led to strong dose-dependent IL-2 secretion compared to hIgG4 isotype control and is much stronger than the reference antibodies where only slight increase of IL-2 secretion was observed for the reference CD137 antibody or the cocktail of the reference CD137 antibody and reference PD-L1 antibody. When co-culturing with mock-transfected CHO cells (PD-L1 negative), only the reference CD137 antibody and the cocktail of the reference CD137 antibody and reference PD-L1 antibody showed slight dose-dependent increase in IL-2 secretion. The results illustrate that the activation of T cells by fusion proteins is PD-L1 dependent, versus the reference CD137 antibody (SEQ ID NOs: 28 and 29) showed CD137 mediated T-cell co-stimulation regardless of the presence or absence of target cells.

### Example 21. Pharmacokinetics of fusion proteins in mice

Analyses of the pharmacokinetics of representative fusion proteins (SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91) were performed in mice. Male CD-1 mice approximately 5 weeks of age (3 mice per timepoint; Charles River Laboratories, Research Models and Services, Germany GmbH) were injected into a tail vein with a fusion protein at a dose of 10 mg/kg. The test articles were administered as a bolus using a volume of 5 mL/kg. Plasma samples from the mice were obtained at the timepoints of 5 min, 1 h, 4 h, 8 h, 24 h, 48 h, 4 d, 8 d, 14 d, 21 d, and 28 d. Sufficient whole blood - taken under isoflurane anesthesia - was collected to obtain at least 100 µL Li-Heparin plasma per animal and time. Drug levels were detected using a Sandwich ELISA detecting the full bispecific construct via the targets PD-L1 and CD137. The data were fitted using a two-compartmental model using Prism GraphPad 5 software.

**Figure 18** shows plots of the plasma concentration over time for the fusion proteins SEQ ID NOs: 90 and 87, SEQ ID NOs: 86 and 91, SEQ ID NOs: 92 and 87, SEQ ID NOs: 86 and 93, SEQ ID NOs: 94 and 87, and SEQ ID NOs: 90 and 91, plotted together with the values obtained for the building block PD-L1 antibody (SEQ ID NOs: 86 and 87) as a reference. The pharmacokinetics looked similar in all cases. Starting from a plasma concentration of around 200 µg/mL, plasma levels fell to a level of around 50 µg/mL within 48 hours, and then further decrease at a much slower rate to a level of around 10 µg/mL at the end of the experiment after 28 days. A non-compartmental analysis was applied to this data. The terminal half-lives are summarized in **Table 13.**

The data demonstrate that the fusion proteins have long, antibody-like terminal half-lives in mice. Because the assay employed to determine fusion proteins plasma concentrations requires a retained activity both towards PD-L1 and CD137, the result also demonstrates that the bispecific molecules remain intact over the time course of 28 days.

**Table 13. Terminal half-lives in mice determined using a non-compartmental analysis**

| **SEQ ID NO** | **Terminal half-life [h]** |
|---|---|
| 90 and 87 | 295 |
| 92 and 87 | 346 |
| 86 and 93 | 332 |
| 94 and 87 | 209 |
| 90 and 91 | 250 |
| 86 and 87 | 390 |

### Example 22. Pharmacokinetics of fusion proteins in mice

An analysis of the pharmacokinetics of a representative fusion protein SEQ ID NOs: 90 and 87 were performed in mice and compared with two previously described CD137- and PD-L1-binding fusion proteins (SEQ ID NO: 147 and SEQ ID NO: 148). Male CD-1 mice approximately 5 weeks of age (2 mice per timepoint; Charles River Laboratories, Research Models and Services, Germany GmbH) were injected into a tail vein with the respective molecule at a dose of 2 mg/kg. Plasma samples from the mice were obtained at the timepoints of 5 min, 24 h, 168 h, and 336 h. Sufficient whole blood - taken under isoflurane anaesthesia - was collected to obtain at least 30-50 µL Li-Heparin plasma per animal and time.

Plasma drug levels were then analyzed with ELISA. HuCD137-His (human CD137 with a C-terminal polyhistidine tag) was dissolved in PBS (1 µg/mL) and coated overnight on microtiter plates at 4°C. The plate was washed after each incubation step with 80 µL PBS supplemented with 0.05% (v/v) Tween 20 five times. The plates were blocked with PBS/BSA/Tween (PBS containing 2% BSA (w/v) and 0.1% (v/v) Tween 20) for 1 h at room temperature and subsequently washed. Plasma samples were diluted in PBS/BSA/Tween to 20% plasma concentration, added to the wells, and incubated for 1 h at room temperature. Another wash step followed. Bound agents under study were detected after 1 h incubation with a mixture of biotinylated human PD-L1 and Streptavidin SULFOTAG (Mesoscale Discovery) at 1 µg/mL each diluted in PBS containing 2% BSA (w/v) and 0.1% (v/v) Tween 20. After an additional wash step, 35 µL reading buffer was added to each well and the electrochemiluminescence (ECL) signal of every well was read using a Mesoscale Discovery reader. Data were transferred to Excel for analysis and quantification. A calibration curve with standard protein dilutions was prepared.

**Figure 19** shows plots of the plasma concentration over time for SEQ ID NOs: 90 and 87, SEQ ID NO: 147, and SEQ ID NO: 148. SEQ ID NOs: 90 and 87 displays a favorable pharmacokinetic profile or an antibody-like pharmacokinetics, while SEQ ID NO: 147 and SEQ ID NO: 148 do not. As described herein, a favorable pharmacokinetic profile or an antibody-like pharmacokinetics may be considered to be achieved if % of cₘₐₓ was above 10 % after 336 h.

### VI. NON-PATENT REFERENCES

1. GATO-CANAS, M., ZUAZO, M., ARASANZ, H., IBANEZ-VEA, M., LORENZO, L., FERNANDEZ-HINOJAL, G., VERA, R., SMERDOU, C., MARTISOVA, E., AROZARENA, I., WELLBROCK, C., LLOPIZ, D., RUIZ, M., SAROBE, P., BRECKPOT, K., KOCHAN, G. & ESCORS, D. 2017. PDL1 Signals through Conserved Sequence Motifs to Overcome Interferon-Mediated Cytotoxicity. Cell Rep, 20, 1818-1829.
2. KARWACZ, K., BRICOGNE, C., MACDONALD, D., ARCE, F., BENNETT, C. L., COLLINS, M. & ESCORS, D. 2011. PD-L1 co-stimulation contributes to ligand-induced T cell receptor down-modulation on CD8+ T cells. EMBO Mol Med, 3, 581-92.
3. ARASANZ, H., GATO-CANAS, M., ZUAZO, M., IBANEZ-VEA, M., BRECKPOT, K., KOCHAN, G. & ESCORS, D. 2017. PD1 signal transduction pathways in T cells. Oncotarget, 8, 51936-51945.
4. PATSOUKIS, N., BARDHAN, K., CHATTERJEE, P., SARI, D., LlU, B., BELL, L. N., KAROLY, E. D., FREEMAN, G. J., PETKOVA, V., SETH, P., LI, L. & BOUSSIOTIS, V. A. 2015. PD-1 alters T-cell metabolic reprogramming by inhibiting glycolysis and promoting lipolysis and fatty acid oxidation. Not Commun, 6, 6692.
5. XU-MONETTE, Z. Y., ZHANG, M., LI, J. & YOUNG, K. H. 2017. PD-1/PD-L1 Blockade: Have We Found the Key to Unleash the Antitumor Immune Response? Front Immunol, 8, 1597.
6. LI, S. Y. & LlU, Y. 2013. Immunotherapy of melanoma with the immune costimulatory monoclonal antibodies targeting CD137. Clin Pharmacol, 5, 47-53.
7. SNELL, L. M., LIN, G. H., MCPHERSON, A. J., MORAES, T. J. & WATTS, T. H. 2011. T-cell intrinsic effects of GITR and 4-1BB during viral infection and cancer immunotherapy. Immunol Rev, 244, 197-217.
8. WYZGOL, A., MULLER, N., FICK, A., MUNKEL, S., GRIGOLEIT, G. U., PFIZENMAIER, K. & WAJANT, H. 2009. Trimer stabilization, oligomerization, and antibody-mediated cell surface immobilization improve the activity of soluble trimers of CD27L, CD40L, 41BBL, and glucocorticoid-induced TNF receptor ligand. J Immunol, 183, 1851-61.
9. YAO, S., ZHU, Y. & CHEN, L. 2013. Advances in targeting cell surface signalling molecules for immune modulation. Not Rev Drug Discov, 12, 130-46.
10. MELERO, I., BACH, N., HELLSTROM, K. E., ARUFFO, A., MITTLER, R. S. & CHEN, L. 1998. Amplification of tumor immunity by gene transfer of the co-stimulatory 4-1BB ligand: synergy with the CD28 co-stimulatory pathway. Eur J Immunol, 28, 1116-21.
11. YANG, Y., YANG, S., YE, Z., JAFFAR, J., ZHOU, Y., CUTTER, E., LIEBER, A., HELLSTROM, I. & HELLSTROM, K. E. 2007. Tumor cells expressing anti-CD137 scfv induce a tumor-destructive environment. Cancer Res, 67, 2339-44.
12. ZHANG, H., KNUTSON, K. L., HELLSTROM, K. E., DISIS, M. L. & HELLSTROM, I. 2006. Antitumor efficacy of CD137 ligation is maximized by the use of a CD137 single-chain Fv-expressing whole-cell tumor vaccine compared with CD137-specific monoclonal antibody infusion. Mol Cancer Ther, 5, 149-55.
13. YE, Z., HELLSTROM, I., HAYDEN-LEDBETTER, M., DAHLIN, A., LEDBETTER, J. A. & HELLSTROM, K. E. 2002. Gene therapy for cancer using single-chain Fv fragments specific for 4-1BB. Not Med, 8, 343-8.
14. MARTINET, O., DIVINO, C. M., ZANG, Y., GAN, Y., MANDELI, J., THUNG, S., PAN, P. Y. & CHEN, S. H. 2002. T cell activation with systemic agonistic antibody versus local 4-1BB ligand gene delivery combined with interleukin-12 eradicate liver metastases of breast cancer. Gene Ther, 9, 786-92.
15. YE, Q., SONG, D. G., POUSSIN, M., YAMAMOTO, T., BEST, A., LI, C., COUKOS, G. & POWELL, D. J., JR. 2014. CD137 accurately identifies and enriches for naturally occurring tumor-reactive T cells in tumor. Clin Cancer Res, 20, 44-55.
16. CHACON, J. A., WU, R. C., SUKHUMALCHANDRA, P., MOLLDREM, J. J., SARNAIK, A., PILON-THOMAS, S., WEBER, J., HWU, P. & RADVANYI, L. 2013. Co-stimulation through 4-1BB/CD137 improves the expansion and function of CD8(+) melanoma tumor-infiltrating lymphocytes for adoptive T-cell therapy. PLoS One, 8, e60031.
17. FISHER, T. S., KAMPERSCHROER, C., OLIPHANT, T., LOVE, V. A., LIRA, P. D., DOYONNAS, R., BERGQVIST, S., BAXI, S. M., ROHNER, A., SHEN, A. C., HUANG, C., SOKOLOWSKI, S. A. & SHARP, L. L. 2012. Targeting of 4-1BB by monoclonal antibody PF-05082566 enhances T-cell function and promotes anti-tumor activity. Cancer Immunol Immunother, 61, 1721-33.
18. SKERRA, A. 2000. Lipocalins as a scaffold. Biochim Biophys Acta, 1482, 337-50.
19. FLOWER, D. R., NORTH, A. C. & SANSOM, C. E. 2000. The lipocalin protein family: structural and sequence overview. Biochim Biophys Acta, 1482, 9-24.
20. FLOWER, D. R. 1996. The lipocalin protein family: structure and function. Biochem J, 318 ( Pt 1), 1-14.
21. ALTSCHUL, S. F., MADDEN, T. L., SCHAFFER, A. A., ZHANG, J., ZHANG, Z., MILLER, W. & LIPMAN, D. J. 1997. Gapped BLAST and PSI-BLAST: a new generation of protein database search programs. Nucleic Acids Res, 25, 3389-402.
22. ALTSCHUL, S. F., GISH, W., MILLER, W., MYERS, E. W. & LIPMAN, D. J. 1990. Basic local alignment search tool. J Mol Biol, 215, 403-10.
23. SMITH, T. F. & WATERMAN, M. S. 1981. Identification of common molecular subsequences. J Mol Biol, 147, 195-7.
24. WARD, E. S., GUSSOW, D., GRIFFITHS, A. D., JONES, P. T. & WINTER, G. 1989. Binding activities of a repertoire of single immunoglobulin variable domains secreted from Escherichia coli. Nature, 341, 544-6.
25. HOLLIGER, P., PROSPERO, T. & WINTER, G. 1993. "Diabodies": small bivalent and bispecific antibody fragments. Proc Natl Acad Sci U S A, 90, 6444-8.
26. JOHNSON, G. & WU, T. T. 2000. Kabat database and its applications: 30 years after the first variability plot. Nucleic Acids Res, 28, 214-8.
27. EHRENMANN, F., KAAS, Q. & LEFRANC, M. P. 2010. IMGT/3Dstructure-DB and IMGT/DomainGapAlign: a database and a tool for immunoglobulins or antibodies, T cell receptors, MHC, IgSF and MhcSF. Nucleic Acids Res, 38, D301-7.
28. BULLIARD, Y., JOLICOEUR, R., ZHANG, J., DRANOFF, G., WILSON, N. S. & BROGDON, J. L. 2014. OX40 engagement depletes intratumoral Tregs via activating FcgammaRs, leading to antitumor efficacy. Immunol Cell Biol, 92, 475-80.
29. BULLIARD, Y., JOLICOEUR, R., WINDMAN, M., RUE, S. M., ETTENBERG, S., KNEE, D. A., WILSON, N. S., DRANOFF, G. & BROGDON, J. L. 2013. Activating Fc gamma receptors contribute to the antitumor activities of immunoregulatory receptor-targeting antibodies. J Exp Med, 210, 1685-93.
30. SILVA, J. P., VETTERLEIN, O., JOSE, J., PETERS, S. & KIRBY, H. 2015. The S228P mutation prevents in vivo and in vitro IgG4 Fab-arm exchange as demonstrated using a combination of novel quantitative immunoassays and physiological matrix preparation. J Biol Chem, 290, 5462-9.
31. GLAESNER, W., VICK, A. M., MILLICAN, R., ELLIS, B., TSCHANG, S. H., TIAN, Y., BOKVIST, K., BRENNER, M., KOESTER, A., PORKSEN, N., ETGEN, G. & BUMOL, T. 2010. Engineering and characterization of the long-acting glucagon-like peptide-1 analogue LY2189265, an Fc fusion protein. Diabetes Metab Res Rev, 26, 287-96.
32. DALL'ACQUA, W. F., KIENER, P. A. & WU, H. 2006. Properties of human IgGls engineered for enhanced binding to the neonatal Fc receptor (FeRn). J Biol Chem, 281, 23514-24.
33. ZALEVSKY, J., CHAMBERLAIN, A. K., HORTON, H. M., KARKI, S., LEUNG, I. W., SPROULE, T. J., LAZAR, G. A., ROOPENIAN, D. C. & DESJARLAIS, J. R. 2010. Enhanced antibody half-life improves in vivo activity. Not Biotechnol, 28, 157-9.
34. SHIELDS, R. L., NAMENUK, A. K., HONG, K., MENG, Y. G., RAE, J., BRIGGS, J., XIE, D., LAI, J., STADLEN, A., LI, B., FOX, J. A. & PRESTA, L. G. 2001. High resolution mapping of the binding site on human IgG1 for Fc gamma RI, Fc gamma RII, Fc gamma RIII, and FcRn and design of IgG1 variants with improved binding to the Fc gamma R. J Biol Chem, 276, 6591-604.
35. ALTSHULER, E. P., SEREBRYANAYA, D. V. & KATRUKHA, A. G. 2010. Generation of recombinant antibodies and means for increasing their affinity. Biochemistry (Mosc), 75, 1584-605.
36. HARLOW, E. & LANE, D. 1999. Using antibodies : a laboratory manual, Cold Spring Harbor, N.Y., Cold Spring Harbor Laboratory Press.
37. HARLOW, E. & LANE, D. 1988. Antibodies : a laboratory manual, Cold Spring Harbor, NY, Cold Spring Harbor Laboratory.
38. LI, J., SAI, T., BERGER, M., CHAO, Q., DAVIDSON, D., DESHMUKH, G., DROZDOWSKI, B., EBEL, W., HARLEY, S., HENRY, M., JACOB, S., KLINE, B., LAZO, E., ROTELLA, F., ROUTHIER, E., RUDOLPH, K., SAGE, J., SIMON, P., YAO, J., ZHOU, Y., KAVURU, M., BONFIELD, T., THOMASSEN, M. J., SASS, P. M., NICOLAIDES, N. C. & GRASSO, L. 2006. Human antibodies for immunotherapy development generated via a human B cell hybridoma technology. Proc Natl Acad Sci USA, 103, 3557-62.
39. KOZBOR, D. & RODER, J. C. 1983. The production of monoclonal antibodies from human lymphocytes. Immunol Today, 4, 72-9.
40. COLE, S. P., CAMPLING, B. G., LOUWMAN, I. H., KOZBOR, D. & RODER, J. C. 1984. A strategy for the production of human monoclonal antibodies reactive with lung tumor cell lines. Cancer Res, 44, 2750-3.
41. HOLLIGER, P. & HUDSON, P. J. 2005. Engineered antibody fragments and the rise of single domains. Not Biotechnol, 23, 1126-36.
42. PERVAIZ, S. & BREW, K. 1987. Homology and structure-function correlations between alpha 1-acid glycoprotein and serum retinol-binding protein and its relatives. FASEB J, 1, 209-14.
43. SAMBROOK, J. & RUSSELL, D. W. 2001. Molecular cloning : a laboratory manual, Cold Spring Harbor, N.Y., Cold Spring Harbor Laboratory Press.
44. FLOWER, D. R. 2000. Beyond the superfamily: the lipocalin receptors. Biochim Biophys Acta, 1482, 327-36.
45. BREUSTEDT, D. A., KORNDORFER, I. P., REDL, B. & SKERRA, A. 2005. The 1.8-A crystal structure of human tear lipocalin reveals an extended branched cavity with capacity for multiple ligands. J Biol Chem, 280, 484-93.
46. SCHMIDT, T. G., KOEPKE, J., FRANK, R. & SKERRA, A. 1996. Molecular interaction between the Strep-tag affinity peptide and its cognate target, streptavidin. J Mol Biol, 255, 753-66.
47. VAJO, Z. & DUCKWORTH, W. C. 2000. Genetically engineered insulin analogs: diabetes in the new millenium. Pharmacol Rev, 52, 1-9.
48. FUERTGES, F. & ABUCHOWSKI, A. 1990. The clinical efficacy of poly(ethylene glycol)-modified proteins. Journal of Controlled Release, 11, 139-148.
49. DENNIS, M. S., ZHANG, M., MENG, Y. G., KADKHODAYAN, M., KIRCHHOFER, D., COMBS, D. & DAMICO, L. A. 2002. Albumin binding as a general strategy for improving the pharmacokinetics of proteins. J Biol Chem, 277, 35035-43.
50. KONIG, T. & SKERRA, A. 1998. Use of an albumin-binding domain for the selective immobilisation of recombinant capture antibody fragments on ELISA plates. J Immunol Methods, 218, 73-83.
51. OSBORN, B. L., OLSEN, H. S., NARDELLI, B., MURRAY, J. H., ZHOU, J. X., GARCIA, A., MOODY, G., ZARITSKAYA, L. S. & SUNG, C. 2002. Pharmacokinetic and pharmacodynamic studies of a human serum albumin-interferon-alpha fusion protein in cynomolgus monkeys. J Pharmacol Exp Ther, 303, 540-8.
52. LOWMAN, H. B. 1997. Bacteriophage display and discovery of peptide leads for drug development. Annu Rev Biophys Biomol Struct, 26, 401-24.
53. RODI, D. J. & MAKOWSKI, L. 1999. Phage-display technology--finding a needle in a vast molecular haystack. Curr Opin Biotechnol, 10, 87-93.
54. VENTURI, M., SEIFERT, C. & HUNTE, C. 2002. High level production of functional antibody Fab fragments in an oxidizing bacterial cytoplasm. J Mol Biol, 315, 1-8.
55. BRUCKDORFER, T., MARDER, O. & ALBERICIO, F. 2004. From production of peptides in milligram amounts for research to multi-tons quantities for drugs of the future. Curr Pharm Biotechnol, 5, 29-43.

## Claims

1. A fusion protein that is capable of binding both CD137 and PD-L1, wherein the fusion protein comprises two subunits, wherein the first subunit comprises a full-length immunoglobulin that is specific for PD-L1, and wherein the second subunit comprises a lipocalin mutein that is specific for CD137,
wherein the fusion protein comprises amino acid sequences having at least 90% sequence identity to the amino acid sequences shown in SEQ ID NOs: 90 and 87,
wherein the heavy chain of the immunoglobulin comprises the following set of CDR sequences: GFSLSNYD (HCDR1, SEQ ID NO: 60), IWTGGAT (HCDR2, SEQ ID NO: 61), and VRDSNYRYDEPFTY (HCDR3, SEQ ID NO: 62), and the light chain of the immunoglobulin comprises the following set of CDR sequences: QSIGTN (LCDR1, SEQ ID NO: 63), YAS (LCDR2), and QQSNSWPYT (LCDR3, SEQ ID NO: 64), and wherein the amino acid sequence of the lipocalin mutein comprises the following set of mutated amino acid residues in comparison with the linear polypeptide sequence of mature hNGAL as shown in SEQ ID NO: 2: Gin 28 → His; Leu 36 → Gin; Ala 40 → lie; lie 41 → Arg; Gin 49 → Ile; Tyr 52 → Met; Asn 65 → Asp; Ser 68 → Met; Leu 70 → Lys; Arg 72 → Asp; Lys 73 → Asp; Asp 77 → Met; Trp 79 → Asp; Arg 81 → Trp; Cys 87 → Ser; Asn 96 → Lys; Tyr 100 → Phe; Leu 103 → His; Tyr 106 → Ser; Lys 125 → Phe; Ser 127 → Phe; Tyr 132 → Glu; and Lys 134 → Tyr.

2. The fusion protein of claim 1, wherein the amino acid sequence of the lipocalin mutein has at least 90% sequence identity to the amino acid sequence of SEQ ID NO: 42.

3. The fusion protein of claim 1 or 2, wherein the amino acid sequence of the lipocalin mutein comprises the amino acid sequence of SEQ ID NO: 42.

4. The fusion protein of any one of claims 1-3, wherein the second subunit is linked at the N-terminus via a linker to the C-terminus of each heavy chain constant region (CH) of the first subunit, wherein the linker is preferably the linker of SEQ ID NO: 13.

5. The fusion protein of any one of claims 1-4, wherein the immunoglobulin comprises a heavy chain variable region and a light chain variable region comprising the amino acid sequences as shown in SEQ ID NOs: 77 and 82.

6. The fusion protein of any one of claims 1-5, wherein the immunoglobulin comprises a heavy chain and a light chain comprising the amino acid sequences as shown in SEQ ID NOs: 86 and 87.

7. The fusion protein of any one of claims 1-5, wherein the immunoglobulin has an IgG4 backbone, wherein the IgG4 backbone preferably has one or more of the following mutations: S228P, N297A, F234A, L235A, M428L, N434S, M252Y, S254T, and T256E, according to the EU index of Kabat.

8. The fusion protein of any one of claims 1-5 or 7 wherein the fusion protein comprises amino acid sequences having at least 92%, at least 95%, at least 97%, at least 98%, or higher sequence identity to the amino acid sequences shown in SEQ ID NOs: 90 and 87.

9. The fusion protein of any one of claims 1-8, wherein the fusion protein comprises the amino acid sequences shown in SEQ ID NOs: 90 and 87.

10. A nucleic acid molecule comprising a nucleotide sequence encoding the fusion protein of any one of claims 1-9, wherein preferably
(i) the nucleic acid molecule is operably linked to a regulatory sequence to allow expression of said nucleic acid molecule; or
(ii) the nucleic acid molecule is comprised in a vector or in a phagemid vector.

11. A host cell containing a nucleic acid molecule of claim 10.

12. A method of producing the fusion protein of any one of claims 1-9, wherein the fusion protein is produced starting from the nucleic acid coding for the fusion protein, wherein the fusion protein is preferably produced in a bacterial or eukaryotic host organism and is isolated from this host organism or its culture.

13. A pharmaceutical composition comprising a fusion protein of any one of claims 1-9.

14. A fusion protein of any one of claims 1-9 for use in therapy.

15. The fusion protein for the use of claim 14, wherein the use is in the treatment of cancer.

## Patentansprüche

1. Fusionsprotein, das in der Lage ist, sowohl CD137 als auch PD-L1 zu binden, wobei das Fusionsprotein zwei Untereinheiten umfasst, wobei die erste Untereinheit ein für PD-L1 spezifisches Volllängen-Immunglobulin umfasst, und wobei die zweite Untereinheit ein für CD137 spezifisches Lipocalin-Mutein umfasst,
wobei das Fusionsprotein Aminosäuresequenzen umfasst, die mindestens 90 % Sequenzidentität mit den Aminosäuresequenzen aufweisen, die in SEQ ID NO: 90 und 87 gezeigt sind,
wobei die schwere Kette des Immunglobulins den folgenden Satz an CDR-Sequenzen umfasst: GFSLSNYD (HCDR1, SEQ ID NO: 60), IWTGGAT (HCDR2, SEQ ID NO: 61) und VRDSNYRYDEPFTY (HCDR3, SEQ ID NO: 62), und die leichte Kette des Immunglobulins den folgenden Satz an CDR-Sequenzen umfasst: QSIGTN (LCDR1, SEQ ID NO: 63), YAS (LCDR2) und QQSNSWPYT (LCDR3, SEQ ID NO: 64), und
wobei die Aminosäuresequenz des Lipocalin-Muteins den folgenden Satz an mutierten Aminosäureresten im Vergleich zur linearen Polypeptidsequenz eines reifen hNGAL umfasst, die in SEQ ID NO: 2 gezeigt ist: Gln 28 --+ His; Leu 36 --+ Gln; Ala 40 --+ Ile; Ile 41 --+ Arg; Gln 49 --+ Ile; Tyr 52 --+ Met; Asn 65 --+ Asp; Ser 68 --+ Met; Leu 70 → Lys; Arg 72 --+ Asp; Lys 73 --+ Asp; Asp 77 --+ Met; Trp 79 --+ Asp; Arg 81 --+ Trp; Cys 87 --+ Ser; Asn 96 --+ Lys; Tyr 100 --+ Phe; Leu 103 --+ His; Tyr 106 --+ Ser; Lys 125 → Phe; Ser 127 --+ Phe; Tyr 132 --+ Glu und Lys 134 --+ Tyr.

2. Fusionsprotein nach Anspruch 1, wobei die Aminosäuresequenz des Lipocalin-Muteins mindestens 90 % Sequenzidentität mit der Aminosäuresequenz von SEQ ID NO: 42 aufweist.

3. Fusionsprotein nach Anspruch 1 oder 2, wobei die Aminosäuresequenz des Lipocalin-Muteins die Aminosäuresequenz von SEQ ID NO: 42 umfasst.

4. Fusionsprotein nach einem der Ansprüche 1 bis 3, wobei die zweite Untereinheit am N-Terminus über einen Linker mit dem C-Terminus jeder konstanten Region der schweren Kette (CH) der ersten Untereinheit verknüpft ist, wobei der Linker vorzugsweise der Linker von SEQ ID NO: 13 ist.

5. Fusionsprotein nach einem der Ansprüche 1 bis 4, wobei das Immunglobulin eine variable Region der schweren Kette und eine variable Region der leichten Kette umfasst, umfassend die Aminosäuresequenzen, die in SEQ ID NOs: 77 und 82 gezeigt sind.

6. Fusionsprotein nach einem der Ansprüche 1 bis 5, wobei das Immunglobulin eine schwere Kette und eine leichte Kette umfasst, umfassend die Aminosäuresequenzen, die in SEQ ID NOs: 86 und 87 gezeigt sind.

7. Fusionsprotein nach einem der Ansprüche 1 bis 5, wobei das Immunglobulin ein IgG4-Grundgerüst aufweist, wobei das IgG4-Grundgerüst vorzugsweise eine oder mehrere der folgenden Mutationen aufweist: S228P, N297A, F234A, L235A, M428L, N434S, M252Y, S254T und T256E gemäß dem EU-Index von Kabat.

8. Fusionsprotein nach einem der Ansprüche 1 bis 5 oder 7, wobei das Fusionsprotein Aminosäuresequenzen umfasst, die mindestens 92 %, mindestens 95 %, mindestens 97 %, mindestens 98 % oder eine höhere Sequenzidentität mit den Aminosäuresequenzen aufweisen, die in SEQ ID NOs: 90 und 87 gezeigt sind.

9. Fusionsprotein nach einem der Ansprüche 1 bis 8, wobei das Fusionsprotein die Aminosäuresequenzen umfasst, die in SEQ ID NOs: 90 und 87 gezeigt sind.

10. Nukleinsäuremolekül, umfassend eine Nukleotidsequenz, die das Fusionsprotein nach einem der Ansprüche 1 bis 9 kodiert, wobei vorzugsweise
(i) das Nukleinsäuremolekül funktionsfähig mit einer regulatorischen Sequenz verknüpft ist, um eine Expression des Nukleinsäuremoleküls zu ermöglichen; oder
(ii) das Nukleinsäuremolekül in einem Vektor oder in einem Phagemid-Vektor umfasst ist.

11. Wirtszelle, enthaltend ein Nukleinsäuremolekül nach Anspruch 10.

12. Verfahren zur Herstellung des Fusionsproteins nach einem der Ansprüche 1 bis 9, wobei das Fusionsprotein ausgehend von der Nukleinsäure, die für das Fusionsprotein kodiert, hergestellt wird, wobei das Fusionsprotein vorzugsweise in einem bakteriellen oder eukaryotischen Wirtsorganismus hergestellt wird und aus diesem Wirtsorganismus oder seiner Kultur isoliert wird.

13. Pharmazeutische Zusammensetzung, umfassend ein Fusionsprotein nach einem der Ansprüche 1 bis 9.

14. Fusionsprotein nach einem der Ansprüche 1 bis 9, zur Verwendung in der Therapie.

15. Fusionsprotein zur Verwendung nach Anspruch 14, wobei die Verwendung bei der Behandlung von Krebs erfolgt.

## Revendications

1. Protéine de fusion qui est capable de se lier à la fois à CD137 et à PD-L1, dans laquelle le protéine de fusion comprend deux sous-unités, dans laquelle la première sous-unité comprend une immunoglobuline de longueur totale qui est spécifique de PD-L1, et dans laquelle la seconde sous-unité comprend une mutéine de lipocaline qui est spécifique de CD137,
dans laquelle la protéine de fusion comprend des séquences d'acides aminés ayant une identité de séquence d'au moins 90 % avec les séquences d'acides aminés présentées dans SEQ ID NO : 90 et 87,
dans laquelle la chaîne lourde de l'immunoglobuline comprend le jeu suivant de séquences de CDR: GFSLSNYD (HCDR1, SEQ ID NO: 60), IWTGGAT (HCDR2, SEQ ID NO : 61), et VRDSNYRYDEPFTY (HCDR3, SEQ ID NO : 62), et la chaîne légère de l'immunoglobuline comprend le jeu suivant de séquences de CDR : QSIGTN (LCDR1, SEQ ID NO : 63), YAS (LCDR2), et QQSNSWPYT (LCDR3, SEQ ID NO : 64), et
dans laquelle la séquence d'acides aminés de la mutéine de lipocaline comprend le jeu suivant de résidus d'acides aminés mutés comparés à la séquence polypeptidique linéaire de la hNGAL mature telle que présentée dans SEQ ID NO : 2 : Gln 28 --+ His ; Leu 36 --+ Gln ; Ala 40 --+ Ile ; Ile 41 --+ Arg ; Gln 49 --+ Ile ; Tyr 52 --+ Met ; Asn 65 --+ Asp ; Ser 68 --+ Met ; Leu 70 --+ Lys ; Arg 72 --+ Asp ; Lys 73 --+ Asp ; Asp 77 --+ Met ; Trp 79 --+ Asp ; Arg 81 --+ Trp ; Cys 87 --+ Ser ; Asn 96 --+ Lys ; Tyr 100 --+ Phe ; Leu 103 → His ; Tyr 106 --+ Ser ; Lys 125 --+ Phe; Ser 127 --+ Phe ; Tyr 132 --+ Glu ; et Lys 134 → Tyr.

2. Protéine de fusion selon la revendication 1, dans laquelle la séquence d'acides aminés de la mutéine de lipocaline présente une identité de séquence d'au moins 90% avec la séquence d'acides aminés de SEQ ID NO : 42.

3. Protéine de fusion selon la revendication 1 ou 2, dans laquelle la séquence d'acides aminés de la mutéine de lipocaline comprend la séquence d'acides aminés de SEQ ID NO : 42.

4. Protéine de fusion selon l'une quelconque des revendications 1 à 3, dans laquelle la seconde sous-unité est liée au niveau de l'extrémité N-terminale par l'intermédiaire d'un lieur à l'extrémité C-terminale de chaque région constante de chaîne lourde (CH) de la première sous-unité, dans laquelle le lieur est de préférence le lieur de SEQ ID NO : 13.

5. Protéine de fusion selon l'une quelconque des revendications 1 à 4, dans laquelle l'immunoglobuline comprend une région variable de chaîne lourde et une région variable de chaîne légère comprenant les séquences d'acides aminés telles que présentées dans SEQ ID NO : 77 et 82.

6. Protéine de fusion selon l'une quelconque des revendications 1 à 5, dans laquelle l'immunoglobuline comprend une chaîne lourde et une chaîne légère comprenant les séquences d'acides aminés telles que présentées dans SEQ ID NO : 86 et 87.

7. Protéine de fusion selon l'une quelconque des revendications 1 à 5, dans laquelle l'immunoglobuline a un squelette d'IgG4, dans laquelle le squelette d'IgG4 a de préférence une ou plusieurs des mutations suivantes : S228P, N297A, F234A, L235A, M428L, N434S, M252Y, S254T, et T256E conformément à l'indice EU de Kabat.

8. Protéine de fusion selon l'une quelconque des revendications 1 à 5 ou 7, dans laquelle la protéine de fusion comprend des séquences d'acides aminés ayant une identité de séquence d'au moins 92%, d'au moins 95%, d'au moins 97%, d'au moins 98%, ou plus avec les séquences d'acides aminés telles que présentées dans les SEQ ID NO : 90 et 87.

9. Protéine de fusion selon l'une quelconque des revendications 1 à 8, dans laquelle la protéine de fusion comprend les séquences d'acides aminés telles que présentées dans SEQ ID NO : 90 et 87.

10. Molécule d'acide nucléique comprenant une séquence nucléotidique codant pour une protéine de fusion selon l'une quelconque des revendications 1 à 9, dans laquelle de préférence
(i) la molécule d'acide nucléique est liée de manière fonctionnelle à une séquence de régulation pour permettre l'expression de ladite molécule d'acide nucléique ; ou
(ii) la molécule d'acide nucléique est comprise dans un vecteur ou dans un vecteur phagémide.

11. Cellule hôte contenant une molécule d'acide nucléique selon la revendication 10.

12. Procédé de production d'une protéine de fusion selon l'une quelconque des revendications 1 à 9, dans lequel la protéine de fusion est produite à partir de l'acide nucléique codant pour la protéine de fusion, dans lequel la protéine de fusion est de préférence produite dans un organisme hôte bactérien ou eucaryote et est isolé de cet organisme hôte ou de sa culture.

13. Composition pharmaceutique comprenant une protéine de fusion selon l'une quelconque des revendications 1 à 9.

14. Protéine de fusion selon l'une quelconque des revendications 1 à 9 pour son utilisation en thérapie.

15. Protéine de fusion pour son utilisation selon la revendication 14, dans laquelle l'utilisation est dans le traitement d'un cancer.
